# EUROPÄISCHE PATENTSCHRIFT

(11) **EP 1 287 142 B1**
(45) Veröffentlichungstag und Bekanntmachung des Hinweises auf die Patenterteilung: **09.11.2005**
(21) Anmeldenummer: 01936423.1
(22) Anmeldetag: 01.06.2001
(51) Int. Cl.: C12N 15/19, C12N 15/71, C12N 15/79, C07K 16/00, C07K 14/52, C12Q 1/68, A61K 38/19, G01N 33/53

(54) **NUKLEINSAURE-MOLEKUL UMFASSEND EINE FUR EIN SDF-1 GAMMA CHEMOKIN,EINEN NEUROPEPTID-PRAKURSOR ODER MINDESTENS EIN NEUROPEPTID KODIERENDE NUKLEINSAURESEQUENZ**
NUCLEIC ACID MOLECULE COMPRISING A NUCLEIC ACID SEQUENCE CODING FOR AN SDF-1 GAMMA CHEMOKINE, A NEUROPEPTIDE PRECURSOR OR AT LEAST ONE NEUROPEPTIDE
MOLECULE D'ACIDE NUCLEIQUE CONTENANT UNE SEQUENCE D'ACIDE NUCLEIQUE CODANT POUR UNE CHIMIOKINE SDF-1 GAMMA, UN PRECURSEUR NEUROPEPTIDIQUE OU AU MOINS UN NEUROPEPTIDE

(30) Priorität: 02.06.2000 DE 10027383
(43) Veröffentlichungstag der Anmeldung: 05.03.2003
(73) Patentinhaber: NEURAXO BIOTEC GmbH, 40225 Düsseldorf (DE)
(72) Erfinder: MÜLLER, Hans, Werner, 40595 Düsseldorf (DE); BOSSE, Frank, 40225 Düsseldorf (DE); GLEICHMANN, Marc, 72070 Tübingen (DE); GILLEN, Clemens, 52076 Aachen (DE); AUER, Johannes, 82445 Schwaigen OT Grafenaschau (DE)
(74) Vertreter: Vogelsang-Wenke, Heike
(86) Internationale Anmeldenummer: PCT/EP2001/006250
(87) Internationale Veröffentlichungsnummer: WO 2001/092530

(56) Entgegenhaltungen:
- EP-A- 0 657 468
- WO-A-00/06698
- WO-A-01/44266
- WO-A-97/38004
- WO-A-99/20759
- WO-A-99/55915
- MARC GLEICHMANN ET AL.: "Cloning and characterization of SDF1-Gamma, a novel SDF-1 chemokine transcript with developmentally regulated expression in the nervous system" EUROPEAN JOURNAL OF NEUROSCIENCE, Bd. 12, Seiten 1857-1866, XP002173374
- KLUGBAUER N ET AL: "STRUCTURE AND FUNCTIONAL EXPRESSION OF A NEW MEMBER OF THE TETRODOTOXIN-SENSITIVE VOLTAGE-ACTIVATED SODIUM CHANNEL FAMILY FROMHUMAN NEUROENDOCRINE CELLS" EMBO JOURNAL,GB,OXFORD UNIVERSITY PRESS, SURREY, Bd. 14, Nr. 6, 15. März 1995 (1995-03-15), Seiten 1084-1090, XP002069908 ISSN: 0261-4189
- DATABASE GENBANK [Online] Acc. U09256, KIM S. ET AL.: XP002173551
- DATABASE BIOSIS [Online] BIOSCIENCES INFORMATION SERVICE, PHILADELPHIA, PA, US; PREV200100088678 , CZARDYBON ET AL.: "Distribution of the novel chemokine SDF-1 Gamma and CXCR-4 Receptor Transcripts in the Nervous System of Rat during Development. Nov. 2000" XP002173375

### SEQUENZPROTOKOLL

<110> Rhein Biotech Gesellschaft für neue biotechnologische Prozesse und Produkte mbH
<120> Nukleinsäure-Molekül umfassend eine für ein Chemokin, einen Neuropeptid-Präkursor oder mindestens ein Neuropeptid kodierende Nukleinsäuresequenz
<130> PCT1385-1966
<140>
   <141>
<150> DE 100 27 383.1
   <151> 2000-06-02
<160> 22
<170> PatentIn Ver. 2.1
<210> 1
   <211> 90
   <212> DNA
   <213> Künstliche Sequenz
<220>
   <223> Beschreibung der künstlichen Sequenz:
   Konsensussequenz für den spezifischen Bereich von SDF-1-gamma
<400> 1
   gggcgcagag aagaaaaagt ggggaaaaaa gaaaagatag gaaaaaagaa gcgacagaag 60
   aagagaaagg ckgcccagaa aargaaaaac                                  90
<210> 2
   <211> 30
   <212> PRT
   <213> Künstliche Sequenz
<220>
   <223> Beschreibung der künstlichen Sequenz:
   Konsensussequenz für den spezifischen Bereich von SDF-1-gamma
<220>
   <221> SITE
   <222> (28)
   <223> Xaa = Arg oder Lys
<400> 2
   Gly Arg Arg Glu Glu Lys Val Gly Lys Lys Glu Lys Ile Gly Lys Lys
    1               5                  10                  15
   Lys Arg Gln Lys Lys Arg Lys Ala Ala Gln Lys Xaa Lys Asn
               20                  25                  30
<210> 3
   <211> 360
   <212> DNA
   <213> Künstliche Sequenz
<220>
   <223> Beschreibung der künstlichen Sequenz:
   Konsensussequenz für den kodierenden Bereich von SDF-1-gamma
<400> 3
   atgracgcca aggtcgtsgy cgtgctggyc ctsgtgctgr ccgcgctctg cmtcagygac 60
   ggkaagccmg tcagcctgag ctacagatgc ccmtgccgat tcttygarag ccatgtygcc 120
   agagccaacg tcaarcatct saaaatyctc aacactccaa actgtgccct tcagattgtw 180
   gcmmggctga ararcaacaa cagacaagtg tgcattgacc cgaarytaaa gtggatycar 240
   gagtacctgg asaaagcytt aaacaagggg cgcagagaag aaaaagtggg gaaaaaagaa 300
   aagataggaa aaaagaagcg acagaagaag agaaaggckg cccagaaaar gaaaaactag 360
<210> 4
   <211> 119
   <212> PRT
   <213> Künstliche Sequenz
<220>
   <223> Beschreibung der künstlichen Sequenz:
   Konsensussequenz für das SDF-1-gamma-Polypeptid
<220>
   <221> SITE
   <222> (7)
   <223> Xaa = Val oder Ala
<220>
   <221> SITE
   <222> (10)
   <223> Xaa = Val oder Ala
<220>
   <221> SITE
   <222> (14)
   <223> Xaa = Thr oder Ala
<220>
   <221> SITE
   <222> (18)
   <223> Xaa = Leu oder Ile
<220>
   <221> SITE
   <222> (65)
   <223> Xaa = Asn oder Ser
<220>
   <221> SITE
   <222> (84)
   <223> Xaa = Glu oder Asp
<220>
   <221> SITE
   <222> (117)
   <223> Xaa = Arg oder Lys
<400> 4
   Met Asx Ala Lys Val Val Xaa Val Leu Xaa Leu Val Leu Xaa Ala Leu
   1               5                   10                  15
   Cys Xaa Ser Asp Gly Lys Pro Val Ser Leu Ser Tyr Arg Cys Pro Cys
               20                  25                  30
   Arg Phe Phe Glu Ser His Val Ala Arg Ala Asn Val Lys His Leu Lys
           35                  40                  45
   Ile Leu Asn Thr Pro Asn Cys Ala Leu Gln Ile Val Ala Arg Leu Lys
       50                  55                  60
   Xaa Asn Asn Arg Gln Val Cys Ile Asp Pro Lys Leu Lys Trp Ile Gln
   65                  70                  75                  80
   Glu Tyr Leu Xaa Lys Ala Leu Asn Lys Gly Arg Arg Glu Glu Lys Val
                   85                  90                  95
   Gly Lys Lys Glu Lys Ile Gly Lys Lys Lys Arg Gln Lys Lys Arg Lys
               100                 105                 110
   Ala Ala Gln Lys Xaa Lys Asn
           115
<210> 5
   <211> 7
   <212> PRT
   <213> Künstliche Sequenz
<220>
   <223> Beschreibung der künstlichen Sequenz: Durch proteolytische Spaltung von SDF-1-gamma erhältliches Peptid
<400> 5
   Arg Arg Glu Glu Lys Val Gly
   1 5
<210> 6
   <211> 6
   <212> PRT
   <213> Künstliche Sequenz
<220>
   <223> Beschreibung der künstlichen Sequenz: Durch proteolytische Spaltung von SDF-1-gamma erhältliches Peptid
<400> 6
   Lys Lys Glu Lys Ile Gly
   1 5
<210> 7
   <211> 90
   <212> PRT
   <213> Künstliche Sequenz
<220>
   <223> Beschreibung der künstlichen Sequenz: Durch proteolytische Spaltung von SDF-1-gamma erhältliches Peptid
<400> 7
   Met Asx Ala Lys Val Val Xaa Val Leu Xaa Leu Val Leu Xaa Ala Leu
   1 5 10 15
   Cys Xaa Ser Asp Gly Lys Pro Val Ser Leu Ser Tyr Arg Cys Pro Cys
   20 25 30
   Arg Phe Phe Glu Ser His Val Ala Arg Ala Asn Val Lys His Leu Lys
   35 40 45
   Ile Leu Asn Thr Pro Asn Cys Ala Leu Gln Ile Val Ala Arg Leu Lys
   50 55 60
   Xaa Asn Asn Arg Gln Val Cys Ile Asp Pro Lys Leu Lys Trp Ile Gln
   65 70 75 80
   Glu Tyr Leu Xaa Lys Ala Leu Asn Lys Gly
   85 90
<210> 8
   <211> 5
   <212> PRT
   <213> Künstliche Sequenz
<220>
   <223> Beschreibung der künstlichen Sequenz: Durch proteolytische Spaltung von SDF-1-gamma erhältliches Peptid
<400> 8
   Lys Lys Lys Arg Gln
   1 5
<210> 9
   <211> 7
   <212> PRT
   <213> Künstliche Sequenz
<220>
   <223> Beschreibung der künstlichen Sequenz: Durch proteolytische Spaltung von SDF-1-gamma erhältliches Peptid
<400> 9
   Lys Lys Arg Lys Ala Ala Gln
   1 5
<210> 10
   <211> 4
   <212> PRT
   <213> Künstliche Sequenz
<220>
   <223> Beschreibung der künstlichen Sequenz: Durch proteolytische Spaltung von SDF-1-gamma erhältliches Peptid
<220>
   <221> SITE
   <222> (2)
   <223> Xaa = Arg oder Lys
<400> 10
   Lys Xaa Lys Asn
   1
<210> 11
   <211> 5391
   <212> DNA
   <213> Rattus norvegicus
<400> 11
   tgtcctcttg ctgtccagct ctgcagcctc cggcgcgccc tcccgcccac gccatggacg 60
   ccaaggtcgt cgccgtgctg gccctggtgc tggccgcgct ctgcatcagt gacggtaagc 120
   cagtcagcct gagctacaga tgcccctgcc gattctttga gagccatgtc gccagagcca 180
   acgtcaaaca tctgaaaatc ctcaacactc caaactgtgc ccttcagatt gttgcaaggc 240
   tgaaaagcaa caacagacaa gtgtgcattg acccgaaatt aaagtggatc caagagtacc 300
   tggacaaagc cttaaacaag gggcgcagag aagaaaaagt ggggaaaaaa gaaaagatag 360
   gaaaaaagaa gcgacagaag aagagaaagg cggcccagaa aaagaaaaac tagttacgtg 420
   cttcctgcag atggaccaca gtacgctctg ctctggcgct ttgtaacccc cccttccctc 480
   tccgggggca gaccccacac tccgggcagg tgctcagact gatggtaaac tcttccctct 540
   tctgggggca gaccacacat cccagggaag accccacacc cccgggcaga tgcttaggct 600
   ttcctgcccc ggcggccaca ccagctgctg tatttacgcg cttcttaagg ccctgctctg 660
   tctgctaagc tatgaagaaa gatgtgcaga gactggggtg gaggctaagc cacagaggac 720
   ctgcctagcc tggcagcttg ccccgagctg agccccttgg ccaggagttc acaaggctca 780
   cacctacaat cccatgaagg ccagggtggt ctgcttagcc aggaaagggc atgtgccttc 840
   ccctcaacca cactgccccc tgtggccttc tcaggtaact gacttgctct caggcccacg 900
   ggaagctttt ccaaatacct gcggcctggg aagggacttc attcagccct gctgcccggg 960
   ctgtgggagc agcttggttt caacacagaa gggtatctgc agactgtgtt gggtgaaaag 1020
   caggaagaat gaagtctcag agaacgcatg ttagctgctt ctcagggaat ctttcctttg 1080
   gaaaattcac tttagagtct ttaaacgggt ccctcatggg gagggcagat gtgctctggg 1140
   actttctgat gggccagcag cttcagggac tcttagtctg tcctccccac ccttggtctc 1200
   aacattccca ggatggtgtg ctatccggtc accaatgcct ccgtcctcac tcctgagaga 1260
   tgtctgcctt ctgtggattg ggttaaagct ctggaattac ctaatatccc aacccaccac 1320
   cctcacctgg caatttttgt ctagtctttt gtttttgtct ttctccattt tggattagaa 1380
   ggatagaggg caaggctctg attttagcag tgttttggag aaaaaatttt ttttcttcat 1440
   ctcatgtaga cacacacaca cacacacaca cacacacaca cacacacaca cacacacaca 1500
   tcttgtaccc cagacctctg ggtctaattt tcataattgg ggcagaaaga agaaatgatc 1560
   tgaagataca gcaaatggat tgcaggggaa ggaaggccca gtgtcctgtg tgtcatgccc 1620
   tcttgggtcc ctaagttcta ggttccttag agggtctaac attaaatagg taagaggcct 1680
   tcatggtcct tgctggggaa gggtctcacc agggagcttc agggaagacc catgttacga 1740
   actcttatgc tttatctgga cagccctcct ggtccatacc ctctcctcag atctgaggta 1800
   gcggggtggg ctattggtgg gcgtctttca agcccagggt tactgtctgt tcttcttggg 1860
   gcagccagtt acagtctggt ctcagtggcc ttggctgcat ccttcctact gttgacaaaa 1920
   cacttctgaa ggccagatct gtgcccaagc catagttctg cctagaaatg gatgcccagc 1980
   ccctccagga cactgggaag gactgttggc ccctaacaac caaaggccat actgaggctg 2040
   ccctgagttg gaagaccact ttccgaaatg cccctggact ctgcctccca ccatccaccc 2100
   ctgactccta ggagttagag agtaggaaaa cagtttgttt cttaggaacc acagcaagct 2160
   cccaggagcc ctctgtgctt atgaagccca tctaatgggc agccccagcc ttctggacag 2220
   agtcctcatg gaaatgcgtg agaagctgat ttcgtctaag gatgggttga aggtaggatg 2280
   tgctcctgta tgttctcagg caggtgagag agggtcttcc ttacagtatc tagcataaac 2340
   accttctgga aggttctgca gctctagaga tcacctcctc agtgccaaga cctcttctgg 2400
   tggtgtggga gcagccaaga gatttcaagg aagagtgatt atttgatgaa ataacttgaa 2460
   ttatatcaag agtgaatatt tgatgggaac tgcctcttct cttggagttc tgaggcctgg 2520
   ggatgcccag gaactcaggg cacctgctgt tgttggagtc gatgcatagt ctcaacacca 2580
   gtgtcctaag gttaaggcag tgtgccttgt catgtgttcc ttgtaccatg cctcctgtgc 2640
   cagtgtgtgt gccttagcct gtgcttgaca tgttcacccg tcttctctgc ttgccaccac 2700
   cacccagacc ctcagcatca gtcctggctg tgcccctccc tgccctccca ctctctcagg 2760
   ccttggaagg aagatggctc gactgcaagc tgaactaagg agtagggcct gtggctcctg 2820
   ccaggccaca cagcatccca ggcacgtggt gagaatccgc cttaatgtgt ctcctctgtt 2880
   cttgtcaaca ggaggctcaa gatgtgagag gtgtgagtca gacgcctgag gaacttacag 2940
   aaggagccta ggtctgaagt cagtgttagg gaagggccca tagccacttc ctctgctcct 3000
   gagcagggct gaagccattt ccaagggact tgctttgcag tttgctacac tttcaccatt 3060
   tgattatata gcaagataca tggtaattat tttattttca tttagtctga ttctccaatg 3120
   tcattggtga caggccaagg ccactatgtc atctcctttg ttctagtatc tttcccatga 3180
   aggacctttt ctgaatagtg gctcccaagg tttgtctctt tgagctgagg caggaggctc 3240
   acctttttct gattagaaac tgggtgttcc tacccccaag gattgcaggg ctttccccaa 3300
   gctgaggcag gagtgtgagg tcagggaaga gcgagatcca ccctcatccc atgctctcct 3360
   cttcatccca ccatgctcat ctctgtctca tccatcaccg tgtgtctgca agactgtctc 3420
   catgacccgg aaaaaggact ctctcaagag gaactccttt actcaaaatg ggacagcaag 3480
   aaggaaaagg aagtgtctgt tgttccgccc aaacccttcg cgcgtytatt gtcttgtttg 3540
   gaatattgtc tcttcaaccc cctgcttctg ttgacctcca tgaccaatgt ctcgtctgtg 3600
   cactgtctct aacccaaatg caaaggctgt gtatgaggta atggccctga ggtccaggtt 3660
   ttcatggaaa cagcgcactg tctccctgtt cacaggctca ttttggacac acagagccca 3720
   aagaaaggtg gtttgcaaca gagctcagct ctaagactgt agatccttca tattttcgta 3780
   ctgttasstt taaattgtgg gttcttasst tcctggaacc gaatgcattc ttctattgag 3840
   nactagcagg tctcagttct ttccaattat ttttaaaagc caatgaataa aagcatcagc 3900
   attgggccca ctgggcgggc atttctctag aaaggggaga accacctacc tttccttagg 3960
   acagccgacc agcacggncc caggaagtgn nnnnntcttc tgcagttttt atacaagctc 4020
   ccctgccacc tttgacaaac gcacagttaa gagtcagtat ctagttcttc agagacaaag 4080
   atggagggag taagaagggg aagggaaacg gagaaagcta ccaaaagatc atcctcaaaa 4140
   gcnggtgttt gagagtgaac gagctgtaga attgttagtg atgtgtgtgt ggtgagggat 4200
   ttctataaat agtcattcaa gttgatttca cagcagatga aaaatccaac cagcaagatt 4260
   ttgatcaaat ttggacaaca gcaacaaawc taaaaatgtg aagccagttg ggataagggg 4320
   catatggttt gctgcagact gggtccccat gtggattcag aattatttta aactctcttg 4380
   acatccgggg cccccacaag agaaatctgg attgctgtgc aatggccact tagcatctaa 4440
   tccaagcttt gaaggaaaca aatacagcct tgcaccttcn ctccagttag ggatcctttt 4500
   aaagtctcct tcacagggag gataaagaga ctgggtagaa actggaggga gatgaatttg 4560
   tgtatcaatt ccgctgctga cagtcatttt ctagawggag acagcctgcc tagagcaaat 4620
   gtgcanttwa ataggrcatt tacatnggra rmgcctctcc ccaccttnat cccccatgct 4680
   cttrctttca aaatnacaag ncacagcagt ccttgaatgg ttgttgacsc cgsacaccta 4740
   actgtccctg atgatcctgg tgcwgcccng aattcccttg gncgccaagt aacctgccag 4800
   gcagccnagt ccctytgtca ccagcctttg catctggata gggaaaaggg ggttggagac 4860
   atacagtctg ctttgtgttg aanccnagat tngtacsctg tgtttacact gtgctgcctg 4920
   ctctcgggna cagtgggaag gaagtgcagc cgaggtggca gacccctctg attcattsct 4980
   ggtcggcttt gagggagggt ttggagagca aaaggctgca ttcctctgtg ggacttgcct 5040
   gagcctttag syctctccat cgagttctgt ttatcttctc atgggtgatt atctcggcgg 5100
   cgtcaccagg ggcttcctca cagaagtcat ncctcngcag agcttgcagt gtctacgcag 5160
   cgatggtttc agtgttgcat gtggtgaata ctgtattttg tttcagttct gtctcccaga 5220
   taatgtgaaa atggtccagg agaaggcagc ttcctatacg cagtgtgtgc tttcttattc 5280
   tcgtttttaa tatatgacag ttatttgaga ggccatttct actttgaagt catatcaatg 5340
   aaaatgatgt atcttcacct acaatttttc ctaataaagt tctgtattcg a          5391
<210> 12
   <211> 119
   <212> PRT
   <213> Homo sapiens
<400> 12
   Met Asn Ala Lys Val Val Val Val Leu Val Leu Val Leu Thr Ala Leu
   1 5 10 15
   Cys Leu Ser Asp Gly Lys Pro Val Ser Leu Ser Tyr Arg Cys Pro Cys
   20 25 30
   Arg Phe Phe Glu Ser His Val Ala Arg Ala Asn Val Lys His Leu Lys
   35 40 45
   Ile Leu Asn Thr Pro Asn Cys Ala Leu Gln Ile Val Ala Arg Leu Lys
   50 55 60
   Asn Asn Asn Arg Gln Val Cys Ile Asp Pro Lys Leu Lys Trp Ile Gln
   65 70 75 80
   Glu Tyr Leu Glu Lys Ala Leu Asn Lys Gly Arg Arg Glu Glu Lys Val
   85 90 95
   Gly Lys Lys Glu Lys Ile Gly Lys Lys Lys Arg Gln Lys Lys Arg Lys
   100 105 110
   Ala Ala Gln Lys Arg Lys Asn
   115
<210> 13
   <211> 119
   <212> PRT
   <213> Rattus norvegicus
<400> 13
   Met Asp Ala Lys Val Val Ala Val Leu Ala Leu Val Leu Ala Ala Leu
   1 5 10 15
   Cys Ile Ser Asp Gly Lys Pro Val Ser Leu Ser Tyr Arg Cys Pro Cys
   20 25 30
   Arg Phe Phe Glu Ser His Val Ala Arg Ala Asn Val Lys His Leu Lys
   35 40 45
   Ile Leu Asn Thr Pro Asn Cys Ala Leu Gln Ile Val Ala Arg Leu Lys
   50 55 60
   Ser Asn Asn Arg Gln Val Cys Ile Asp Pro Lys Leu Lys Trp Ile Gln
   65 70 75 80
   Glu Tyr Leu Asp Lys Ala Leu Asn Lys Gly Arg Arg Glu Glu Lys Val
   85 90 95
   Gly Lys Lys Glu Lys Ile Gly Lys Lys Lys Arg Gln Lys Lys Arg Lys
   100 105 110
   Ala Ala Gln Lys Lys Lys Asn
   115
<210> 14
   <211> 360
   <212> DNA
   <213> Homo sapiens
<400> 14
   atgaacgcca aggtcgtggt cgtgctggtc ctcgtgctga ccgcgctctg cctcagcgac 60
   gggaagcccg tcagcctgag ctacagatgc ccatgccgat tcttcgaaag ccatgttgcc 120
   agagccaacg tcaagcatct caaaattctc aacactccaa actgtgccct tcagattgta 180
   gcccggctga agaacaacaa cagacaagtg tgcattgacc cgaagctaaa gtggattcag 240
   gagtacctgg agaaagcttt aaacaagggg cgcagagaag aaaaagtggg gaaaaaagaa 300
   aagataggaa aaaagaagcg acagaagaag agaaaggctg cccagaaaag gaaaaactag 360
<210> 15
   <211> 360
   <212> DNA
   <213> Rattus norvegicus
<400> 15
   atggacgcca aggtcgtcgc cgtgctggcc ctggtgctgg ccgcgctctg catcagtgac 60
   ggtaagccag tcagcctgag ctacagatgc ccctgccgat tctttgagag ccatgtcgcc 120
   agagccaacg tcaaacatct gaaaatcctc aacactccaa actgtgccct tcagattgtt 180
   gcaaggctga aaagcaacaa cagacaagtg tgcattgacc cgaaattaaa gtggatccaa 240
   gagtacctgg acaaagcctt aaacaagggg cgcagagaag aaaaagtggg gaaaaaagaa 300
   aagataggaa aaaagaagcg acagaagaag agaaaggcgg cccagaaaaa gaaaaactag 360
<210> 16
   <211> 2819
   <212> DNA
   <213> Rattus norvegicus
<400> 16
   tgtcctcttg ctgtccagct ctgcagcctc cggcgcgccc tcccgcccac gccatggacg 60
   ccaaggtcgt cgccgtgctg gccctggtgc tggccgcgct ctgcatcagt gacggtaagc 120
   cagtcagcct gagctacaga tgcccctgcc gattctttga gagccatgtc gccagagcca 180
   acgtcaaaca tctgaaaatc ctcaacactc caaactgtgc ccttcagatt gttgcaaggc 240
   tgaaaagcaa caacagacaa gtgtgcattg acccgaaatt aaagtggatc caagagtacc 300
   tggacaaagc cttaaacaag aggctcaaga tgtgagaggt gtgagtcaga cgcctgagga 360
   acttacagaa ggagcctagg tctgaagtca gtgttaggga agggcccata gccacttcct 420
   ctgctcctga gcagggctga agccatttcc aagggacttg ctttgcagtt tgctacactt 480
   tcaccatttg attatatagc aagatacatg gtaattattt tattttcatt tagtctgatt 540
   ctccaatgtc attggtgaca ggccaaggcc actatgtcat ctcctttgtt ctagtatctt 600
   tcccatgaag gaccttttct gaatagtggc tcccaaggtt tgtctctttg agctgaggca 660
   ggaggctcac ctttttctga ttagaaactg ggtgttccta cccccaagga ttgcagggct 720
   ttccccaagc tgaggcagga gtgtgaggtc agggaagagc gagatccacc ctcatcccat 780
   gctctcctct tcatcccacc atgctcatct ctgtctcatc catcaccgtg tgtctgcaag 840
   actgtctcca tgacccggaa aaaggactct ctcaagagga actcctttac tcaaaatggg 900
   acagcaagaa ggaaaaggaa gtgtctgttg ttccgcccaa acccttcgcg cgtytattgt 960
   cttgtttgga atattgtctc ttcaaccccc tgcttctgtt gacctccatg accaatgtct 1020
   cgtctgtgca ctgtctctaa cccaaatgca aaggctgtgt atgaggtaat ggccctgagg 1080
   tccaggtttt catggaaaca gcgcactgtc tccctgttca caggctcatt ttggacacac 1140
   agagcccaaa gaaaggtggt ttgcaacaga gctcagctct aagactgtag atccttcata 1200
   ttttcgtact gttassttta aattgtgggt tcttassttc ctggaaccga atgcattctt 1260
   ctattgagna ctagcaggtc tcagttcttt ccaattattt ttaaaagcca atgaataaaa 1320
   gcatcagcat tgggcccact gggcgggcat ttctctagaa aggggagaac cacctacctt 1380
   tccttaggac agccgaccag cacggnccca ggaagtgnnn nnntcttctg cagtttttat 1440
   acaagctccc ctgccacctt tgacaaacgc acagttaaga gtcagtatct agttcttcag 1500
   agacaaagat ggagggagta agaaggggaa gggaaacgga gaaagctacc aaaagatcat 1560
   cctcaaaagc nggtgtttga gagtgaacga gctgtagaat tgttagtgat gtgtgtgtgg 1620
   tgagggattt ctataaatag tcattcaagt tgatttcaca gcagatgaaa aatccaacca 1680
   gcaagatttt gatcaaattt ggacaacagc aacaaawcta aaaatgtgaa gccagttggg 1740
   ataaggggca tatggtttgc tgcagactgg gtccccatgt ggattcagaa ttattttaaa 1800
   ctctcttgac atccggggcc cccacaagag aaatctggat tgctgtgcaa tggccactta 1860
   gcatctaatc caagctttga aggaaacaaa tacagccttg caccttcnct ccagttaggg 1920
   atccttttaa agtctccttc acagggagga taaagagact gggtagaaac tggagggaga 1980
   tgaatttgtg tatcaattcc gctgctgaca gtcattttct agawggagac agcctgccta 2040
   gagcaaatgt gcanttwaat aggrcattta catnggrarm gcctctcccc accttnatcc 2100
   cccatgctct trctttcaaa atnacaagnc acagcagtcc ttgaatggtt gttgacsccg 2160
   sacacctaac tgtccctgat gatcctggtg cwgcccngaa ttcccttggn cgccaagtaa 2220
   cctgccaggc agccnagtcc ctytgtcacc agcctttgca tctggatagg gaaaaggggg 2280
   ttggagacat acagtctgct ttgtgttgaa nccnagattn gtacsctgtg tttacactgt 2340
   gctgcctgct ctcgggnaca gtgggaagga agtgcagccg aggtggcaga cccctctgat 2400
   tcattsctgg tcggctttga gggagggttt ggagagcaaa aggctgcatt cctctgtggg 2460
   acttgcctga gcctttagsy ctctccatcg agttctgttt atcttctcat gggtgattat 2520
   ctcggcggcg tcaccagggg cttcctcaca gaagtcatnc ctcngcagag cttgcagtgt 2580
   ctacgcagcg atggtttcag tgttgcatgt ggtgaatact gtattttgtt tcagttctgt 2640
   ctcccagata atgtgaaaat ggtccaggag aaggcagctt cctatacgca gtgtgtgctt 2700
   tcttattctc gtttttaata tatgacagtt atttgagagg ccatttctac tttgaagtca 2760
   tatcaatgaa aatgatgtat cttcacctac aatttttcct aataaagttc tgtattcga  2819
<210> 17
   <211> 93
   <212> PRT
   <213> Rattus norvegicus
<400> 17
   Met Asp Ala Lys Val Val Ala Val Leu Ala Leu Val Leu Ala Ala Leu
   1 5 10 15
   Cys Ile Ser Asp Gly Lys Pro Val Ser Leu Ser Tyr Arg Cys Pro Cys
   20 25 30
   Arg Phe Phe Glu Ser His Val Ala Arg Ala Asn Val Lys His Leu Lys
   35 40 45
   Ile Leu Asn Thr Pro Asn Cys Ala Leu Gln Ile Val Ala Arg Leu Lys
   50 55 60
   Ser Asn Asn Arg Gln Val Cys Ile Asp Pro Lys Leu Lys Trp Ile Gln
   65 70 75 80
   Glu Tyr Leu Asp Lys Ala Leu Asn Lys Arg Leu Lys Met
   85 90
<210> 18
   <211> 5
   <212> PRT
   <213> Künstliche Sequenz
<220>
   <223> Beschreibung der künstlichen Sequenz: Durch proteolytische Spaltung von SDF-1-beta erhältliches Peptid
<400> 18
   Lys Arg Leu Lys Met
   1 5
<210> 19
   <211> 21
   <212> DNA
   <213> Künstliche Sequenz
<220>
   <223> Beschreibung der künstlichen Sequenz: Primer MMSE2
<400> 19 <210> 20
   <211> 25
   <212> DNA
   <213> Künstliche Sequenz
<220>
   <223> Beschreibung der künstlichen Sequenz: Primer GAS2
<400> 20
<210> 21
   <211> 25
   <212> DNA
   <213> Künstliche Sequenz
<220>
   <223> Beschreibung der künstlichen Sequenz: Primer GAS3
<400> 21
   gttgagacta tgcatcgact ccaac                                          25
<210> 22
   <211> 101
   <212> PRT
   <213> Künstliche Sequenz
<220>
   <223> Beschreibung der künstlichen Sequenz:
   Aminosäuresequenz des reifen menschlichen SDF-1-gamma-Proteins mit einem C-terminalen Metionin (Konstrukt M-mhSDF-1-gamma)
<400> 22
   Met Asp Gly Lys Pro Val Ser Leu Ser Tyr Arg Cys Pro Cys Arg Phe
    1               5                  10                  15
   Phe Glu Ser His Val Ala Arg Ala Asn Val Lys His Leu Lys Ile Leu
               20                  25                  30
   Asn Thr Pro Asn Cys Ala Leu Gln Ile Val Ala Arg Leu Lys Asn Asn
           35                  40                  45
   Asn Arg Gln Val Cys Ile Asp Pro Lys Leu Lys Trp Ile Gln Glu Tyr
       50                  55                  60
   Leu Glu Lys Ala Leu Asn Lys Gly Arg Arg Glu Glu Lys Val Gly Lys
   65                  70                  75                  80
   Lys Glu Lys Ile Gly Lys Lys Lys Arg Gln Lys Lys Arg Lys Ala Ala
                   85                  90                  95
   Gln Lys Arg Lys Asn
               100

## Beschreibung

Die Erfindung betrifft ein Nukleinsäure-Molekül, das eine für ein Chemokin, einen Neuropeptid-Präkursor oder mindestens ein Neuropeptid kodierende Nukleinsäuresequenz umfasst, sowie dieses Nukleinsäure-Molekül enthaltende Wirtszellen. Die Erfindung betrifft ferner ein Polypeptid-Molekül, das als Chemokin oder Neuropeptid wirkt oder mindestens ein Neuropeptid enthält, sowie Fragmente davon, die mindestens ein Neuropeptid enthalten, und ein Verfahren zum Herstellen des Polypeptid-Moleküls oder eines Fragments davon. Darüber hinaus betrifft die Erfindung Antikörper, Nachweisverfahren und Test-Kits sowie pharmazeutische Zusammensetzungen.

SDF-1α (stromal cell derived factor 1α) und seine durch alternatives Spleißen entstehende Isoform SDF-1β wurden ursprünglich aus einer Zelllinie von Stromazellen des Knochenmarks der Maus kloniert (Tashiro et al, 1993). Auf der Grundlage der festgestellten Homologie der abgeleiteten Aminosäuresequenz zu den Sequenzen des Interleukins 8 (32%) und des Makrophagen-Entzündungsproteins 1α (32%) und der Anwesenheit von vier charakteristischen Cystein-Resten wurden SDF-1α und SDF-1β der Gruppe der

CXC(α)-Chemokine zugeordnet. Die CXC(α)-Chemokine sind eine Untergruppe der Familie der interkrinen Cytokine, die aus verschiedenen entfernt verwandten entzündungsfördemden Cytokinen besteht. Die cDNA-Sequenzen für SDF-1α und SDF-1β der Maus und des Menschen weisen eine starke Homologie zueinander auf und entstehen durch alternatives Spleißen eines einzigen Gens.

Die biologische Funktion von SDF-1 wurde anhand des menschlichen SDF-1α untersucht. SDF-1α ist für die Reifung von B-Zellen erforderlich, wirkt T-lymphotrop und induziert den Zelltod bei der neuronalen Zeillinie hNT. SDF-1α ist ein natürlicher Ligand des CXCR4(LESTR/Fusin)-Chemokin-Rezeptors von T-Zellen, der ein bindender Kofaktor von T-lymphotropen HIV-1-Stämmen ist. SDF-1α und -1β zeigen sowohl in vitro als auch in vivo ein "growth arrest"-spezifisches Expressionsmuster in Fibroblasten und Hepatozyten. Mäuse, bei denen das SDF-1-Gen inaktiviert wurde, zeigen eine verminderte Bildung von B-Zellen, einen Defekt des ventrikulären Septums und Defekte bei der Zellmigration im Kleinhirn, und sterben kurz nach der Geburt. SDF-1 könnte bei der Nervenregeneration eine wichtige Rolle spielen.

Der vorliegenden Erfindung liegt die Aufgabe zugrunde, neue Mittel zur Verfügung zu stellen, die gezielt zur Diagnose und/oder Behandlung von Erkrankungen, die in Zusammenhang mit einem Defekt des SDF-1-Faktors oder seines Rezeptors (CXCR4) stehen.

Diese Aufgabe wird erfindungsgemäß gelöst durch ein Nukleinsäure-Molekül, umfassend:
(1) eine für ein Chemokin, einen Neuropeptid-Präkursor oder mindestens ein Neuropeptid kodierende Nukleinsäuresequenz, die aus folgenden Sequenzen ausgewählt ist:
   (a) einer Nukleinsäuresequenz nach SEQ ID NO:1;
   (b) einer Nukleinsäuresequenz, die ein Polypeptid mit einer Aminosäuresequenz nach SEQ ID NO:2 kodiert;
   (c) einer Nukleinsäuresequenz, die mit der in (a) angegebenen Sequenz zu mindestens 80 % identisch ist;
   (d) einer Sequenz, die mit dem Gegenstrang der in (a) angegebenen Sequenz unter stringenten Bedingungen hybridisiert;
   oder
(2) eine komplementäre Sequenz zu einer der in (a) bis (d) angegebenen Nukleinsäuresequenzen.

Der Begriff "Polypeptid", wie nachfolgend in der Beschreibung verwendet, umfaßt aus 7 oder mehr Aminosäuren zusammengesetzte Peptide oder Proteine.

Der Begriff "Chemokin" steht für ein Mitglied einer Familie relativ kleiner Proteine, die auf der Basis der charakteristischen Anordnung von Cystein-Gruppen in vier Untergruppen unterteilt werden: C, CC, CXC und CX₃C. Die Chemokine binden an spezifische Rezeptoren (Rollins, B.J., 1997). In Zusammenhang mit der vorliegenden Erfindung bezieht sich der Begriff "Chemokin" insbesondere auf Mitglieder der CXC-Chemokinfamilie. Vorzugsweise handelt es sich bei dem Chemokin um ein Polypeptid-Molekül, das in einem Ca-Imaging-Experiment unter den in Köller et al (2001) beschriebenen Bedingungen einen 1,5 - 10-fachen Anstieg der intrazellulären Calcium-Konzentration in primären Astrozyten und/oder Neuronen aus dem Zentralnervensystem von Ratten oder Menschen hervorruft.

Als "Neuropeptide" werden biologisch aktive und physiologisch wichtige Signalmoleküle mit regulatorischen und modulatorischen Funktionen im Nervensystem bezeichnet. Die Funktionsbereiche umfassen unter anderem Neurotransmission, Rezeptormodulation,

Veränderung etektrophysiologischer Eigenschaften von Zellmembranen und metabolischer Prozesse. Neuropeptide werden von Neuronen synthetisiert und meist an der Synapse freigesetzt (Siegel et at., 1989).

Unter "Neuropeptid-Präkursor" wird ein Vorläuferprotein verstanden, das durch proteolytische Spaltung in ein aktives Neuropeptid überführt wird.

Die im erfindungsgemäßen Nukleinsäure-Molekül enthaltene Nukleinsäuresequenz kann eine genomische DNA, cDNA oder synthetische DNA sein, wobei unter synthetischen DNA-Sequenzen auch solche verstanden werden, die modifizierte Internukleosid-Bindungen enthalten.

In Zusammenhang mit den erfindungsgemäßen Nukleinsäure-Molekülen bezieht sich der Ausdruck "mindestens 80% identisch" auf Identität auf DNA-Ebene, die gemäß bekannter Verfahren, z.B. der computergestützten Sequenzvergleiche (Altschul et al., 1990) bestimmt werden kann.

Der dem Fachmann bekannte Ausdruck "Identität" bezeichnet den Grad der Verwandtschaft zwischen zwei oder mehr Nukleinsäuremolekülen, der durch die Übereinstimmung zwischen den Sequenzen bestimmt wird. Der Prozentsatz der "Identität" ergibt sich aus dem Prozentsatz identischer Bereiche in zwei oder mehr Sequenzen unter Berücksichtigung von Lücken oder anderen Sequenzbesonderheiten.

Die Identität miteinander verwandter Nukleinsäuremoleküle kann mit Hilfe bekannter Verfahren bestimmt werden. In der Regel werden spezielle Computerprogramme mit den besonderen Anforderungen Rechnung tragenden Algorithmen eingesetzt. Bevorzugte Verfahren zur Bestimmung der Identität erzeugen zunächst die größte Übereinstimmung zwischen den untersuchten Sequenzen. Computerprogramme zur Bestimmung der Identität zwischen zwei Sequenzen umfassen das GCG Programmpaket, einschließlich GAP (Devereux, J., et al., Nucleic Acids Research 12 (12): 387, 1984); Genetics Computer Group University of Wisconsin, Madison, (WI)); BLASTP, BLASTN und FASTA (Altschul et al., 1990), sind jedoch nicht auf diese eingeschränkt. Das BLASTX Programm kann vom National Centre for Biotechnology Information (NCBI) und aus weiteren Quellen bezogen werden (BLAST Handbuch, Altschul S., et al., NCB NLM NIH Bethesda MD 20894; Altschul et al., 1990). Auch der bekannte Smith Waterman-Algorithmus kann zur Bestimmung von Identität verwendet werden.

Bevorzugte Parameter für den Nukleinsäuresequenz-Vergleich umfassen die nachstehenden:

| | |
|---|---|
| Algorithmus | Needleman und Wunsch (1970) |
| Vergleichsmatrix | Übereinstimmung (matches) = + 10 |
| | Nichtübereinstimmung (mismatch) = 0 |
| Lücken-Wert (Gap Penalty) | 50 |
| Lückenlängen-Wert: (Gap Length Penalty) | 3 |

Das GAP-Programm ist auch zur Verwendung mit den vorstehenden Parametern geeignet. Die vorstehenden Parameter sind die Standardparameter (default parameters) für Nukleinsäuresequenz-Vergleiche.

Weitere beispielhafte Algorithmen, Lücken-Öffnungs-Werte (gap opening penalties), Lückenausdehnungs-Werte (gap extension penalties), Vergleichsmatrizen einschließlich der im Programm-Handbuch, Wisconsin-Paket, Version 9, September 1997, genannten können verwendet werden. Die Auswahl wird von dem durchzuführenden Vergleich abhängen und weiterhin davon, ob der Vergleich zwischen Sequenzpaaren, wobei GAP oder Best Fit bevorzugt sind, oder zwischen einer Sequenz und einer umfangreichen Sequenz-Datenbank, wobei FASTA oder BLAST bevorzugt sind, durchgeführt wird. Eine mit dem oben genannten Algorithmus ermittelte Übereinstimmung von 80% wird im Rahmen dieser Anmeldung als 80%ige Identität bezeichnet. Entsprechendes gilt für höhere Identitätsgrade.

Das Merkmal "Sequenz, die mit dem Gegenstrang der in (a) angegebenen Sequenz hybridisiert" weist auf eine Sequenz hin, die unter stringenten Bedingungen mit dem Gegenstrang der unter (a) angegebenen Sequenz hybridisiert. Beispielsweise können die Hybridisierungen bei 42°C mit einer Hybridisierungslösung bestehend aus 5 × SSPE, 5 × Denhardt's, 0,1% SDS, 100 µg/ml Lachssperma-DNA, 30-50% Formamid (Sambrook et al., 1989) durchgeführt werden. Als Waschschritt eignet sich eine zweimal wiederholte 10-15minütige Waschung in 2 x SSPE, 0,1% SDS bei 42°C, gefolgt von einer zweimal wiederholten 20minütigen Waschung in 0,2 x SSPE, 0,1 % SDS bei 50°C. Alternativ kann bei den Waschlösungen SSC anstelle von SSPE verwendet werden.

Überraschenderweise wurde nun gefunden, dass das erfindungsgemäße Nukleinsäure-Molekül ein neues Mitglied der SDF-Familie von Chemokinen darstellt, das im folgenden als SDF-1γ bezeichnet wird. Die Klonierung und Charakterisierung der SDF-1γ-cDNA sowie die Nukleinsäuresequenz und die davon abgeleitete Aminosäuresequenz für das humane SDF-1γ und das SDF-1γ von Ratte werden in den Beispielen beschrieben.

Die SDF-1γ-Nukleinsäuresequenz besteht aus der vollständigen Nukleinsäuresequenz von SDF-1β und einer zusätzlichen Sequenz von 2572 Nukleotiden, die im gleichen Leseraster stromabwärts an das Codon 89 von SDF-1β anschließt. Durch dieses Insert ergibt sich für das neue SDF-1γ-Polypeptid eine Aminosäuresequenz mit 119 Aminosäuren und einem theoretischen Molekulargewicht von 13,6 Kd, wobei die Sequenz am Carboxyterminus im Vergleich zu der bekannten SDF-1α-Sequenz um 30 Aminosäuren verlängert ist. Es wird vermutet, dass SDF-1γ durch Insertion eines neuen alternativen Exons IIIa zwischen den bekannten Exons III und IV (vgl. Shirozu et al., 1995) erzeugt wird.

Im Carboxy-terminalen Bereich der Aminosäuresequenz von SDF-1γ können 5 Gruppen aus zwei basischen Aminosäuren (Lys-Lys, Arg-Arg und Lys-Arg) Erkennungsmuster für eine Membran-gebundenen Protease des Golgi-Systems und der Sekretionsvesikel bilden. Durch proteolytische Spaltung an diesen Stellen entstehen fünf kurze Peptide (SEQ ID NO:5, SEQ ID NO:6, SEQ ID NO:8, SEQ ID NO:9, SEQ ID NO:10) und ein verkürztes Protein (SEQ ID NO:7), von denen zwei Peptide und ein Polypeptid (SEQ ID NO:5, SEQ ID NO:6 und SEQ ID NO:7) einen Carboxy-terminalen Glycin-Rest aufweisen. Peptide mit einem Glycin-Rest am Carboxyterminus sind potentielle Substrate für die Peptidyl-α-amidierende Monooxigenase (PAM), die die Carboxy-terminale Spaltung von Carboxylat katalysiert und dadurch α-amidierte Carboxytermini (CONH₂) erzeugt, die für Neuropeptide charakteristisch sind (siehe Übersicht von Eipper et al., 1992).

Das SDF-1β-Transkript von Ratte (siehe SEQ ID NO:16) kodiert ein Protein mit 93 Aminosäuren (siehe SEQ ID NO:17) und einem theoretischen Molekulargewicht von 10,5 Kd, wobei die ersten 19 Aminosäuren eine Signalsequenz für sezemierte Proteine darstellen. Die ersten 17 Aminosäuren des reifen Proteins, die in allen Isoformen vorkommen, werden für die Bindung an den CXCR4-Rezeptor benötigt (vgl. Loetscher et al. 1998; Doranz et al., 1999). Zwei basische Aminosäuren (Lys 89 und Arg 90) im Carboxy-terminalen Bereich liefern ein Erkennungsmuster für die proteolytische Spaltung, aus der sich ein Pentapeptid (Lys 89-Met 93, SEQ ID NO:18) und ein verkürztes Protein ergeben.

In einer Ausführungsform der Erfindung umfasst das erfindungsgemäße Nukleinsäure-Molekül eine Nukleinsäuresequenz, die mit der Nukleinsäuresequenz nach SEQ ID NO:1 zu mindestens 80%, vorzugsweise zu mindestens 90%, besonders bevorzugt zu mindestens 95% identisch ist.

Nukleinsäure-Moleküle, die eine Nukleinsäuresequenz nach SEQ ID NO:3 oder eine für ein Polypeptid mit einer Aminosäuresequenz nach SEQ ID NO:4 kodierende Nukleinsäuresequenz umfassen, sind besonders bevorzugt.

Das erfindungsgemäße Nukleinsäure-Molekül kann weiterhin einen zur Expression geeigneten Promotor umfassen, wobei die kodierende Nukleinsäuresequenz unter der Kontrolle des Promotors steht. Ein "zur Expression geeigneter Promotor", wie hier verwendet, bezeichnet ein DNA-Fragment, durch das der Initiationspunkt und die Initiationshäufigkeit der Transkription (RNA-Synthese) einer unter der Kontrolle des Promotorelements stehenden Nukleinsäuresequenz, die ein Chemokin, einen Neuropeptid-Präkursor oder mindestens ein Neuropeptid kodiert, im Wirtsorganismus festgelegt werden. Die Wahl des Promotors hängt vom zur Expression verwendeten Expressionssystem ab. Generell sind konstitutive Promotoren bevorzugt, jedoch sind auch induzierbare Promotoren, wie z. B. der Metallothionein-Promotor, möglich. Zur Ausführung der Erfindung in Betracht kommende Promotoren schließen unter anderem die *FMD-, MOX-, TPS1-, PMA1-* und *DAS*-Promotoren aus *Hansenula polymorpha*, die *AOX1*- und *GAP1*-Promotoren aus *Pichia pastoris*, die *ADH1*-, *PDC1*-, *GAP1-* und *CUP1*-Promotoren aus *S. cerevisiae*, die *AXDH*- und *AHSB4*-Promotoren von *Arxula adeninovorans* und die *NDK1*- und *CPC2*-Promotoren von *Sordaria macrospora* ein.

Das erfindungsgemäße Nukleinsäure-Molekül kann darüber hinaus Sequenzen eines Vektors umfassen, die die Replikation des Nukleinsäure-Moleküls in einer Wirtszelle und/oder die Integration des Nukleinsäure-Moleküls in das Genom einer Wirtszelle ermöglichen. Im Stand der Technik sind zahlreiche Klonierungs- und Expressions-Vektoren bekannt, vgl. Recombinant Gene Expression Protocols, Meth. Mol. Biol. Vol. 62, Humana Press, New Jersey, USA. Zur Replikation in einer Wirtszelle muß der verwendete Vektor einen Replikationsursprung und gegebenenfalls weitere regulatorische Regionen enthalten. Der Vektor kann aus Bakteriophagen wie λ-Derivaten, Adenoviren, Vacciniaviren, Baculoviren, SV40-Virus, Retroviren; Plasmiden, wie Ti-Plasmiden von *Agrobacterium tumefaciens*, YAC-Vektoren und BAC-Vektoren ausgewählt sein.

Gegenstand der vorliegenden Erfindung ist ferner eine Wirtszelle, die mindestens ein erfindungsgemäßes Nukleinsäure-Molekül enthält, wobei die Wirtszelle eine zur Expression des Nukleinsäure-Moleküls und gegebenenfalls zur Prozessierung des entstandenen Polypeptid-Moleküls geeignete prokaryontische oder eukaryontische Zelle ist. Im Stand der Technik sind zahlreiche prokaryontische und eukaryontische Expressionssysteme bekannt. Wirtszellen können beispielsweise aus prokaryontischen Zellen, wie *E. coli* oder *B. subtilis*, oder aus eukaryontischen Zellen, wie Pilzzellen, Pflanzenzellen, Insektenzellen und Säugerzellen, z. B. CHO-Zellen, COS-Zellen oder HeLa-Zellen, sowie Derivaten davon ausgewählt sein. Im Stand der Technik sind beispielsweise bestimmte CHO-Produktionslinien bekannt, deren Glykosylierungsmuster im Vergleich zu CHO-Zellen verändert sind. Vorzugsweise ist die eukaryontische Wirtszelle die Hefe *Saccharomyces cerevisiae*, die methylotrophe Hefe *Hansenula polymorpha*, die dimorphe Hefe *Arxula adeninivorans* oder der filamentöse Pilz *Sordaria macrospora*.

Die Erfindung stellt ferner ein Polypeptid-Molekül bereit, das eine Aminosäuresequenz umfasst, die aus folgenden Sequenzen ausgewählt ist:
(i) einer Aminosäuresequenz, die eine Aminosäuresequenz nach SEQ ID NO:5 und/oder eine Kombination aus zwei oder mehreren der Aminosäuresequenzen SEQ ID NO:5, SEQ ID NO:6, SEQ ID NO:8, SEQ ID NO:9 und SEQ ID NO:10 umfasst;
(ii) einer Aminosäuresequenz nach SEQ ID NO:4;
(iii) einer Aminosäuresequenz, die der Sequenz von Aminosäure 20 bis Aminosäure 119 in SEQ ID NO:4 entspricht;
(iv) einer Aminosäuresequenz nach SEQ ID NO:22;
(v) einer Aminosäuresequenz, die mit der in (i), (ii) oder (iv) angegebenen Sequenz zu mindestens 85 % identisch ist;
(vi) einer Aminosäuresequenz, die eine Sequenz nach SEQ ID NO:6 oder eine Sequenz umfasst, die mit dieser zu mindestens 85 % identisch ist, ausgenommen folgende Aminosäuresequenz:
(vii) einer Aminosäuresequenz, die eine Sequenz nach SEQ ID NO:7, eine Kombination aus SEQ ID NO:7 und einer oder mehreren der Aminosäuresequenzen SEQ ID NO:5, SEQ ID NO:6, SEQ ID NO:8, SEQ ID NO:9 und SEQ ID NO:10 oder einer Sequenz umfasst, die mit einer Kombination aus dieser Sequenz und einer oder mehreren d3er vorgenannten Sequenzen zu mindestens 95 % identisch ist.
(viii) einer Aminosäuresequenz, die eine Sequenz nach SEQ ID NO:8 oder eine Sequenz umfasst, die mit dieser zu mindestens 85% identisch ist, ausgenommen die Aminosäuresequenz: und die Aminosäuresequenz: und die Aminosäuresequenz:
(ix) einer Aminosäuresequenz, die eine Sequenz nach SEQ ID NO:9 oder eine Sequenz, die mit dieser zu mindestens 90% identisch ist, umfasst;
(x) einer Aminosäuresequenz, die eine Sequenz nach SEQ ID NO:10 umfasst, ausgenommen folgende Aminosäuresequenz: worin das Polypeptidmolekül ein Chemokin oder ein Neuropeptid ist.

In diesem Zusammenhang bezieht sich der Ausdruck "mindestens 85% identisch" auf Übereinstimmung auf der Ebene der Aminosäuresequenz, die gemäß bekannter Verfahren, z.B. der computergestützten Sequenzvergleiche (Altschul et al., 1990) bestimmt werden kann.

Der Ausdruck "Identität" bezeichnet hier den Grad der Verwandtschaft zwischen zwei oder mehreren polypeptid-Molekülen, der durch die Übereinstimmung zwischen den Sequenzen bestimmt wird, wobei unter Übereinstimmung sowohl identische Übereinstimmung als auch konservativer Aminosäure-Austausch zu verstehen ist. Der Prozentsatz der "Identität" ergibt sich aus dem Prozentsatz übereinstimmender Bereiche in zwei oder mehr Sequenzen unter Berücksichtigung von Lücken oder anderen Sequenzbesonderheiten.

Der Begriff "konservativer Aminosäure-Austausch" bezieht sich auf einen Austausch eines Aminosäurerestes durch einen anderen Aminosäurerest, wobei der Austausch einen möglichst geringen Einfluß auf die (räumliche) Struktur des Polypeptid-Moleküls ausüben soll. Grundsätzlich werden vier physiko-chemische Gruppen unterschieden, in die die natürlicherweise vorkommenden Aminosäuren eingeteilt werden. Zur Gruppe der basischen Aminosäuren gehören Arginin, Lysin und Histidin. Zur Gruppe der sauren Aminosäuren gehören Glutaminsäure und Asparaginsäure. Die ungeladenen/polaren Aminosäuren umfassen Glutamin, Asparagin, Serin, Threonin und Tyrosin. Die nichtpolaren Aminosäuren umfassen Phenylalanin, Tryptophan, Cystein, Glycin, Alanin, Valin, Methionin, Isoleucin, Leucin und Prolin. Ein konservativer Aminosäure-Austausch bedeutet in diesem Zusammenhang den Austausch einer gegebenen Aminosäure durch eine Aminosäure, die zur gleichen physiko-chemischen Gruppe gehört.

Die Identität miteinander verwandter Polypeptid-Moleküle kann mit Hilfe bekannter Verfahren bestimmt werden. Bevorzugte Verfahren zur Bestimmung der Identität erzeugen zunächst die größte Übereinstimmung zwischen den untersuchten Sequenzen. Computerprogramme zur Bestimmung der Identität zwischen zwei Aminosäuresequenzen umfassen das GCG-Programmpaket, einschließlich GAP (Devereux et al., 1984; Genetics Computer Group University of Wisconsin, Madison, (WI)); BLASTP, BLASTN und FASTA (Altschul et al., 1990), sind jedoch nicht auf diese eingeschränkt. Das BLAST X Programm kann vom National Centre for Biotechnology Information (NCBI) und aus weiteren Quellen bezogen werden (BLAST Handbuch, Altschul, S. et al., NCB NLM NIH Bethesda MD 20894; Altschul et al., 1990). Auch der bekannte Smith Waterman-Algorithmus kann zur Bestimmung von Sequenzidentität verwendet werden.

Bevorzugte Parameter für den Sequenz-Vergleich umfassen die nachstehenden:

| | |
|---|---|
| Algorithmus | Needleman und Wunsch (1970) |
| Vergleichsmatrix | BLOSUM 62 von Henikoff und Henikoff (1992) |
| Lücken-Wert (Gap Penalty) | 12 |
| Lückenlängen-Wert (Gap Length Penalty) | 4 |
| Schwellenwert der Ähnlichkeit | 0 |

Das GAP-Programm ist auch zur Verwendung mit den vorstehenden Parametern geeignet. Die vorstehenden Parameter sind die Standardparameter (default parameters) für Aminosäuresequenz-Vergleiche, wobei Lücken an den Enden den Identität-Wert nicht verringern. Bei sehr kurzen Sequenzen im Vergleich zur Referenzsequenz kann es weiterhin notwendig sein, den Erwartungswert (expected value) auf bis zu 100.000 zu erhöhen und gegebenenfalls die Wortlänge (word size) auf bis zu 2 zu verkleinern.

Weitere beispielhafte Algorithmen, Lücken-Öffnungs-Werte (gap opening penalties), Lückenausdehnungs-Werte (gap extension penalties), Vergleichsmatrizen einschließlich der im Programm-Handbuch, Wisconsin-Paket, Version 9, September 1997, genannten können verwendet werden. Die Auswahl wird von dem durchzuführenden Vergleich abhängen und weiterhin davon, ob der Vergleich zwischen Sequenzpaaren, wobei GAP oder Best Fit bevorzugt sind, oder zwischen einer Sequenz und einer umfangreichen Sequenz-Datenbank, wobei FASTA oder BLAST bevorzugt sind, durchgeführt wird.

Eine mit dem oben genannten Algorithmus ermittelte Übereinstimmung von 85% wird im Rahmen dieser Anmeldung als 85%ige Identität bezeichnet. Entsprechendes gilt für höhere Identitätsgrade.

In einer Ausführungsform der Erfindung umfasst das erfindungsgemäße Polypeptid-Molekül eine Sequenz, die mit der vorstehend unter (i), (ii), (iii) oder (iv) angegebenen Aminosäuresequenz zu mindestens 90%, vorzugsweise zu mindestens 95% identisch ist. Besonders bevorzugt sind Polypeptid-Moleküle, die eine Aminosäuresequenz nach SEQ ID NO:12 oder SEQ ID NO:13 umfassen.

In einer Ausführungsform der Erfindung umfaßt das erfindungsgemäße Polypeptid-Molekül die Aminosäuresequenzen nach SEQ ID NO:5, SEQ ID NO:6 und/oder SEQ ID NO:7. Bevorzugt sind Polypeptid-Moleküle gemäß der Erfindung, die die Aminosäuresequenzen nach SEQ ID NO:5 und SEQ ID NO:6 umfassen.

In einer weiteren Ausführungsform stellt die Erfindung ein Fusionsprotein bereit, das mindestens ein erfindungsgemäßes Polypeptid umfasst.

Fragmente der erfindungsgemäßen Polypeptid-Moleküle, die mindestens ein Neuropeptid umfassen, werden von der Erfindung ebenfalls umfasst. Bevorzugt sind Fragmente, die mindestens eine der Aminosäuresequenzen nach SEQ ID NO:5, SEQ ID NO:6, SEQ ID NO:7, SEQ ID NO:8, SEQ ID NO:9 und/oder SEQ ID NO:10 umfassen. Besonders bevorzugt sind Fragmente mit der Aminosäuresequenz nach SEQ ID NO:5, SEQ ID NO:6 oder SEQ ID NO:7. Die Fragmente der erfindungsgemäßen Polypeptid-Moleküle können auch modifiziert sein, beispielsweise durch Glykosilierung, Phosphorylierung, Acetylierung oder Amidierung.

Vorzugsweise handelt es sich bei dem erfindungsgemäßen Polypeptid-Molekül, Fusionsprotein oder Fragment um ein Polypeptid-Molekül, das in einem Ca-Imaging-Experiment unter den in Köller et al (2001) beschriebenen Bedingungen einen 1,5-10-fachen Anstieg der intrazellulären Calcium-Konzentration in primären Astrozyten und/oder Neuronen aus dem Zentralnervensystem von Ratten oder Menschen hervorruft.

Gegenstand der Erfindung ist weiterhin ein Verfahren zum Herstellen eines erfindungsgemäßen Polypeptid-Moleküls und/oder eines Fragments davon, das das Kultivieren einer erfindungsgemäßen Wirtszelle unter zur Expression und eventuellen Prozessierung geeigneten Bedingungen und gegebenenfalls das Aufreinigen des exprimierten Polypeptid-Moleküls oder Fragments umfasst. Alternativ können die erfindungsgemäßen Polypeptid-Moleküle und Fragmente davon auch durch chemische und enzymatische Synthese, wie beispielsweise Merrifield-Synthese, und/oder Fragmentkondensation erhalten werden. Kombinationen von chemischen, enzymatischen und rekombinanten Herstellungsverfahren kommen ebenfalls in Betracht.

Ein weiterer Gegenstand der Erfindung ist ein Antikörper, der für ein erfindungsgemäßes Polypeptid-Molekül und/oder ein Fragment davon spezifisch ist. Allgemein sind spezifische Antikörper durch Immunisieren von Versuchstieren, wie z. B. Mäusen oder Kaninchen, mit den erfindungsgemäßen Polypeptid-Molekülen und/oder Fragmenten, die vorzugsweise an geeignete hochmolekulare Trägermoleküle (häufig Proteine) gekoppelt sind, erhältlich. Das Immunisieren kann hierbei durch den Zusatz geeigneter im Stand der Technik bekannter Adjuvanzien erleichtert werden. Monoklonale Antikörper sind üblicherweise durch Fusionieren von Milzzellen, die aus einer immunisierten Maus entnommen wurden, mit Tumorzellen und Selektionieren der dabei entstehenden Hybridome erhältlich. Diejenigen Hybridome, die effizient spezifische Antikörper sezemieren, können hierbei durch Absuchen des Überstandes bestimmt werden. Alternativ können Antikörper rekombinant hergestellt werden; bei der Herstellung rekombinanter Antikörper wird die mRNA aus Hybridomazellen oder B-Lymphozyten isoliert, die als Grundlage für die Synthese der entsprechenden cDNA fungiert und über PCR amplifiziert wird. Nach der Ligation in einen geeigneten Vektor und der Einführung in eine geeignete Wirtszellkultur läßt sich der Antikörper aus den Zellkulturüberständen oder den Zelllysaten gewinnen. Rekombinante Antikörper erlauben eine "Humanisierung" des Antikörpers und sind dadurch weniger immunogen. Die diesbezüglichen Verfahren sind im Stand der Technik bekannt.

Zum Nachweisen eines erfindungsgemäßen Polypeptid-Moleküls und/oder Fragments davon in einer biologischen Probe stellt die Erfindung ein in vitro-Verfahren bereit, das das Inkontaktbringen der Probe mit einem für das Polypeptid-Molekül und/oder für ein Fragment davon spezifischen Reagenz und das Nachweisen der Bindung umfasst.

Die Erfindung stellt ferner einen Test-Kit zum Nachweisen eines Polypeptids und/oder eines Fragments davon bereit, der mindestens ein für das erfindungsgemäße Polypeptid und/oder Fragment spezifisches Reagenz umfasst. Ein Beispiel solcher spezifischen Reagenzien sind Antikörper, Antikörperfragmente, z. B. Fab oder F(ab)₂-Fragmente oder Antikörperderivate, wobei Antikörper besonders bevorzugt sind. Die Antikörper, Antikörperfragmente, z. B. Fab oder F(ab)₂-Fragmente oder Antikörperderivate können monoklonalen oder polyklonalen Ursprungs sein.

Ferner stellt die Erfindung ein in vitro-Verfahren zum Nachweisen einer ein erfindungsgemäßes Polypeptid-Molekül kodierenden Nukleinsäure in einer biologischen Probe bereit, das:
- das Inkontaktbringen der Probe mit einem erfindungsgemäßen Nukleinsäure-Molekül und/oder Fragment davon, wobei das Nukleinsäure-Molekül und/oder das Fragment eine nachweisbare Markierung tragen, und
- das Nachweisen der Markierung
umfasst.

Gegenstand der Erfindung sind ferner pharmazeutische Zusammensetzungen, die mindestens ein erfindungsgemäßes Polypeptid-Molekül und/oder ein Fragment davon und/oder ein pharmazeutisch verträgliches Salz eines solchen Polypeptid-Moleküls oder Fragments enthalten. Diese pharmazeutischen Zusammensetzungen können einen pharmakologisch verträglichen Träger und/oder Verdünnungsmittel enthalten. Geeignete Träger und/oder Verdünnungsmittel sind im Stand der Technik bekannt. Bevorzugt sind pharmazeutische Zusammensetzungen, die sich zur intravenösen, subkutanen oder intramuskulären Verabreichung eignen. In einer Ausführungsform der erfindungsgemäßen pharmazeutischen Zusammensetzungen enthält die Zusammensetzung neben einem oder mehreren erfindungsgemäßen Polypeptid-Molekülen oder Fragmenten derselben auch mindestens einen Antikörper.

Erfindungsgemäß können diese pharmazeutischen Zusammensetzungen zur Therapie von demyelinisierenden oder neurodegenerativen Erkrankungen oder von Entwicklungsstörungen des Nervensystems eingesetzt werden. Eine weitere Anwendung der erfindungsgemäßen pharmazeutischen Zusammensetzungen ist auf die Vorbeugung oder Behandlung einer HIV-Infektion und insbesondere einer HIV-Enzephalopathie in Menschen gerichtet. Von der Erfindung wird ebenfalls die Anwendung der erfindungsgemäßen pharmazeutischen Zusammensetzungen zur Therapie von Erkrankungen des hämatopoietischen Systems, des Immunsystems und des Herz/Kreislaufsystems umfaßt.

Pharmazeutische Zusammensetzungen, die mindestens eine (n) für ein erfindungsgemäßes Polypeptid-Molekül und/oder ein Fragment davon spezifische Nukleinsäuresonde und/oder Antikörper enthalten, werden von der Erfindung ebenfalls umfasst. Solche pharmazeutische Zusammensetzungen können zur Diagnose oder Therapie von demyelinisierenden oder neurodegenerativen Erkrankungen oder von Entwicklungsstörungen des Nervensystems eingesetzt werden. Eine weitere Anwendung betrifft die Diagnose oder Behandlung einer HIV-Infektion und insbesondere einer HIV-Enzephalopathie in Menschen.

Die folgenden Figuren und Beispiele erläutern die Erfindung:

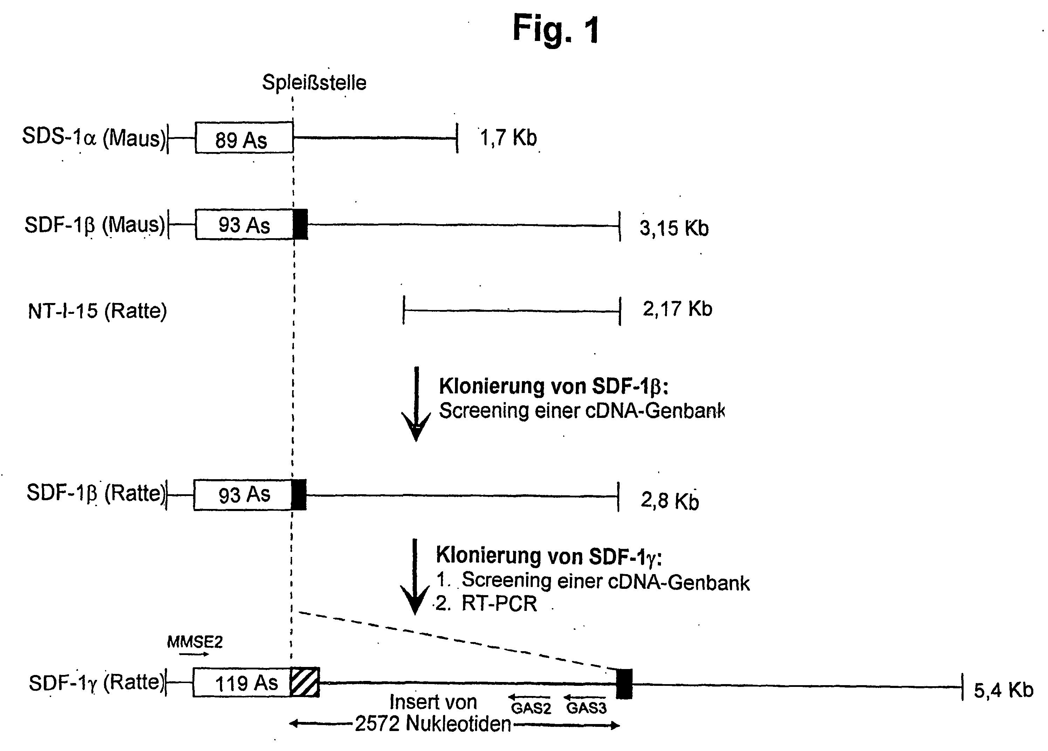

Figur 1 zeigt die auf RT-PCR beruhende Strategie zur Klonierung von SDF-1β- und SDF-1γ-cDNA aus Ratte. 5'- und 3'-UTR-Bereiche werden durch Linien, kodierende Bereiche durch Kästchen dargestellt. Kleine Pfeile zeigen die Position und Orientierung der Primer. Identische oder homologe Sequenzen werden durch identische graphische Elemente dargestellt. Die letzten vier Codons der kodierenden Region von SDF-1β werden als schwarzes Kästchen dargestellt. Die Strichlinien markieren ein Insert von 2572 Nukleotiden in SDF-1γ. Die 30 carboxyterminalen Codons von SDF-1γ werden als schraffiertes Kästchen dargestellt.

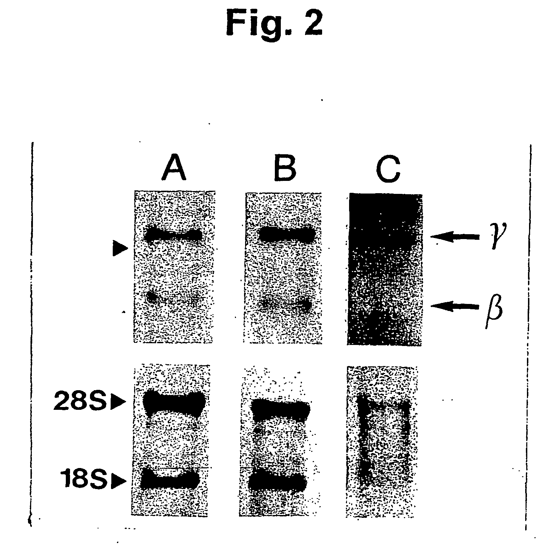

Figur 2 zeigt das Ergebnis eines Northem-Blot-Versuchs zum Nachweis von SDF-1 β- und SDF-1γ-Transkripten im Ischiasnerv von erwachsenen Ratten. Die Filter wurden (A) mit einem radioaktiven markierten, 626 Nukleotiden langen PCR-Fragment der NT-I-15-cDNA, das den Nukleotiden 743-1368 von SDF-1β entspricht und Teil der SDF-1β und SDF-1γ gemeinsamen 3'-UTR-Sequenz ist, (B) mit einem 190 Nukleotide langen PCR-Fragment der allen SDF-1-Isoformen gemeinsamen kodierenden Region, das den Nukleotiden 49-239 in der SEQ ID NO:16 entspricht, (C) mit einem 1702 Nukleotide langen Fragment, das den Nukleotiden 625-2327 der SDF-1γ-cDNA entspricht und von dem SDF-1γ-spezifischen Insert durch Verdau des mit den Primern GAS2 und MMSE2 erhaltenen PCR-Produktes mit *Pvu*II entstand, hybridisiert.
Figur 3 zeigt die Nukleinsäuresequenzen sowie die davon abgeleitete Aminosäuresequenzen der SDF-1β- und SDF-1γ-cDNA aus Ratte. Das SDF-1γ-spezifische Insert ist durch einen Rahmen hervorgehoben. Die Nukleinsäuresequenz des gemeinsamen Signalpeptids ist unterstrichen. Die Numerierung der Nukleotide (links) und der Aminosäuren (rechts) entspricht der Sequenz von SDF-1γ. (93) kennzeichnet die letzte Aminosäure von SDF-1β.
Figur 4 zeigt einen Vergleich der Aminosäuresequenzen der SDF-1 Proteine von Maus und Ratte. Die Punkte stehen für identische Aminosäuren. Das 19 Aminosäuren lange Signalpeptid ist umrahmt.

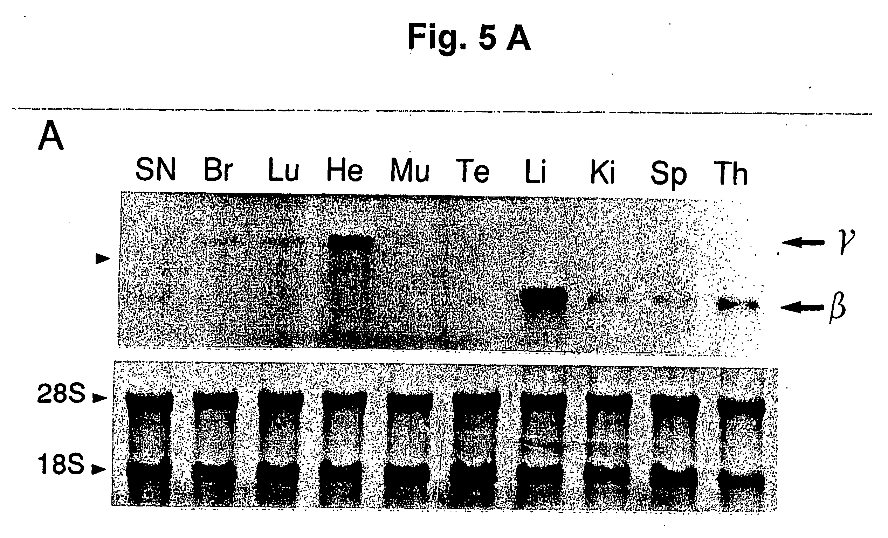

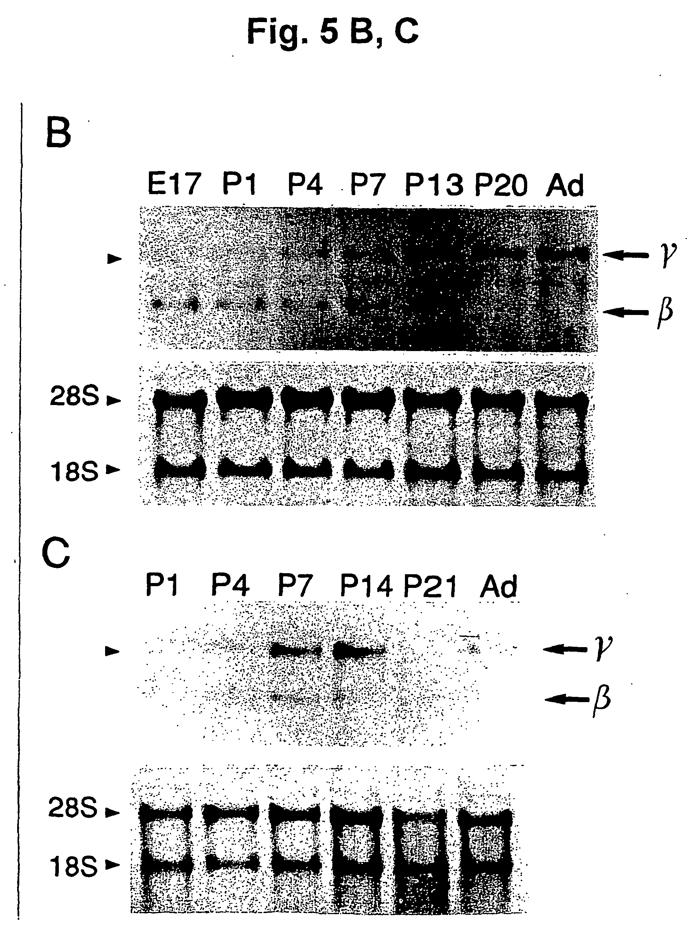

Figur 5 zeigt das Ergebnis verschiedener Northem-Blot-Versuche zum Nachweis von SDF-1β- und SDF-1γ-Transkripten in verschiedenen Geweben (A) und Entwicklungsstadien (B). (A) Northem-Blot-Filter mit Gesamt-RNA aus Ischiasnerv (SN), Him (Br), Lunge (Lu), Herzen (HE), Muskel (Mu), Hoden (Te), Leber (Li), Niere (Ki), Milz (Sp) und Thymus (Th) von Ratten wurden mit einer radioaktiv markierten cDNA-Sonde aus dem SDF-1β und SDF-1γ gemeinsamen 3'-UTR-Bereich hybridisiert. (B) Nachweis von SDF-1β- und SDF-1γ-mRNA im Him von Ratten während der Entwicklung. Northern-Blot-Filter mit Gesamt-RNA aus dem Hirn von 17 Tage alten Rattenembryonen (E 17) sowie von Ratten 1, 4, 7, 13 und 20 Tage nach der Geburt (P1-20) und von erwachsenen Tieren (Ad) wurden wie bei (A) hybridisiert. (C) Nachweis von SDF-β- und SDF-1γ-mRNA im Ischiasnerv von Ratten im Laufe der Entwicklung. Northern-Blot-Filter mit Gesamt-RNA, die aus Nerven von 1, 4, 7, 14 und 21 Tage alten Ratten (P1-21) und von erwachsenen Ratten (Ad) gewonnen wurde, wurden mit einer SDF-1 β/γ-Sonde wie bei (A) hybridisiert. Die Pfeilspitze in dem oberen Teil zeigt die Position der ribosomalen 28S-RNA; die unteren Teile zeigen mit Methylenblau gefärbte Northern-Blot-Filter.

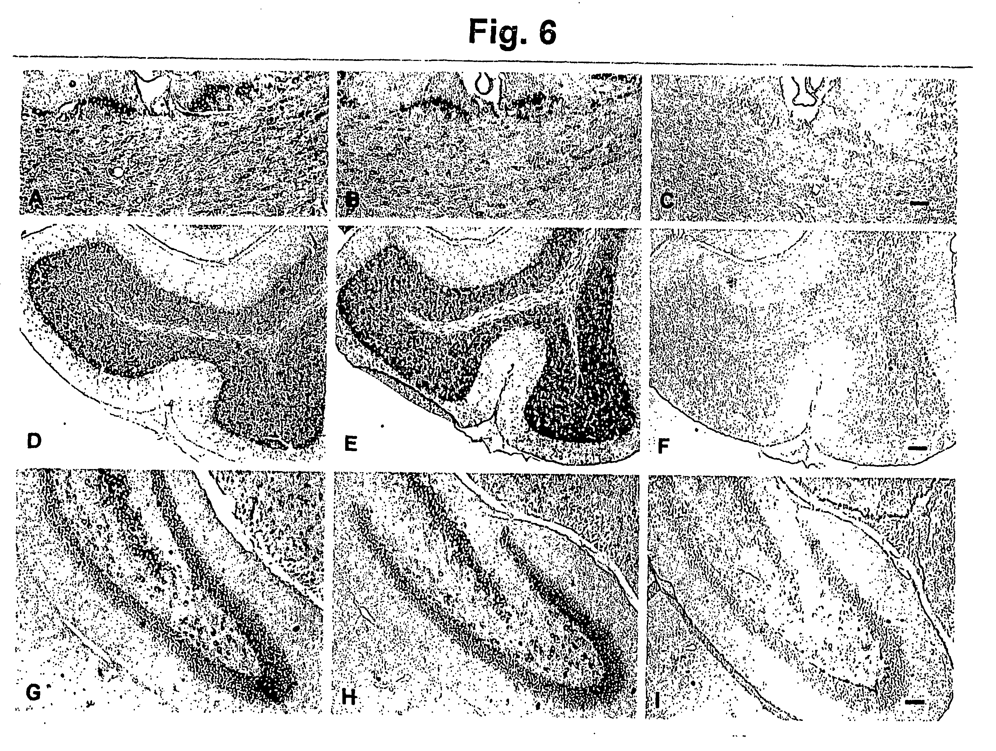

Figur 6 zeigt das Ergebnis von *in situ*-Hybridisierungsversuchen zur zellulären Lokalisierung von SDF-1γ-mRNA im Hirn von erwachsenen Ratten. Die Schnitte wurden mit einem Digoxigenin-UTP-markierten, 596 Nukleotide langen Antisense-Transkript, das aus einem Subklon der allen SDF-1-mRNA-Isoformen gemeinsamen 5'-UTR- und kodierenden Sequenz (A, D, E) bzw. von einem Subklon des SDF-1γ-spezifischen Inserts (B, E, H) abgeleitet ist, inkubiert. Als negative Kontrolle diente die Hybridisierung mit Sense-Transkripten (C, F, I). (A, B, C) *Corpus callosum* mit "Perlenkette"-ähnlicher Anreihung von markierten Oligodendrozyten und stark markierten Neuronen in den bilateralen Schichten des *Indusium griseum dorsale* des *Corpus callosum*. (D, E, F) Starke Hybridisierungssignale werden in Purkinje- und Granulärzellen des Kleinhims und schwache Signale in der äußeren Schicht beobachtet. (G, H, I) Sehr starke Hybridisierungssignale werden in pyramidalen und granularen Neuronen des Hippokampus nachgewiesen. Strich: 10 µm.
Figur 7 zeigt das Ergebnis von *in situ*-Hybridisierungsversuchen zur zellulären Lokalisierung von SDF-1γ-mRNA im Neocortex von Ratten unter Verwendung der gleichen Sonden wie in Figur 6. Bei Schnitten aus den frontolateralen (A) und mediolateralen (B) Bereichen des Neocortex sind die Neuronen in sämtlichen Neocortex-Schichten (I - VI) sowohl mit der allen SDF-1-mRNA-Isoformen gemeinsamen Antisense-Sonde (A) als auch der SDF-1γ-spezifischen Sonde (B) stark markiert.

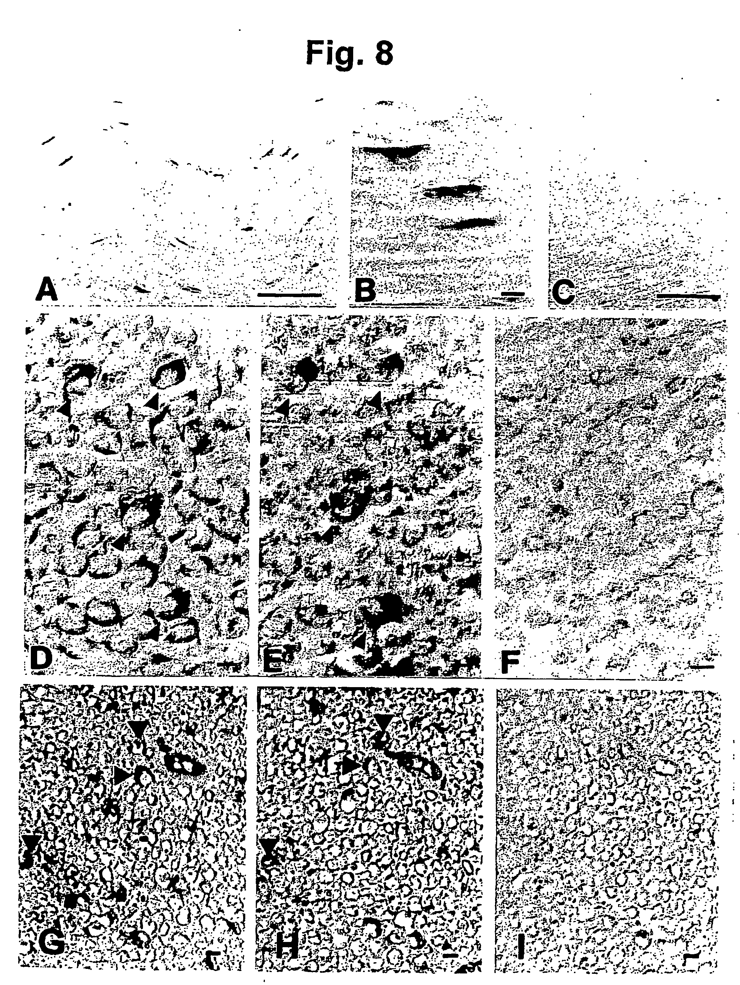

Figur 8 zeigt das Ergebnis von *in situ*-Hybridisierungsversuchen zur zellulären Lokalisierung von SDF-1γ-mRNA im Ischiasnerv von erwachsenen Ratten. Die Schnitte wurden mit Digoxigenin-UTP-markierten RNA-Sonden in Sense- und Antisense-Orientierung hybridisiert, die (a) von dem SDF-1β und SDF-1γ gemeinsamen 3'-UTR-Bereich (A-C, E, F), (b) von den allen SDF-1-Isoformen gemeinsamen 5'-UTR- und kodierenden Bereichen (G) und von dem SDF-1γ-spezifischen Insert (H, I) abgeleitet wurden. (A, B, C) Der Längsschnitt zeigt mehrere spindelförmige Schwann-Zellen in der Nähe der Axone. (D) Mit einem Antikörper gegen das S100-Protein (einen Marker für Schwann-Zellen) immungefärbter Querschnitt. (E) Die Hybridisierungssignale bei einem dem Querschnitt in (D) benachbarten Querschnitt zeigen halbkreisförmige, die Axone umgebende markierte Schwann-Zellen, die an den gleichen Stellen wie die S100-immunpositiven Zellen auftreten (Pfeilspitzen in D, E). (G, H) Mit je einem der beiden Antisense-Transkripten markierte benachbarte Querschnitte, die zahlreiche halbkreisförmige Schwann-Zellen (siehe Pfeilspitzen) und die Wand eines Blutgefäßes in der oberen rechte Ecke zeigen. (C, F, I) Als negative Kontrolle diente die Hybridisierung mit Transkripten in Sense-Orientierung. Striche in A, C, G-I: 100 µm, in D-F: 10 µm.

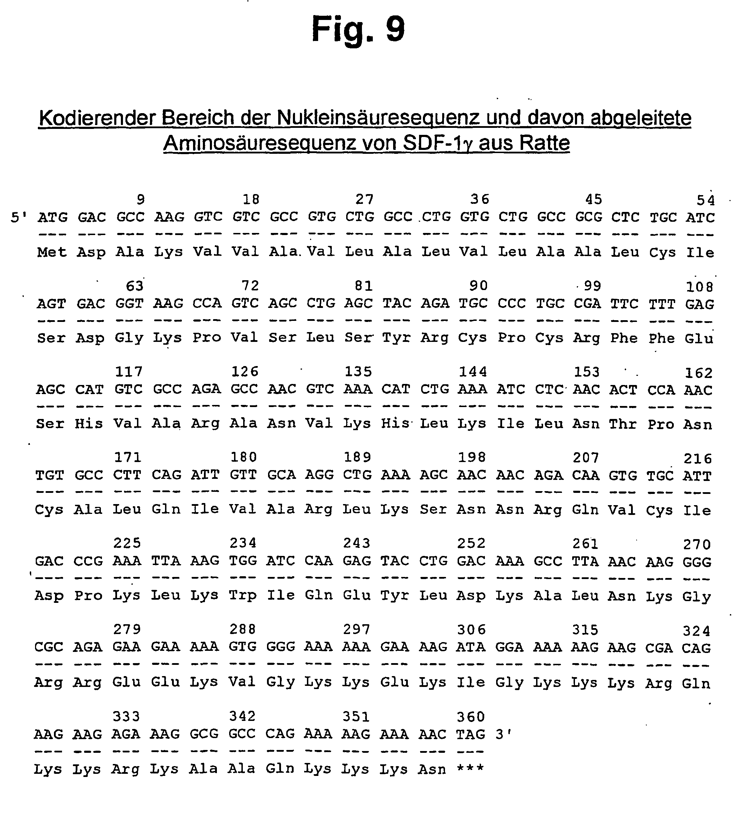

Figur 9 zeigt den kodierenden Bereich der Nukleinsäuresequenz von SDF-1γ aus Ratte und die davon abgeleitete Aminosäuresequenz.

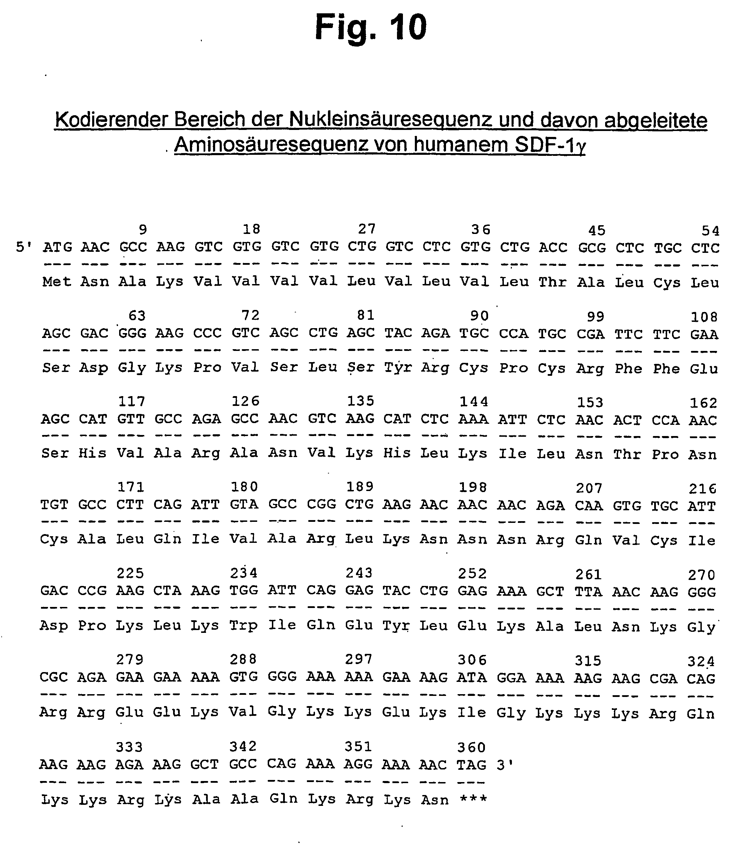

Figur 10 zeigt den kodierenden Bereich der Nukleinsäuresequenz von humanem SDF-1γ und die davon abgeleitete Aminosäuresequenz.

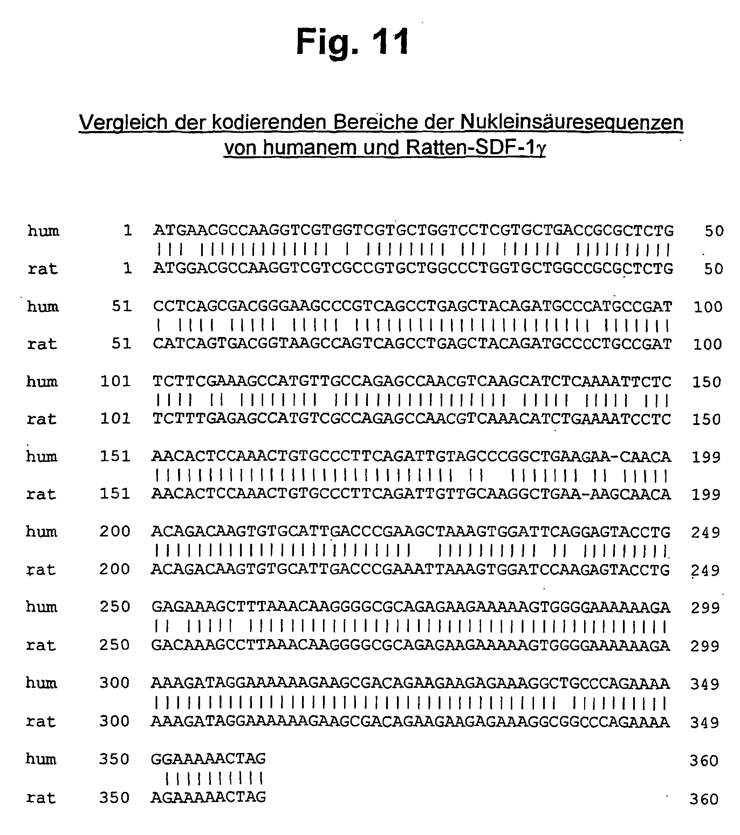

Figur 11 zeigt einen Vergleich der kodierenden Bereiche der Nukleinsäuresequenz von humanem und Ratten-SDF-1γ. "hum": Humansequenz; "rat": Ratte.

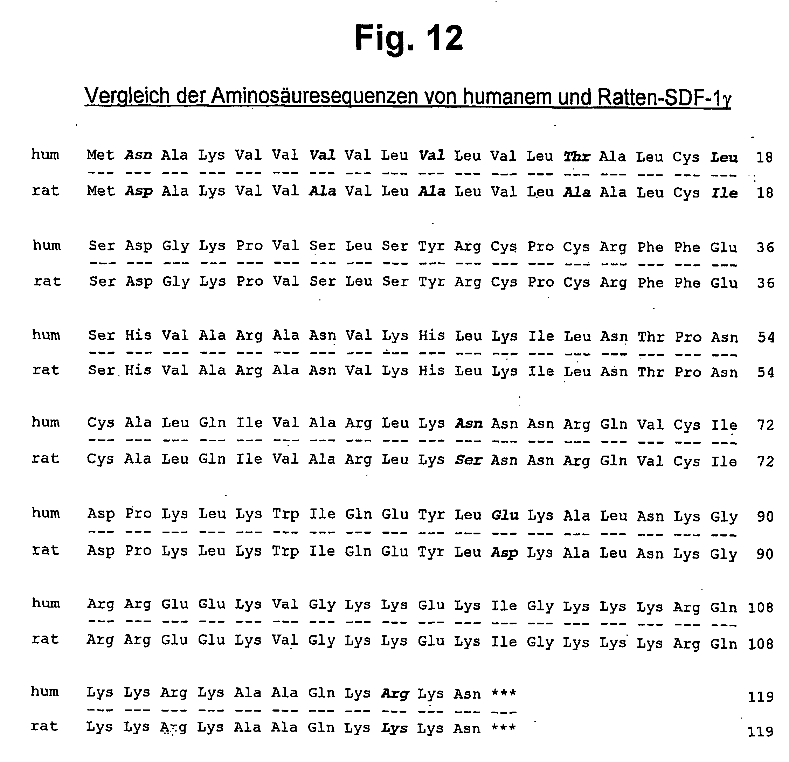

Figur 12 zeigt einen Vergleich der von den Nukleinsäuresequenzen in Figur 11 abgeleiteten Aminosäuresequenzen von humanem und Ratten-SDF-1γ. "hum": Humansequenz; "rat": Ratte.
Figur 13 zeigt schematisch die in dem Plasmid PCRII-TOPO (Invitrogen, Groningen, NL) eingebauten hSDF-1γ- und hSDF-1γ-H6-Konstrukte sowie die Konstrukte M-mhSDF-1γ-H6, hSDF-1γ-H6 und MFα-mhSDF-1γ-H6 in dem Plasmid pFPMT121.
Figur 14 zeigt die Restriktionskarte des 439 bps langen DNA-Fragments mit dem kodierenden Bereich des hSDF-1γ-Gens.
Figur 15 zeigt die Restriktionskarte des 457 bps langen DNA-Fragments mit dem kodierenden Bereich des hSDF-1γ-Gens und dem His-Tag.
Figur 16 zeigt die Restriktionskarte des Expressionsplasmids pFPMT-M-mhSDF-1γ-H6.
Figur 17 zeigt die Restriktionskarte des Expressionsplasmids pFPMT-hSDF-1γ-H6.
Figur 18 zeigt schematisch die Strategie zur Generierung des Expressionsplasmids pFPMT-MFα-mhSDF-1γ-H6. Die mit "P" gekennzeichneten Pfeile stellen PCR-Primer dar.
Figur 19 zeigt die Restriktionskarte des Expressionsplasmids pFPMT-MFα-mhSDF-1γ-H6.

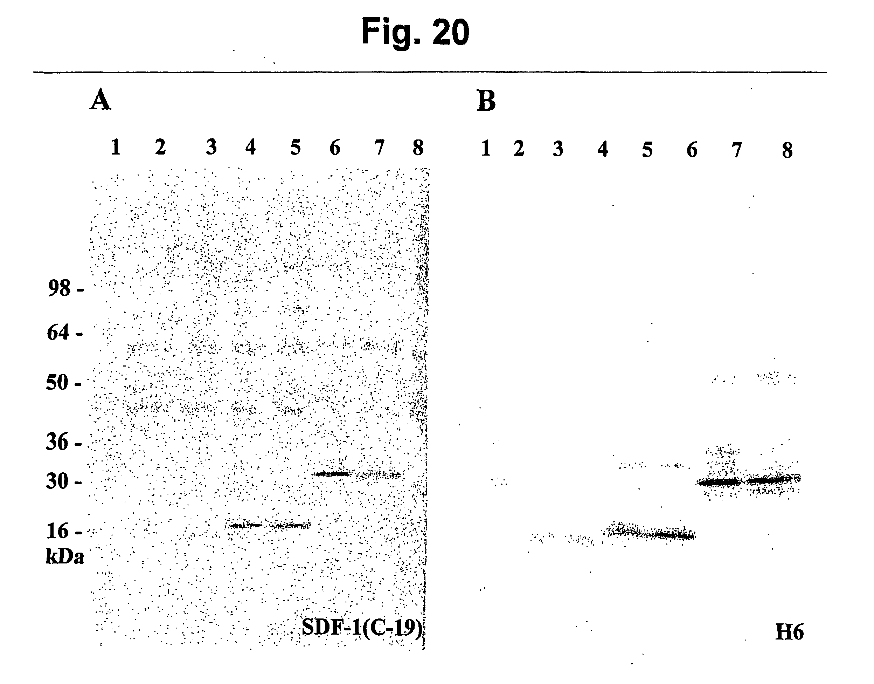

Figur 20 zeigt das Ergebnis eines Westem-Blot-Versuchs zum Nachweis der Expressionsprodukte in Zellextrakten von H. *polymorpha* (A) mit dem SDF-1-spezifischen Antikörper SDF-1(C19) (Santa Cruz Biotechnology, USA) und (B) mit einem His-Tag-spezifischen Antikörper (RGS-His Antibody, Mouse IgG1, Qiagen, Hilden, BRD). Die Spuren in (A) enthalten: (1) Sea Blue Prestained Standard, (2) M-mhSDF-1γ-H6, (3) M-mhSDF-1γ-H6 behandelt mit PNGaseF, (4) hSDF-1γ-H6, (5) hSDF-1γ-H6 behandelt mit PNGaseF, (6) MFα-mhSDF-1γ-H6, (7) MFα-mhSDF-1γ-H6 behandelt mit PNGaseF und (8) Zellextrakt ohne SDF-1γ. Die Spuren in (B) enthalten: (1) Zellextrakt ohne SDF-1γ, (2) Sea Blue Prestained Standard, (3) M-mhSDF-1γ-H6, (4) M-mhSDF-1γ-H6 behandelt mit PNGaseF, (5) hSDF-1γ-H6, (6) hSDF-1γ-H6 behandelt mit PNGaseF, (7) MFα-mhSDF-1γ-H6 und (8) MFα-mhSDF-1γ-H6 behandelt mit PNGaseF.
Figur 21 zeigt einen Vergleich der Wirkung von SDF-1α und SDF-1γ auf die Ca-Konzentration in Astrozyten. (A) 50 nM SDF-1α; (B) 35 µg Hefezellextrakt mit rekombinantem SDF-1γ (M-mhSDF-1γ-H6); (C) 22,4 µg Kontroll-Extrakt; (D) quantitative Auswertung des intrazellulären Calciumanstiegs bei SDF-1γ und dem Kontroll-Extrakt bezogen auf den von SDF-1α hervorgerufenen Calciumanstieg.
Figur 22 zeigt das Ergebnis eines Ca-Imaging-Experiments in Astrozyten für SDF-1α ohne (A) und mit (B) Vorinkubation mit Antikörper gegen CXCR4.
Figur 23 zeigt das Ergebnis eines Ca-Imaging-Experiments in Astrozyten für SDF-1γ ohne (A) und mit (B) Vorinkubation mit Antikörper gegen CXCR4.
Figur 24 zeigt das Ergebnis eines Ca-Imaging-Experiments in Cortexneuronen für SDF-1γ ohne (A) und mit (B) Vorinkubation mit Antikörper gegen CXCR4.
Figur 25 zeigt das Ergebnis eines Ca-Imaging-Experiments in Astrozyten für das C-terminale basische Peptid von SDF-1γ (30 Aminosäuren) ohne (A) und mit (B) Vorinkubation mit Antikörper gegen CXCR4.
Figur 26 zeigt das Ergebnis eines Ca-Imaging-Experiments in Astrozyten für: (A) das Peptid 2 (KKEKIG; SEQ ID NO:6) und (B) das Peptid 3 (KKKRQ; SEQ ID NO:8).

### Beispiele

### Beispiel 1

### Klonierung und Sequenzanalyse von SDF-1γ

### A. Materialien und Methoden

### Tierversuche

Erwachsene Wistar-Ratten (Körpergewicht 200-250 g) wurden durch intraperitoneale Verabreichung von Chloralhydrat (350 ml/kg Körpergewicht) betäubt. Der Ischiasnerv wurde im oberen Oberschenkelbereich mit Pinzetten kurzzeitig gequetscht (Müller et al., 1986). Vor der Gewinnung von RNA aus den Nervenbahnen wurde das Gewebe 2-3 mm um die Wunde herum entfemt und entsorgt. Alle Tierversuche wurden gemäß den Richtlinien des deutschen Tierschutzgesetzes durchgeführt.

### RNA-Isolierung

Gesamt-RNA aus Rattengewebe wurde durch das Phenol-Guanidiniumthiocyanat-Verfahren (Chomczynski und Sacchi, 1987) isoliert. Die gefrorenen Gewebeproben wurden zweimal 45 Sekunden lang bei 2500 Upm mit einem Polytron (Brinkmann, Westbury, NY) homogenisiert. Poly(A)⁺-RNA wurde durch Oligo(dT)-Cellulose-Chromatographie isoliert (Sambrook et al., 1998).

### Herstellung einer cDNA-Genbank

Zur Herstellung einer cDNA-Genbank wurden 4,5 µg aus Ischiasnerv von erwachsenen Ratten isolierte Poly(A)⁺-RNA, als Matrize und Oligo(dT)₁₂₋₁₈ als Primer verwendet. cDNA wurde mit dem TimeSaver-cDNA-Synthese-Kit (Pharmacia-LKB, Piscataway, NJ) generiert. Die cDNA wurde unter Verwendung des Gigapack II-Verpackungsextraktes (Stratagene) mit *EcoRI* vorgeschnittenen λ-ZAP II-Phagenpartikeln durch Ligation verknüpft. Die Titration der erhaltenen cDNA-Genbank ergab eine Komplexität von etwa 0,5 x 10⁶. Das Screening der cDNA-Genbank wurde durch Standardverfahren (Sambrook et al., 1989) mit einem radioaktiv markiertem cDNA-Fragment aus dem nicht translatierten 3'-Bereich von rSDF-1β (Nukleotide 743-1368) durchgeführt.

### Oligonukleotide

Folgende Oligonukleotide wurden mit einem GeneAssembler Plus-Synthesator (Pharmacia, Piscataway, NJ) synthetisiert.

### DNA-Sequenzierung und -analyse

Beide cDNA-Stränge von SDF-1β und von dem 2,5 Kb langen Insert in SDF-1γ einschließlich der flankierenden Bereiche wurden mit der Didesoxy-DNA-Sequenzierungsmethode (Sanger et. al., 1977) mit Hilfe des T7-Sequenzierungskits (Pharmacia-LKB) sequenziert. Die Sequenzen wurden durch Sequenzierung mehrerer unabhängiger Klone aus RT-PCR-Reaktionen bestätigt. Die Daten wurden mit Hilfe der Programme FASTA (Pearson, 1990) und BLAST (Altschul et al., 1990) mit der EMBL-Datenbank verglichen. Eine weitergehende Analyse der Sequenzen wurde mit Hilfe des PCGENE-Software-Pakets (Intelligenetics, Mountain View, CA) durchgeführt.

### RT-PCR

Die Umkehrtranskription wurde mit 1-5 µg Gesamt-RNA und Reverse-Transkriptase Superscript (Gibco, Gaithersburg) gemäß den Anweisungen des Herstellers durchgeführt. Der erste cDNA-Strang wurde mit RNase H (Boehringer Mannheim) verdaut und anschließend wurde 1/10 des Volumens als Matrize für die PCR-Amplifizierung mit Amplitaq-Polymerase (Perkin Elmer) oder Pfu-Polymerase (Stratagene, La Jolla) (für SDF-1γ) verwendet.

### B. Klonierung und Sequenzierung von SDF-1γ

Bei der Identifizierung von Genen, die nach einer Nervenläsion differentiell exprimiert werden, wurde aus einer cDNA-Genbank, die aus Ischiasnerv von Ratten erzeugt wurde, der cDNA-Klon NT-I-15 mit 2174 Nukleotiden isoliert. Die Analyse der Sequenz NT-I-15-Klons zeigte, dass dieser Klon eine 86%ige Homologie zu dem nicht translatierten 3'-Bereich (UTR) der SDF-1β-cDNA von Maus (vgl. Tashiro et al., 1993) aufweist. Bei Northem Blot-Versuchen unter stringenten Waschbedingungen hybridisierte NT-I-15 mit zwei Transkripten aus dem Ischiasnerv von erwachsenen Ratten (Figur 2). Während das kleinere Transkript mit etwa 3 Kb der Größe der SDF-1β-mRNA entsprach, war das längere Transkript mit 5,5 Kb unbekannt. Dieses Transkript wurde SDF-1γ genannt.

Die Isolierung von vollständigen Klonen für beide Transkripte wurde sowohl durch Screenen einer cDNA-Genbank als auch durch Umkehrtranskription-PCR (RT-PCR) angegangen. Durch Screenen einer cDNA-Genbank aus Ischiasnerv von Ratten mit einem 626 Nukleotide langen cDNA-Fragment des NT-I-15-Klons, das den Nukleotiden 734-1368 des 3'-UTR-Bereiches von SDF-1β der Ratte entspricht, erhielt man einen vollständigen cDNA-Klon mit einer Länge von 2819 Nukleotiden, der den vollständigen kodierenden Bereich von SDF-1β enthält.

Durch erneutes Screenen der cDNA-Genbank mit dem 626 Nukleotide langen cDNA-Fragment von NT-I-15 erhielt man einen unvollständigen SDF-1γ-Klon von etwa 3400 Nukleotiden (SDF-I-G6), der nicht nur den vollständigen 3'-UTR-Bereich und die letzten 4 Codons (90-93) von SDF-1β, sondern auch eine neue (nicht-kodierende) Sequenz mit einer Länge von etwa 1 Kb stromaufwärts des Codons 90 enthielt. Es wurde dann angenommen, dass das im Northem Blot identifizierte Transkript mit einer Länge von 5,5 Kb eine alternativ gespleißte Isoform darstellt, die durch ein 2,5 Kb langes Insert zwischen den Codons 89 und 90 von SDF-1β erzeugt wird. Zur Bestätigung dieser Hypothese wurde durch RT-PCR mit Antisense-Primem, die für die neue Sequenz am 5'-Ende des SDF-G6-Klons spezifisch sind (Primer GAS2 und GAS3), und einem Sense-Primer, der der Translationsinitationsstelle von SDF-1β entspricht (Primer MNSE2), ein neues Fragment erzeugt. Die Sequenzierung des amplifizierten PCR-Fragments zeigte ein Transkript, das ab Codon 89 stromabwärts eine andere Sequenz als SDF-1β aufwies. Dieses Transkript kodierte für ein Peptid von 119 Aminosäuren, von denen die ersten 89 Aminosäuren mit den ersten 89 Aminosäuren von SDF-1α und -β identisch waren. Anschließende Northem Blot-Analysen bestätigten, dass die erhaltene Sequenz das 5,5 Kb lange Transkript darstellte. cDNA-Sonden aus dem SDF-1β und -γ gemeinsamen 3'-UTR-Bereich (Fig. 2A) oder aus dem allen SDF-Isoformen gemeinsamen 5'-Bereich der kodierenden Region (Fig. 2B) hybridisierten sowohl mit dem 3 Kb langem (SDF-1β) als auch mit dem 5,5 Kb langem (SDF-1γ) Transkript, während eine cDNA-Sonde, die für das 2,5 Kb lange Insert spezifisch war, nur mit dem 5,5 Kb SDF-1γ-Transkript hybridisierte (Fig. 2C). Im Ischiasnerv von Ratten konnte keine SDF-1α-mRNA mit einer Länge von 1,5 Kb nachgewiesen werden.

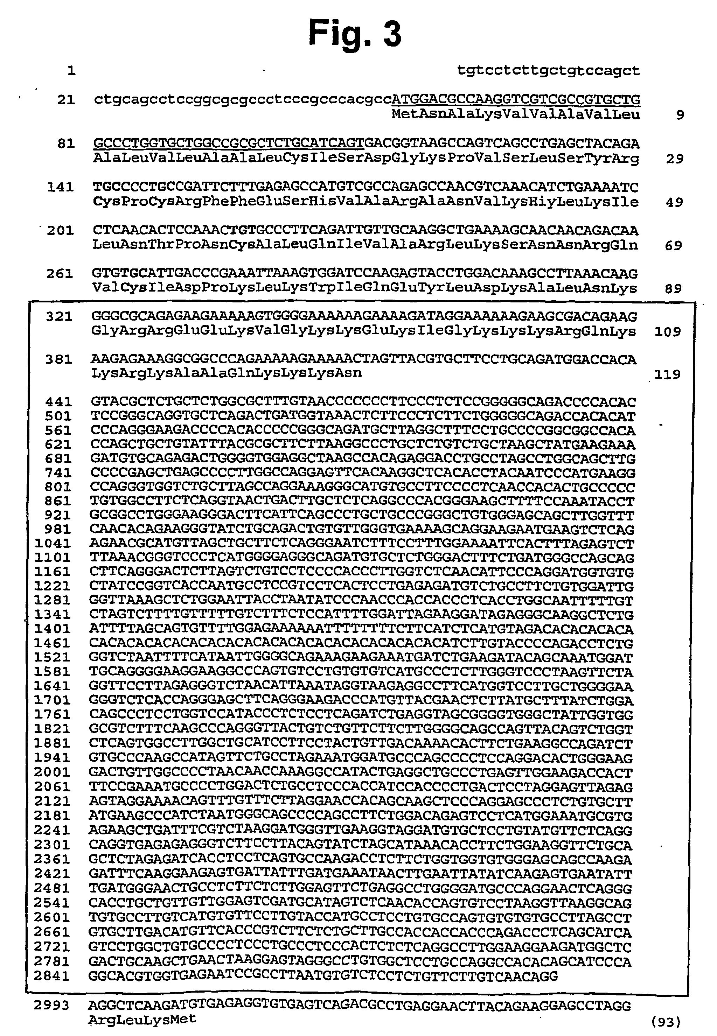

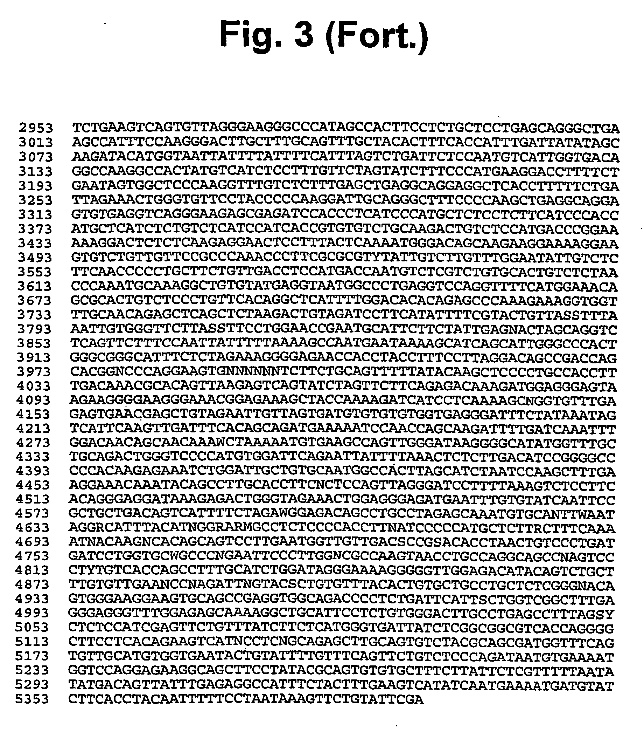

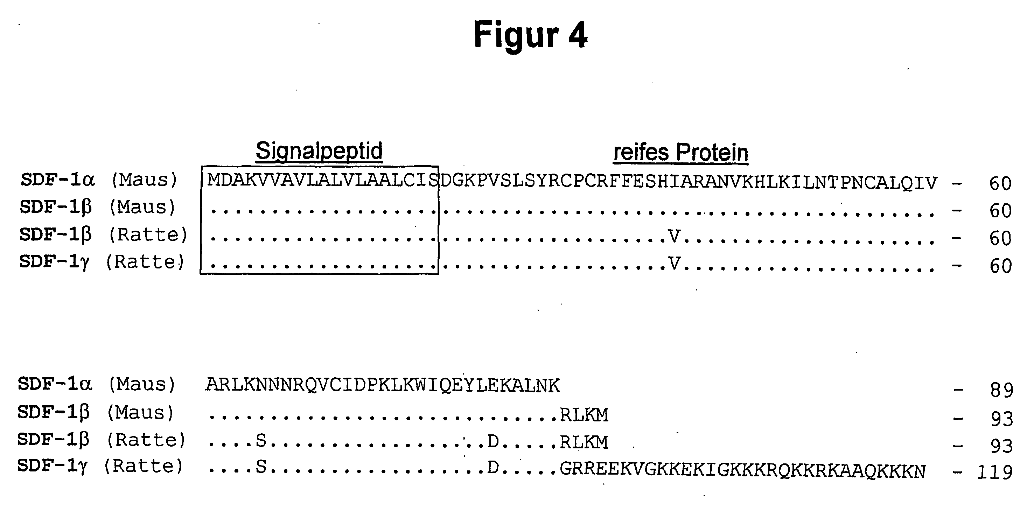

Beide Stränge der SDF-1β-cDNA von Ratte wurden sequenziert. Bei SDF-1γ wurde das neue Insert mit einer Länge von 2572 Nukleotiden und die flankierenden Bereiche mit den bekannten SDF-1β-Sequenzen ebenfalls doppelt sequenziert. Die abgeleitete Aminosäuresequenz für SDF-1β ergibt ein Peptid von 93 Aminosäuren mit einem theoretischem Molekulargewicht von 10,5 Kd. Die ersten 19 Aminosäuren stellen ein Signalpeptid für sezemierte Proteine dar. Die abgeleitete Aminosäuresequenz für SDF-1β beinhaltet die ersten 89 Aminosäurereste von SDF-1β und 30 zusätzliche Aminosäuren im Carboxyterminalen Bereich, die keine Homologie zu SDF-1β zeigen (vgl. Fig. 3). Das theoretische Molekulargewicht des 119 Aminosäuren langen SDF-1γ Peptids ist 13,5 Kd. Die Aminosäuresequenz des SDF-1β von Ratte zeigt eine starke Homologie (96,8%) zu dem entsprechendem Maus-Protein (98,9% unter Berücksichtigung konservativer Aminosäureausstausche). Ein Vergleich der Aminosäuresequenzen der neuen SDF-1-Isoformen SDF-1-γ und SDF-1 β mit den bekannten SDF-1-Sequenzen wird in Figur 4 gezeigt.

### Beispiel 2

### Nachweis von SDF-1β- und SDF-1γ-mRNA in verschiedenen Geweben und Entwicklungsstadien

### A. Northem Blot-Analyse

Je 10 µg Gesamt-RNA wurden in 1,2%igen Agarosegelen, die 15% Formaldehyd enthielten, fraktioniert und dann durch Standardverfahren auf Nytran NY 13 N-Membranen (Schleicher und Schüll, Keene, NH) übertragen. Die Filter wurden mit UV-Licht bestrahlt und mit Methylenblau gefärbt (Sambrook et at., 1989), in einer 0,5 M NatriumphosphatLösung (pH 7,0) mit 7% SDS vorhybridisiert und mit 1-5 x 10⁶ cpm/ml einer ³²P-markierten cDNA-Sonde in derselben Lösung hybridisiert. cDNA-Fragmente entsprechend (i) dem gemeinsamen 3'-UTR-Bereich von SDF-1β/γ (Nukleotide 743-1368 in SDF-1β), (ii) dem allen SDF-1-Isoformen gemeinsamen kodierenden Bereich (Nukleotide 49-239) und (iii) einem 1702 Nukleotide langen Abschnitt des SDF-1γ-spezifischen Inserts (Nukleotide 625-2327 in der SDF-1γ-cDNA) wurden durch einseitig gerichtete PCR (Stürzl et al., 1991) radioaktiv markiert. Nach dem Hybridisieren wurden die Filter mindestens 15 Minuten lang bei 60°C in 2 x SSC/1% SDS und 15 Minuten lang bei 60°C in 0,1 x SSC/1% SDS gewaschen. Die Filter wurden entweder zusammen mit einem Röntgenfilm (X-Omat AR, Kodak) exponiert oder mit einem BAS-1050-Bioimager (Fuji) direkt quantifiziert.

### B. Nachweis von SDF-1β- und SDF-1γ-mRNA in verschiedenen Geweben

Die in Figur 5 A gezeigten Northem Blot-Hybridisierungsversuche wurden mit Gesamt-RNA aus verschiedenen Geweben von erwachsenen Ratten und einem 602 Nukleotide langen Fragment aus dem gemeinsamen 3'-UTR-Bereich von SDF-1β/γ , das mit ³²p-dCTP radioaktiv markiert worden war, durchgeführt. Die Verteilung der SDF-1β- und SDF-1γ-mRNA über verschiedene Gewebe von erwachsenen Ratten zeigte ein komplementäres Muster. Während die β-Isoform vor allem in der Leber, der Niere, der Milz und dem Thymus nachgewiesen wurde, trat SDF-1γ vorwiegend im Herz- und Lungengewebe sowie im reifen Gewebe des Nervensystems auf (Fig. 5). Die Tatsache, dass das SDF-1β-Transkript vor allem im embryonalen und neonatalen Himgewebe und im Ischiasnerv bevorzugt auftritt, deutet auf eine differentielle Regulation der SDF-1-Expression während der Entwicklung des Nervensystems hin. In Muskel- und Hodengewebe konnten weder SDF-1β- noch SDF-1γ-mRNA nachgewiesen werden (Fig. 5A).

### C. Nachweis von SDF-1β- und SDF-1γ-mRNA im Gehim und im Ischiasnerv im Laufe der Entwicklung

### Gehirn:

Zur Untersuchung der entwicklungsspezifischen Verteilung von SDF-1β und SDF-1γ wurde RNA aus Himgewebe zu verschiedenen Stadien der Entwicklung in Ratten (von 17 Tage alten Embryonen (E17) bis zu erwachsenen Ratten) mit einem 602 Nukleotide langen Fragment des gemeinsamen 3'-UTR-Bereichs von SDF-1 β/γ geprobt. Im Himgewebe von E17-Embryonen wurde vorwiegend SDF-1β-mRNA nachgewiesen, die Transkriptmenge sank jedoch mit zunehmendem Alter und das Transkript konnte im Himgewebe von erwachsenen Ratten nicht mehr nachgewiesen werden. Im Gegensatz dazu war die Menge an SDF-1γ-Transkript bei E17-Embryonen sehr niedrig, sie nahm jedoch stetig zu und erreichte ein Maximum bei erwachsenen Ratten (Fig. 5B).

### Ischiasnerv:

Aus Ischiasnerv von Ratten in verschiedenen Entwicklungsstadien (von einem Tag nach der Geburt (P1) bis zum Erreichen des Erwachsenenalters) wurde Gesamt-RNA isoliert. Im P1-Stadium wurde SDF-1β-mRNA in kleinen Mengen nachgewiesen; die Transkriptmenge stieg im P4-P7-Stadium und sank im Nervengewebe von erwachsenen Ratten unter der Nachweisgrenze (Fig. 5C). SDF-1γ-mRNA wurde erstmals im P4-Stadium beobachtet; die Transkriptmenge erreichte im P-14-Stadium ein Maximum und sank dann im Gewebe von erwachsenen Ratten auf ein mittleres Niveau (Fig. 5C).

SDF-1β- und SDF-1γ-mRNAs scheinen also während der Entwicklung und im Nervensystem der erwachsenen Ratte ein unterschiedliches Expressionsmuster zu zeigen. Während die SDF-1 β-Isoform überwiegend im embryonalen oder perinatalem ZNS und PNS auftritt, ist SDF-1γ die wichtigste Variante im Nervensystem von erwachsenen Ratten (Fig.5B,C). In dem Zeitraum zwischen dem 4. und dem 7. Tag nach der Geburt, in dem die Differenzierung der Gliazellen und die Neuronenreifung einsetzt, treten die SDF-1β-und SDF-1γ-Transkripte in nahezu gleichen Mengen auf.

### D. Nachweis von SDF-1β- und SDF-1γ-mRNA nach einer Läsion des Ischiasnervs

Nach Verletzung des Ischiasnerv durch Quetschung wurden am distalen Ende des Nervs kleine Änderungen des SDF-1β- und SDF-1γ-mRNA-Musters beobachtet. Mittels "multiple quantitative bioimaging" von radioaktiven Northem Blot-Filtern wurde nach der Läsion eine vorübergehende Erhöhung der SDF-1β-mRNA-Menge festgestellt; die zwei Tage nach der Nervenquetschung ein Maximum bei 175% erreichte; danach sanken die Werte, bis sie am 7. Tag nach der Verletzung das gleiche Niveau wie die Kontrolle erreichten. Für die SDF-1γ-mRNA wurden nach der Nervenläsion keine signifikanten Änderungen festgestellt.

### Beispiel 3

### Zelluläre Lokalisierung des SDF-1γ-Transkripts durch in situ-Hybridisierung

### A. In situ-Hybridisierung

Die Gewebeproben wurden in Tissue Tec II (Miles, Napperville, IL) eingebettet, in Methylbutan bei -70°C eingefroren und in 20 µm starke Schnitte geschnitten. Die Schnitte wurden fixiert und anschließend acetyliert und 4 Stunden lang bei 55°C gemäß Angerer et al. (1987) vorhybridisiert. *In vitro*-Transkripte (i) eines Subklons des gemeinsamen 3'-UTR-Bereiches von SDF-1β/γ (Nukleotide 1758-2199 in SDF-1β), (ii) eines Subklons, der allen SDF-1-mRNA-Isoformen gemeinsamen 5'-UTR- und kodierenden Bereiche (Nukleotide 1-596 in der SDF-1 β-cDNA) und (iii) eines Subklons des SDF-1γ-spezifischen Inserts (Nukleotide 661-1313 in der SDF-1γ-cDNA) wurden mit Hilfe des DIG-RNA-Markierungskits von Boehringer Mannheim unter Verwendung von Digoxigenin-UTP erzeugt. Nach der Hybridisierung bei 55°C über Nacht wurde eine RNase A-Behandlung (20 µg/ml in 0,6 M NaCl, 20 mM Tris-HCl, 2 mM EDTA, pH 8) 20 Minuten lang bei 37°C durchgeführt. Die Schnitte wurden dann dreimal je 20 Minuten lang bei 50°C mit 2 x SSC und dreimal je 20 Minuten lang bei 50°C mit 0,2 x SSC gewaschen. Der Digoxigenin-Nachweis wurde nach den Anweisungen des Herstellers (Boehringer Mannheim) durchgeführt.

### B. Zelluläre Lokalisierung des SDF-1γ-Transkripts

Für die *in situ*-Hybridisierung wurden Antisense-Transkripte aus (a) dem gemeinsamen 3'-UTR-Bereich von SDF-1β/γ, (b) dem allen SDF 1-Isoformen gemeinsamen 5'-UTR- und kodierenden Bereich und (c) dem SDF-1γ-spezifischen Insert mit Digoxigenin-UTP markiert. Die Sense-Transkripte dienten als negative Kontrollen.

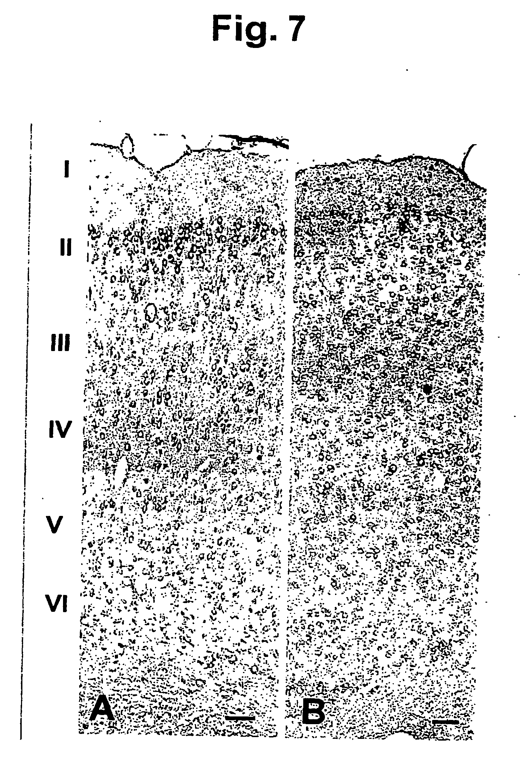

In ZNS von erwachsenen Ratten wurden sowohl mit der allen SDF-1-Isoformen gemeinsamen als auch mit der SDF-1γ-spezifischen Sonde starke und ausgedehnte Hybridisierungssignale in Bereichen mit grauer Himsubstanz sowie in "Perlenketten"-ähnlichen Anreihungen von Oligodendrozyten in myelinisierten Nervenfasem wie etwa dem *Corpus callosum* beobachtet (Fig. 6A,B). Weitere Hybridisierungssignale traten vor allem in Verbindung mit Purkinje- und granulären Neuronen im Kleinhim (Fig. 6D,E), in pyramidalen und granulären Neuronen im Hippocampus (Fig.6G,H) sowie in Neuronen sämtlicher Hauptschichten des Neocortex (Fig.7A,B), auf. Mit den entsprechenden Sense-Transkripten erhielt man keine Hybridisierungssignale (siehe Fig. 6C,F,I für die Sense-Sonden für SDF-1γ).

Die mit dem SDF-1γ-spezifischen Antisense-Transkript erhaltenen Signale waren nahezu identisch mit dem Hybridisierungsmuster, das mit der gemeinsamen SDF-1-Antisense-Sonde erhalten wurde, was darauf hindeutet, dass die SDF-1γ-Isoform in Neuronen und Oligodendrozyten des Gehirns von erwachsenen Ratten exprimiert wird. SDF-1 β-Transkripte, soweit sie im Gehirn von erwachsenen Ratten auftreten, scheinen in den gleichen Bereichen und Zellpopulationen wie SDF-1γ vorhanden zu sein.

In Längsschnitten des Ischiasnervs von erwachsenen Ratten erzeugt eine Antisense-Sonde aus dem gemeinsamen 3'-UTR-Bereich von SDF-1β/γ spindelförmige Hybridisierungssignale, die der typischen Form von Schwann-Zellen in der Nähe der Axonfasem ähnelten (Fig. 8A,B). Die identische Lokalisierung der S-100-Immunreaktivität sowie der Hybridisierungssignale in Querschnitten des Ischiasnervs (siehe Pfeilspitzen) bestätigten die Expression von SDF-1 β/γ-mRNA in Schwann-Zellen (Fig. 8 D,E). Bei Verwendung einer SDF-1γ-spezifischen Antisense-Sonde erhält man im Ischiasnerv von erwachsenen Ratten ein Markierungsmuster (Fig. 8H), das dem Hybridisierungsmuster des mit dem allen SDF-Isoformen gemeinsamen Antisense-Transkripts (Fig. 8G) stark ähnelte.

SDF-1γ-mRNA (und vermutlich auch die SDF-1β-Isoform) treten sowohl in Schwann-Zellen (siehe Pfeilspitzen) als auch in vaskulären Zellen (obere rechte Ecke in Fig. 8G,H) des Ischiasnervs auf . Die Ergebnisse der *in situ*-Hybridisierungsversuche stimmen mit den Ergebnissen der Northem Blot-Versuche der Figuren 2 und 5 überein. SDF-1-α-mRNA wurde mit Antisense-/Sense-Transkripten aus dem SDF-1-α-spezifischen 3'-UTR-Bereich weder im Gehirn noch im Ischiasnerv von erwachsenen Ratten nachgewiesen.

### Beispiel 4

### Expression von SDF-1γ-Produkten in Hansenula polymorpha

### A. hSDF-1γ-Konstrukte

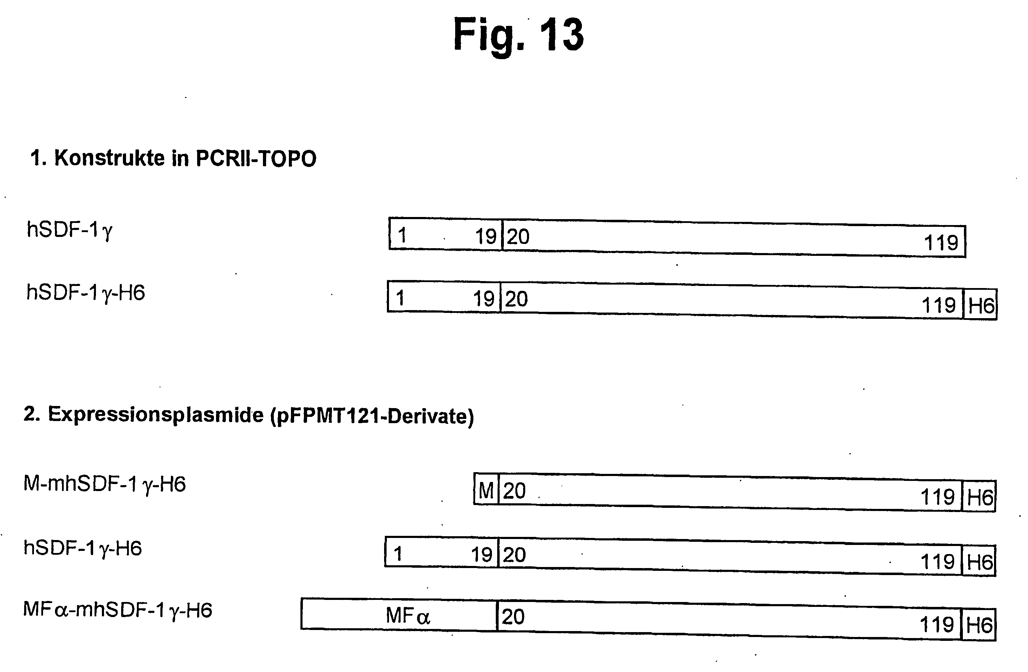

Zur Expression von SDF 1γ in *Hansenula polymorpha* wurden drei verschiedene Konstrukte erzeugt, die zwecks besserer analytischer Zugänglichkeit mit einem His-Tag ausgestattet wurden. Die Konstrukte werden in Figur 13 gezeigt.
1. M-mhSDF-1γ-H6 (Methionin/matures humanes SDF 1γ/His-Tag). Bei diesem Fusionsprotein befindet sich die Sequenz des reifen humanen SDF-1γ (Aminosäuren 20-119 in SEQ ID NO:12) hinter einem N-terminalen Methionin-Rest. Da keine Leader-Sequenz vorhanden ist, wurde eine cytosolische Lokalisierung erwartet. Am C-Terminus befinden sich sechs Histidin-Reste (His-Tag).
2. hSDF-1γ-H6 (unreifes humanes SDF-1γ/His-Tag). Dieses Konstrukt umfaßt Aminosäuren 1-119 von SDF-1γ (SEQ ID NO:12), gefolgt von einem C-terminalen His-Tag. Es enthält somit die natürliche, in menschlichen Zellen erkannte Leader-Sequenz. Da mitunter Leader-Sequenzen auch in heterologen Wirtszellen erkannt werden, sollte mit diesem Konstrukt untersucht werden, ob *H. polymorpha* das authentische SDF-1γ-Leader-Peptid erkennt.
3. MFα-mhSDF-1γ-H6 (Prepro-Sequenz des Mating-Faktor α aus *Saccharomyces cerevisiae*/matures humanes SDF-1γ/His-Tag). Hier befindet sich die Sequenz des reifen SDF-1γ (Aminosäuren 20-119 in SEQ ID NO:12) hinter der in *H. polymorpha* häufig genutzten Prepro-Sequenz des Mating-Faktor α aus der verwandten Bierhefe *Saccharomyces cerevisae*. Dieses Konstrukt sollte von *H. polymorpha* sezemiert werden.

### B. Konstruktion von Expressionsplasmiden

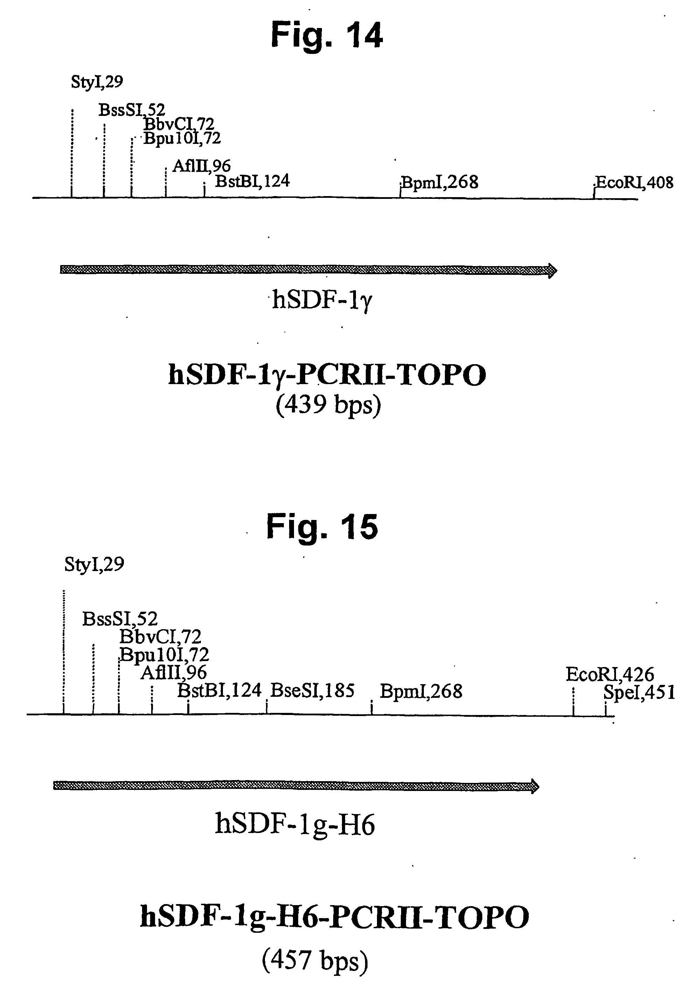

Als Ausgangskonstrukt diente das Plasmid SDF-1γ-PCRII-TOPO, das das 439 bps lange SDF-1γ-Insert enthielt (Fig. 14). In einem ersten Schritt wurden der hSDF-1γ-Sequenz durch PCR-Mutagenese sechs Kodons für einen C-terminalen His-Tag hinzugefügt (hSDF-1γ-H6, Fig. 15).

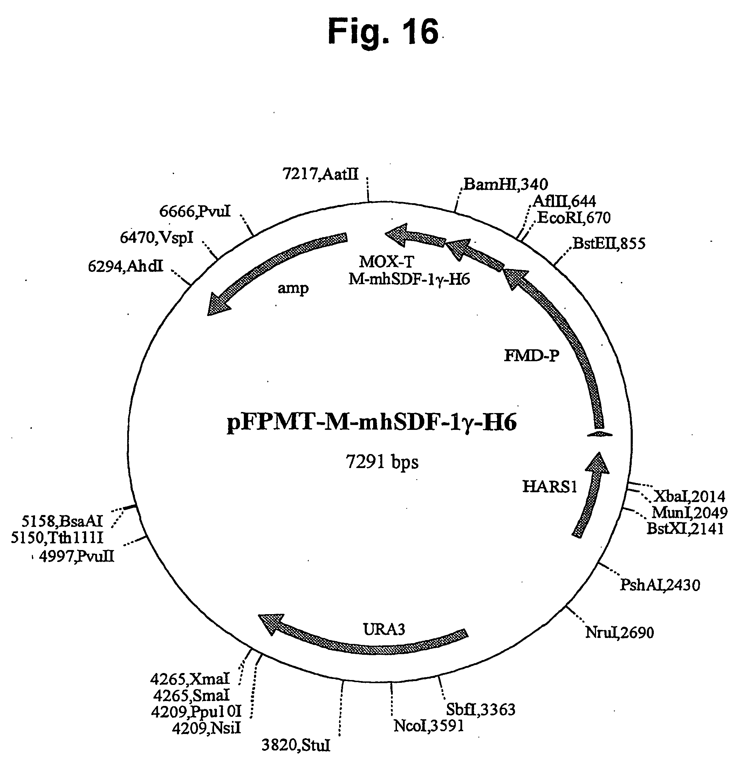

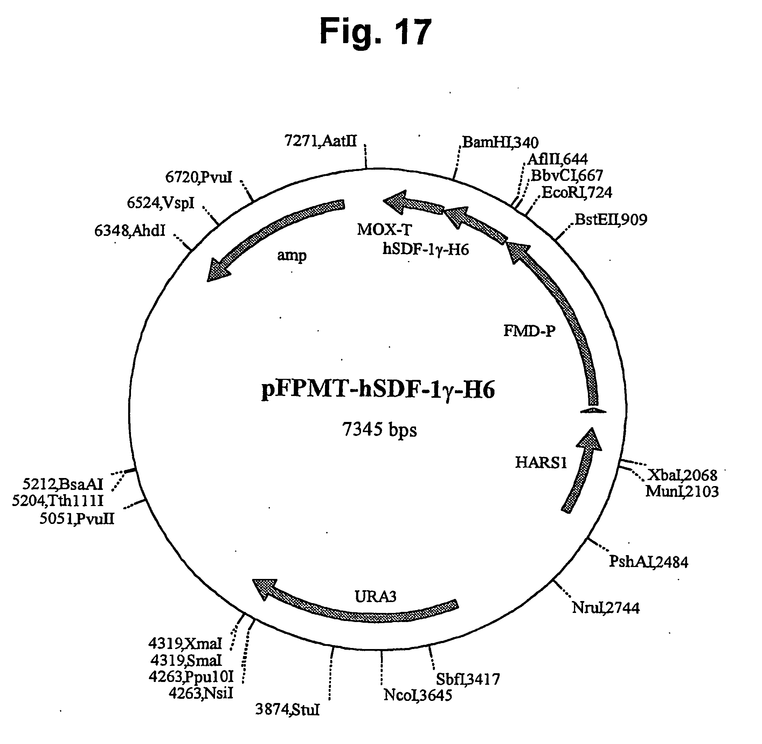

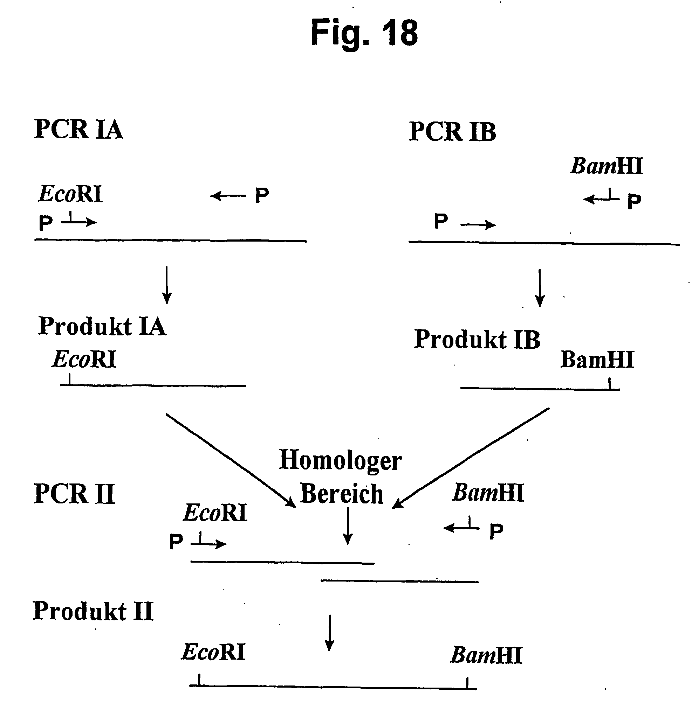

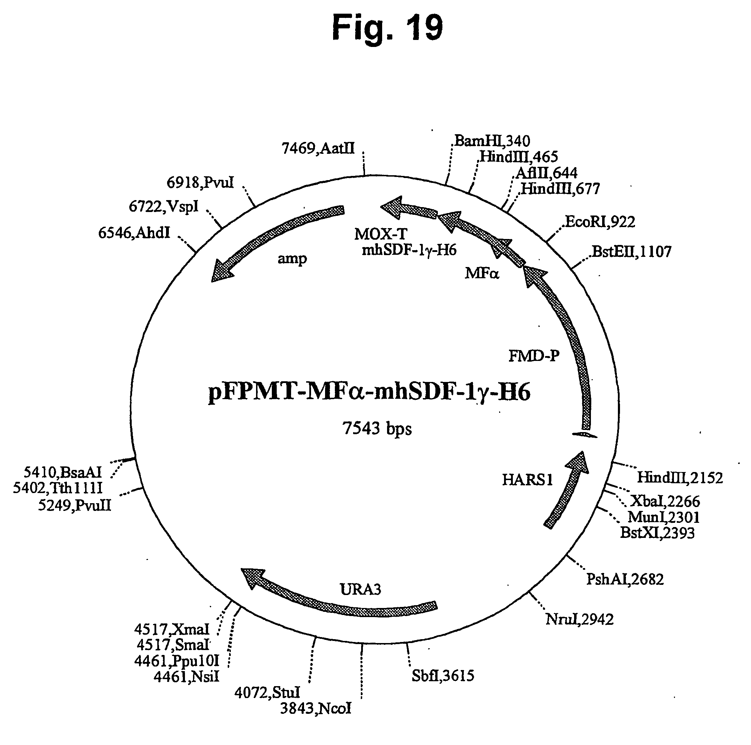

Als Basisvektor für die spätere Expression von SDF-1γ-Konstrukten in *H. polymorpha* wurde das integrative Plasmid pFPMT121 (Gellissen, 2000) eingesetzt, in dem das zu exprimierende fremde Gen unter der Kontrolle des FMD-Promotors steht. Auf der Grundlage dieses Plasmids wurden folgende Expressionsvektoren konstruiert:
1. pFPMT-M-mhSDF-1γ-H6. Von dem M-mhSDF-1γ-H6-ORF wurde mittels PCR ein DNA-Fragment generiert, bei dem die kodierende Sequenz von SDF-1γ von einer *Eco*RI- (vor dem Startkodon) und einer *Bam*HI-Restriktionsschnittstelle flankiert ist. Als Template-DNA diente hSDF-1γ-H6 in PCRII-TOPO (Invitrogen, Groningen, NL). Das PCR-Produkt wurde mit *Eco*RI/*Bam*HI verdaut und zwischen die entsprechenden Stellen des pFPMT121-Plasmids kloniert. Die Karte des resultierenden Plasmids pFPMT-M-mhSDF-1γ-H6 wird in Fig. 16 gezeigt.
2. pFPMT-hSDF-1γ-H6. Auch bei diesem Konstrukt wurde zunächst ein von einer *Eco*RI- und einer *Bam*HI-Schnittstelle flankiertes PCR-Produkt erzeugt, wobei wiederum hSDF-1γ-H6 in PCRII-TOPO als Template-DNA diente. Das mit *Eco*RI und *Bam*HI verdaute PCR-Produkt wurde zwischen die entsprechenden Stellen des pFPMT121-Plasmids kloniert. Die Karte des resultierenden Plasmids pFPMT-hSDF-1γ-H6 wird in Fig. 17 gezeigt.
3. pFPMT-MFα-mhSDF-1γ-H6. Zur Generierung dieses Plasmids wurden zwei separate PCR-Produkte erzeugt (siehe Fig. 18). Das erste PCR-Produkt (PCR IA) umfaßte die Kodons der Prepro-Sequenz des Mating-Faktors α, flankiert von einer *Eco*RI-Schnittstelle (vor dem Startkodon) sowie am anderen Ende von Basen mit Homologie zu den ersten Kodons der reifen hSDF-1γ-Sequenz. Das zweite PCR-Produkt (PCR IB) enthielt die Sequenz des reifen hSDF-1γ, am vorderen Teil flankiert von Basen mit Homologie zum hinteren Teil der Prepro-Sequenz des Mating-Faktors α, am hinteren Teil flankiert von einer *Bam*HI-Schnittstelle (hinter dem Stopkodon). Dann wurde eine weitere PCR-Reaktion (PCR II) durchgeführt, bei der die Produkte der beiden oben beschriebenen PCR IA und IB gemischt wurden. Als Primer wurden der Hin-Primer aus PCR IA (der mit der *Eco*RI-Schnittstelle) und der Rück-Primer aus PCR IB (der mit der *Bam*HI-Schnittstelle) eingesetzt. Das entstandene PCR-Produkt umfaßte die Prepro-Sequenz des Mating-Faktors α fusioniert mit der Sequenz des mhSDF-1γ-H6, flankiert von *Eco*RI- (vor dem Startkodon) und *Bam*HI-Schnittstellen (hinter dem Stopkodon). Nach Verdauung mit *Eco*RI/*Bam*HI wurde das Fragment zwischen die entsprechenden Schnittstellen des pFPMT121-Plasmids kloniert. Die Karte des resultierenden Plasmids pFPMT-MFα-mhSDF-1γ-H6 wird in Fig. 19 gezeigt.

### C. Transformation von H. polymorpha mit den erzeugten Expressionsvektoren, Stammgenerierung und -identifizierung

Zur Herstellung kompetenter H. *polymorpha*-Zellen für die Elektroporation wurden 5 ml YPD-Medium wurden mit einer Einzelkolonie von *H. polymorpha* RB11 (*odc*, Orotidin-5-phosphat-decarboxylase-defizienter (Uracil-auxotropher) H. *polymorpha*-Stamm (Weydemann et al., 1995 )) beimpft und 16 Std lang bei 37°C geschüttelt. Anschließend wurden 100 ml YPD-Medium in einem 2 I-Erlen-Meyer-Kolben mit 3 ml dieser Vorkultur beimpft und bis zu einer OD₆₀₀ von 0,8-1 bei 37°C inkubiert (Schüttelfrequenz 140 rpm). Die Zellernte erfolgte durch Zentrifugation der Kultur in 50 ml-Falcon-Röhrchen (4000 rpm; 6') in einer Beckmann-Zentrifuge. Nach Entfernung der Überstände wurden die Zellen in 20 ml 50 mM Kaliumphosphat-Puffer (pH 7,5; vorgewärmt auf 37°C) resuspendiert, mit 0,5 ml 1 M DTT versetzt und 15' lang bei 37°C inkubiert (Wasserbad). Anschließend wurden die Zellen erneut abzentrifugiert (3000 rpm; 10', Beckmannzentrifuge) und mit 100 ml, anschließend mit 50 ml STM-Puffer (270 mM Saccharose; 10 mM Tris-HCl pH 7,5; 1 mM MgCl₂) gewaschen. Nach erneutem Abzentrifugieren wurden die Zellen in 0,5 ml STM-Puffer resuspendiert und als 60 µl-Aliquots entweder direkt für Transformationen eingesetzt oder für spätere Verwendung bei -70°C eingefroren.

Kompetente Zellen von *H. polymorpha* RB11 wurden mit den drei erzeugten Expressionsplasmiden (siehe oben) wie folgt transformiert: 60 µl kompetente *H. polymorpha*-Zellen wurden mit 1-2 µg der einzuführenden zirkulären Plasmid-DNA versetzt und in Elektroporationsküvetten mit 2 mm Spaltbreite überführt. Die Elektroporation erfolgte bei 2 kV, 25 µF und 200 Ohm. Anschließend wurden die Zellen in Reagenzgläser mit 1 ml YPD-Medium überführt und 1 Std. lang bei 37°C geschüttelt (Winkel 45°; 160 rpm). Nach dieser Erholung wurden je 330 µl der Zellen auf YNB-Agar-Platten (1% Glukose; ohne Uracil) ausplattiert. Die Platten wurden bei 37°C inkubiert, bis makroskopische Uracil-prototrophe Kolonien sichtbar wurden (ca. 1 Woche).

Anschließend wurden jeweils 36 Uracil-prototrophe Kolonien durch viermaliges Passagieren und zweimaliges Stabilisieren in stabile Stämme überführt. Zur Passagierung wurden je 2 ml YNB-Medium (1%Glukose) mit einzelnen Uracil-prototrophen Kolonien von den Transformanten-Platten angeimft und 2 Tage lang bei 37°C inkubiert (Winkel 45°; Schüttelfrequenz 160 rpm). Je 150 µl der resultierenden Kulturen wurden in je 2 ml frisches YNB-Medium überführt und erneut 2 Tage lang inkubiert (s. o.). Dieser Vorgang wurde insgesamt viermal durchgeführt (= vier Passagen). Zur Stabilisierung wurden je 150 µl der Kulturen aus der letzten Passage in 2 ml YPD-Medium überführt und für 2 Tage bei 37°C inkubiert (s. o.). Anschließend wurden Aliquots dieser Kulturen auf YNB-Agar-Platten (1% Glukose; ohne Uracil) ausplattiert. Pro Ausstrich wurde eine einzelne Kolonie isoliert und als Stamm definiert.

### D. Induktion der Expression und Nachweis von SDF-1γ-Produkten

Nach Vereinzelung wurden alle Stämme einer MeOH-Induktion unterzogen und die intrazellulären löslichen Fraktionen wurden mittels Western-Blot bezüglich ihres Gehalts an hSDF-1γ-Produkten analysiert. Zunächst wurden je 2 ml YPD-Medium in 10 ml-Reagenzgläsern mit Einzelkolonien der zu testenden Stämme beimpft und anschließend wurden die Kulturen zur Induktion der Fremdgenexpression 16 Std. lang bei 37°C inkubiert (Winkel 45°; Schüttelfrequenz 160 rpm). Anschließend wurden 150 µl der resultierenden dichtgewachsenen Kulturen zum Beimpfen von je 3 ml YNB-Medium (1 % Glycerin) eingesetzt. Nach 24 Std. Schütteln bei 37°C wurden die Zellen abzentrifugiert und in je 3 ml YNB-Medium (1 % MeOH) resuspendiert. Die Fremdgenexpression wurde dann durch erneutes Schütteln 24 Stunden lang bei 37°C induziert.

Nach Abzentrifugation der Zellen aus den Induktionskulturen wurden zur Präparation von Kulturüberständen Aliquots der Überstände mit 4 x SAB (8% w/v SDS; 40% w/v Glycerin; 8 mM EDTA pH 6,8; 250 mM Tris pH 6,8; 0,04% w/v Bromphenolblau; 40% v/v α-Mercaptoethanol) versetzt und für 5' bei 95°C denaturiert.

Zur Präparation intrazellulärer löslicher Fraktionen wurden folgende Schritte auf Eis oder bei 4°C ausgeführt: Die Zellpellets aus den Induktionskulturen wurden in je 500 µl Extraktionspuffer (50 mM Tris pH 7,5, 150 mM NaCl, 0,1% v/v Triton X-100 oder PBS-Puffer) resuspendiert und mit je 12,5 µl PMSF versetzt. Anschließend wurden die Proben in 1,5 ml-Eppendorf-Gefäße überführt. Nach Zugabe von 500 µl Glasperlen erfolgte der Zellaufschluß in einem Vibrax bei 2500 rpm. Die Überstände wurden in frische Eppendorf-Gefäße überführt und 10' lang bei 10.000 rpm (Eppendorf-Zentrifuge mit Kühlfunktion) zentrifugiert. Die Überstände dieser Zentrifugation stellten die sogenannte intrazelluläre lösliche Fraktion dar. Diese wurden für direkte Proteingelelektrophorese mit ¼ Vol. 4 x SAB versetzt und 5' lang bei 95°C denaturiert, oder für weitere Verwendungen ohne SAB-Zusatz bei -20°C eingefroren.

Für die PNGaseF-Verdauung wurden je 8 µl der nativen intrazellulären löslichen Fraktion mit 1 µl 1% SDS versetzt und 5' lang bei 95°C inkubiert. Anschließend erfolgte Zugabe von 1 µl PNGaseF (2 u; Roche) oder H₂O. Nach Inkubation bei 37°C 16 Std. lang wurden 4 µl 4 x SAB hinzugefügt, die Proben 5' lang bei 95°C denaturiert und auf Proteingelen aufgetrennt.

Die Auftrennung der denaturierten Proben durch Proteingelelektrophorese erfolgte auf 4-20%igen Tricine-SDS-Gelen (Novex) nach Herstellerangaben. Anschließend wurden die Proteinbanden in einer Semy-Dry-Blot-Apparatur (Trans-Blot SD; Biorad) nach Herstellerangaben auf Nitrocellulose-Membranen übertragen. Für die Westem-Blots wurde als primäre Antikörper (-seren) ein His-Tag-spezifischer monoklonaler Antikörper aus Maus (RGS-His-Antikörper, Qiagen, Hilden, BRD) oder ein SDF-1-spezfisches polyklonales Serum aus Ziege (SDF-1 (C19); #sc6193; Santa Cruz Biotechnology, USA) eingesetzt. Die Western-Blots wurden mit den Westem Breeze-Kits Maus oder Ziege (Novex) nach Herstellerangaben durchgeführt.

Auf diese Weise konnten für jedes der drei Konstrukte Stämme identifiziert werden, die signifikante Mengen des jeweiligen hSDF-1γ-H6-Derivats produzierten. Für weitere Produktanalysen wurde der jeweils produktivste Stamm ausgewählt. Für pFPMT-M-mhSDF-1y-H6 war dies der Stamm g7-5/36; für pFPMT-hSDF-1γ-H6 und pFPMT-MFα-mhSDF-1γ-H6 wurden entsprechend die Stämme g8-28/7 und g9c-20/6 ausgewählt.

### E. Produktanalysen

In den Kulturüberständen der Stämme g7-5/36, g8-28/7 und g9c-20/6 konnten mittels Western-Blot keine sezemierten SDF-1γ-Produkte nachgewiesen werden. In der intrazellulären löslichen Fraktion dieser Stämme konnten SDF-1γ-Produkte sowohl mit einem His-Tag-spezifischen Antikörper aus Maus als auch mit einem SDF-1-spezifischen Serum aus Ziege (SDF-1 (C19); #sc6193; Santa Cruz Biotechnology, USA) identifiziert werden (siehe Fig. 20 A, B). Die intrazelluläre lösliche Fraktion eines Kontrollstamms (ohne SDF-1γ) zeigt nicht die als SDF-1γ-Produkte identifizierten Produkte (Fig. 20, Spur 8 (A), Spur 1 (B)).

Die im Western-Blot beobachteten Molekulargewichte der SDF-1γ-Hauptprodukte liegen generell etwas oberhalb der berechneten Molekulargewichten. M-mhSDF-1γ-H6: 12,692 kDa berechnet, etwa 16 kDa beobachtet (Fig. 20, Spuren 2 und 3 (A), Spuren 3 und 4 (B)); hSDF-1γ-H6: 14,529 kDa berechnet, etwa 17 kDa beobachtet (Fig. 20, Spuren 4 und 5 (A), Spuren 5 und 6 (B)); MFα-mhSDF-1γ-H6: 21,468 kDa berechnet, etwa 30 kDa beobachtet (Fig. 20, Spuren 6 und 7 (A), Spuren 7 und 8 (B)). Da alle Hauptbanden sowohl mit dem His-Tag-spezifischen Antikörper als auch mit dem SDF-1-spezifischen Serum detektierbar sind, müssen die den Banden zugehörigen Proteine C-terminal intakt sein. Darüber hinaus zeigen die apparenten Molekulargewichte der verschiedenen Produkte die erwarteten relativen Abstufungen (M-mhSDF-1γ-H6 < hSDF-1γ-H6 < MFα-mhSDF-1γ-H6; Fig. 20).

Die Aminosäure-Sequenzen von M-mhSDF-1γ-H6 und hSDF-1γ-H6 enthalten keine potentiellen N-Glycosylierungsstellen. Dementsprechend hat eine PNGaseF-Verdauung keinen Einfluß auf das apparente Molekulargewicht der jeweiligen Produkt-Hauptbande (Fig. 20, Spuren 2/3 und 4/5 (A), Spuren 3/4 und 5/6 (B)). MFα-mhSDF-1γ-H6 hat drei N-Glycosylierungsstellen im Bereich der MFα-Prepro-Sequenz, die typischerweise im ER N-glycosyliert werden. Die fehlende Reduktion des apparenten Molekulargewichts des 30 kDa-Produkts nach PNGaseF-Verdauung deutet an, daß es sich bei diesem Produkt um die Prepro-Form handelt, die nicht in das ER eingeschleust wurde (Fig. 20, Spuren 6/7 (A), Spuren 7/8 (B)). Oberhalb von 30 kDa befinden sich drei schwache PNGaseF-sensitive Banden (Spur 7 (B)), die nach N-Deglycosylierung nach unterhalb von 30 kDa verschoben werden (Spur 8 (B)). Diese Banden können als an der Pro-Sequenz N-glycosylierte Pro-Formen von MFα-mhSDF-1γ-H6 interpretiert werden, bei denen die Pre-Sequenz während des Eintritts in das ER abgespalten wurde.

### Beispiel 5

### Wirkung von rekombinantem humanen SDF-1γ auf die Calcium-Konzentration in Nervenzellen und Gliazellen

In diesem Beispiel wurde die Wirkung von rekombinantem humanem SDF-1γ (M-mhSDF-1γ-H6) in Zellextrakten von *Hansenula polymorpha* auf die Calcium-Konzentration in Nervenzellen und Gliazellen untersucht. Zur Bestimmung der Calcium-Konzentration wurde die Calcium-Imaging-Methode benutzt (siehe Köller et al., 2001).

Für die Ca-Imaging-Experimente wurden primäre Astrozyten und primäre Cortexneurone der Ratte (Stamm Wistar) aus neugeborenen Ratten (Postnataltag 0-1; Astrocyten) bzw. aus Rattenembryonen (Embryonaltag 15; Cortexneurone) wie von Köller et al. (2001) beschrieben präpariert und kultiviert. Nach 5-15 Tagen in Kultur wurden die Zellen *in vitro* 1 Std. lang mit dem Calcium-Indikator Fura-2 beladen. Das intrazelluläre Fura-2 reagiert mit freigesetztem Calcium zu Fura-Calcium, das bei einer anderen Wellenlänge (340 nm) absorbiert als Fura-2 (380 nm). Mit Hilfe des Extinktionskoeffizienten (F340/F380) kann somit die relative intrazelluläre Calcium-Konzentration ermittelt und graphisch aufgetragen werden. Dieses Verfahren ermöglicht die Detektion intrazellulärer Calcium-Konzentrationsänderungen, die durch extrazelluläre Stimuli (z.B. Ligand/Rezeptor-Interaktionen) hervorgerufen werden.

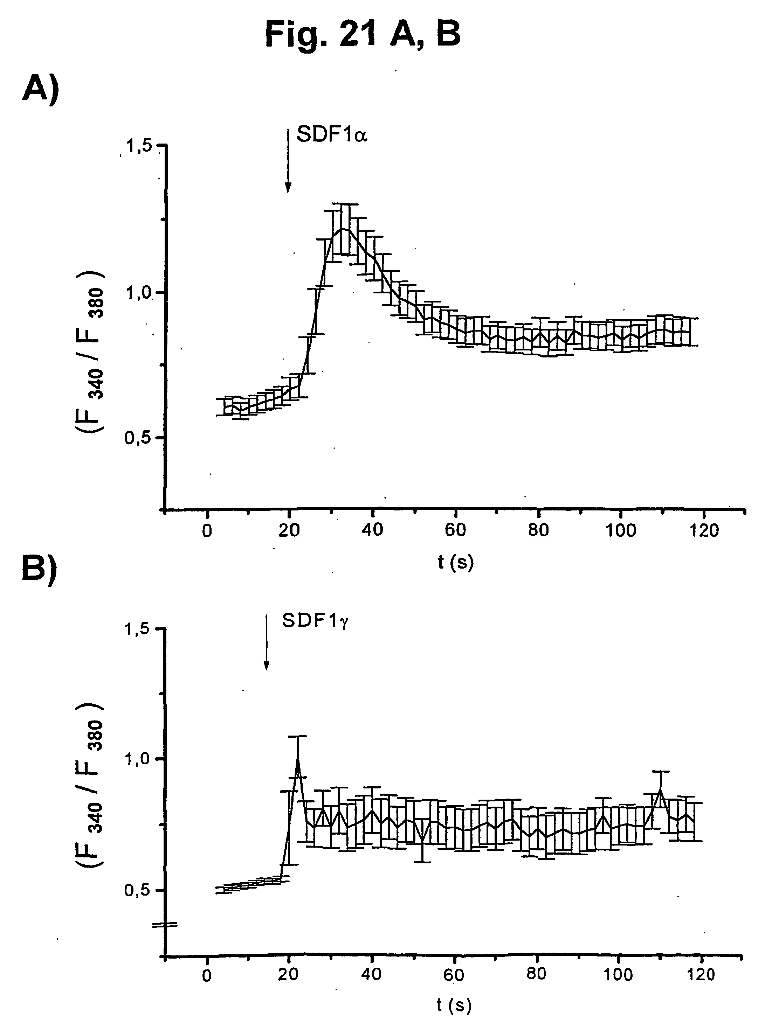

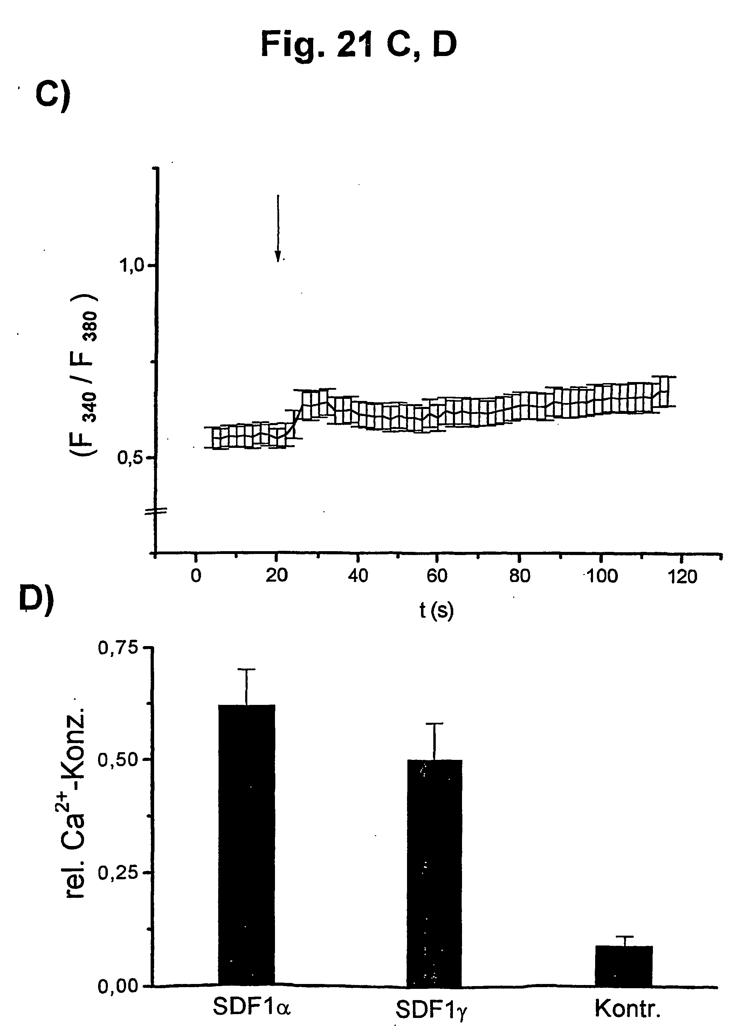

Figur 21 zeigt das Ergebnis von Ca-Imaging-Experimenten, bei denen die Wirkung von SDF-1α und SDF-1γ auf die Ca-Konzentration in Astrozyten verglichen wurde. Nach Applikation von SDF-1α (50 nM, R&D Systems, Wiesbaden, BRD) steigt die intrazelluläre Calciumkonzentration in kultivierten Astrozyten (Figur 21 A). Nach Applikation von Hefezellextrakt mit rekombinantem SDF-1γ (M-mhSDF-1γ-H6) (35 µg Gesamtprotein) kommt es zu einem intrazellulären Calciumanstieg in kultivierten Astrozyten (Figur 21 B). Die Antwort ist jedoch etwas geringer als die Antwort auf SDF-1 α.

Nach Applikation von Kontroll-Extrakt (22,4 µg Protein von einem Zellextrakt von *H. polymorpha* RB11-Zellen, die mit dem pFPMT121-Plasmid ohne Insert transformiert wurden) bleibt der intrazelluläre Calciumanstieg in kultivierten Astrozyten aus (Figur 21 C).

Figur 21 D zeigt die quantitative Auswertung der intrazellulären Calciumanstiege bei SDF-1γ und dem Kontroll-Extrakt bezogen auf den von SDF-1α hervorgerufenen Calciumanstieg.

Ferner wurde getestet, ob die Vorinkubation der Zellen mit einem CXCR4-spezifischen Antikörper (monoklonaler Antikörper 12G5; R&D Systems, Wiesbaden, BRD) eine solche Calcium-Antwort reduzieren kann, wie es zuvor für die von SDF-1α induzierte Calcium-Reaktion beobachtet wurde (für die methodischen Details zu den Ca-Imaging-Experimenten siehe Köller et al., 2001).

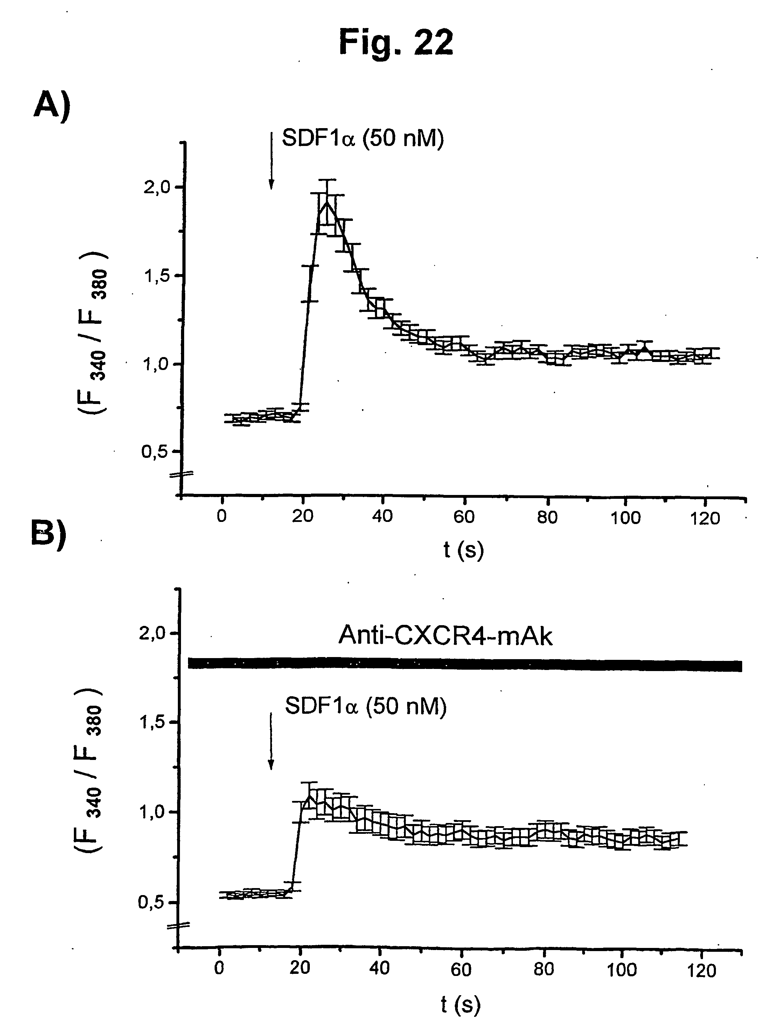

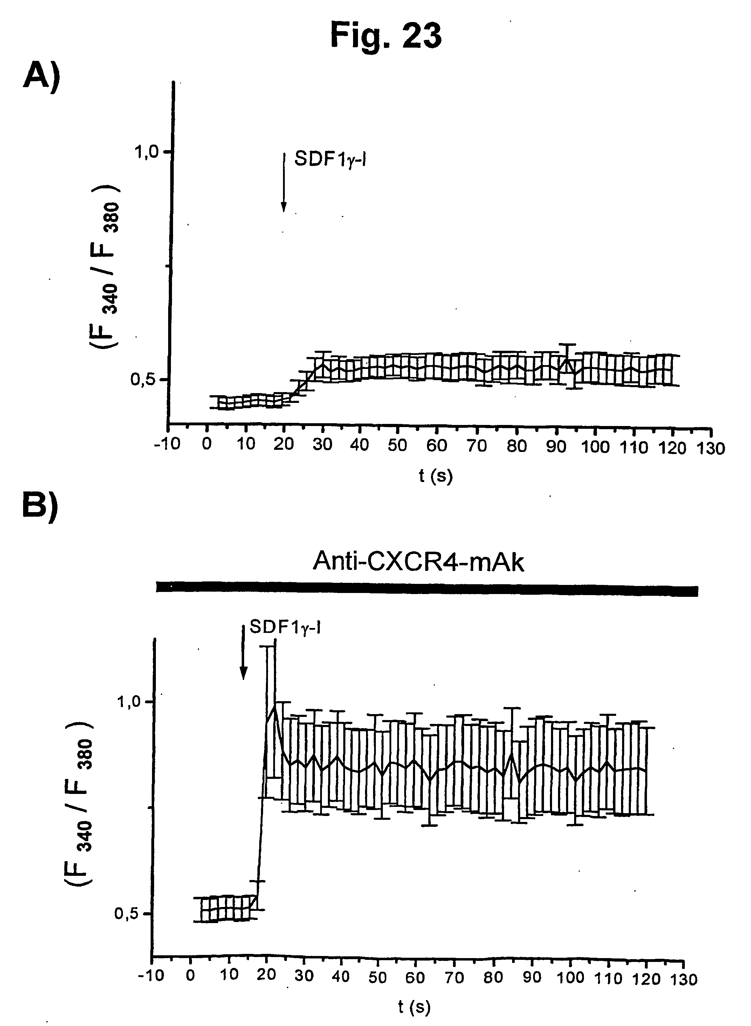

Figur 22 zeigt das Ergebnis eines Ca-Imaging-Experiments in Astrozyten für SDF-1α (A) ohne und (B) mit Antikörper gegen CXCR4, Figur 23 zeigt das Ergebnis der entsprechenden Experimente für SDF-1γ. Nach Applikation von SDF-1α (50 nM; R&D Systems, Wiesbaden, BRD) steigt die intrazelluläre Calciumkonzentration in kultivierten Astrozyten stark an (Figur 22 A). Gibt man jedoch SDF-1α nach 5 Min. Vorinkubation mit dem monoklonalen Antikörper 12G5, so zeigen kultivierte Astrozyten eine um ca. 50% verminderte intrazelluläre Calciumausschüttung (Figur 22 B). Diese Ergebnisse bestätigen Literaturbefunde, die zeigen, dass der Einfluß von SDF-1α bzw. -1 β auf die intrazelluläre Calcium-Konzentration in Astrozyten aus dem Zentralnervensystem durch den CXCR4-Rezeptor vermittelt wird.

Nach Applikation von 35 µg Hefezellextrakt mit rekombinantem SDF-1γ (M-mhSDF-1γ-H6; Zellaufschluss in PBS-Puffer) kommt es zu einem messbaren intrazellulären Calciumanstieg in kultivierten Astrozyten (Figur 23 A). Anders als bei SDF-1α führt die SDF-1γ-Applikation bei kultivierten Astrozyten, die mit dem monoklonalen Antikörper 12G5 gegen den CXCR4-Rezeptor 5 Min. lang vorinkubiert wurden, zu einer stark erhöhten signifikanten intrazellulären Calciumausschüttung (Figur 23 B).

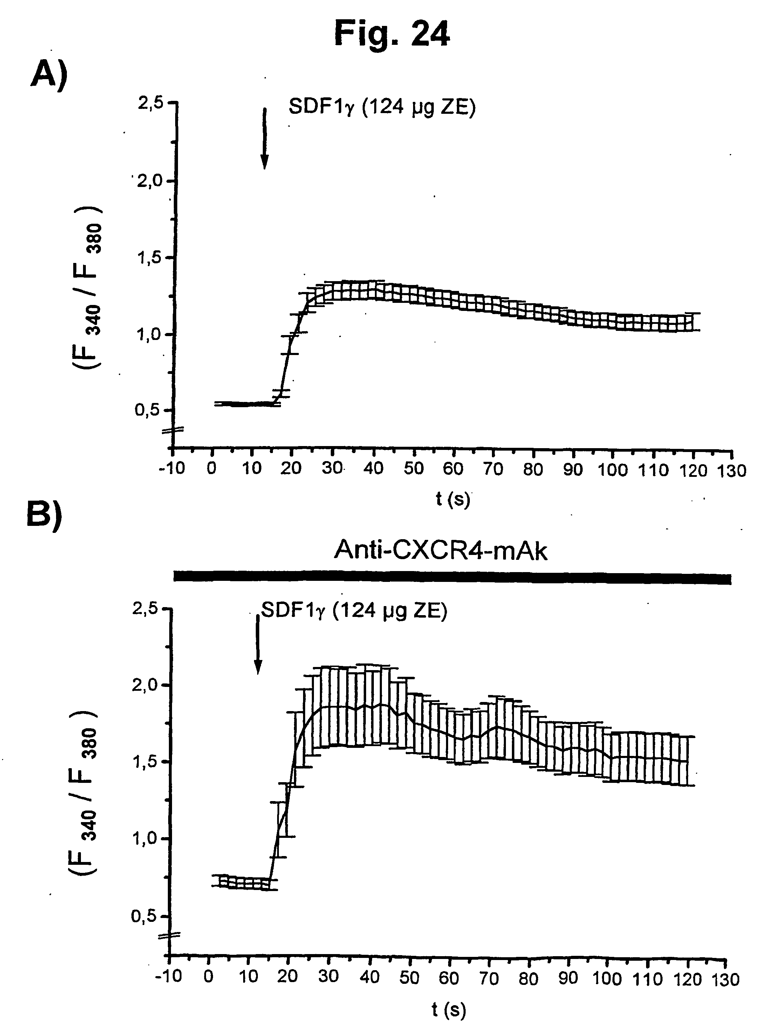

Auch Zellkulturen von Cortexneuronen aus dem Rattenhirn zeigen nach Vorinkubation mit dem CXCR4-Antikörper eine weitere Erhöhung der Calcium-Konzentration als Reaktion auf SDF-1γ. Figur 24 zeigt das Ergebnis eines entsprechenden Ca-Imaging-Experiments in Cortexneuronen. Die Applikation sowohl von 35 µg als auch von 124 µg (Gesamtprotein) eines Hefezellextrakts mit rekombinantem SDF-1γ (M-mhSDF-1γ-H6) führt zu einem signifikanten Anstieg der intrazellulären Calciumkonzentration in kultivierten primären Cortexneuronen (Figur 24 A). Nach 5-minütiger Vorinkubation mit dem monoklonalen Antikörper 12G5 (Antikörper gegen CXCR4) bewirkt die Zugabe von SDF-1γ eine stark erhöhte intrazelluläre Calciumausschüttung bei kultivierten primären Cortexneuronen (Figur 24 B).

Diese vorstehenden Ergebnisse belegen, dass sich die zellphysiologische Reaktion von Neuronen und Astrozyten aus dem Zentralnervensystem auf SDF-1α bzw. -1β von den Reaktionen auf das neue SDF-1γ-Chemokin deutlich unterscheidet.

### Beispiel 6

### Wirkung des C-terminalen basischen Peptids aus SDF-1γ und der davon abgeleiteten, synthetisch hergestellten Peptidspaltprodukte auf die intrazelluläre Ca-Konzentration in Astrozyten

Es wurde zunächst untersucht, wie sich die Zugabe eines basischen Peptids mit einer Aminosäuresequenz, die der Sequenz der letzten 30 Aminosäuren im C-terminalen Bereich von SDF-1γ entspricht, auf die intrazellulären Ca-Konzentration in Astrozyten auswirkt.

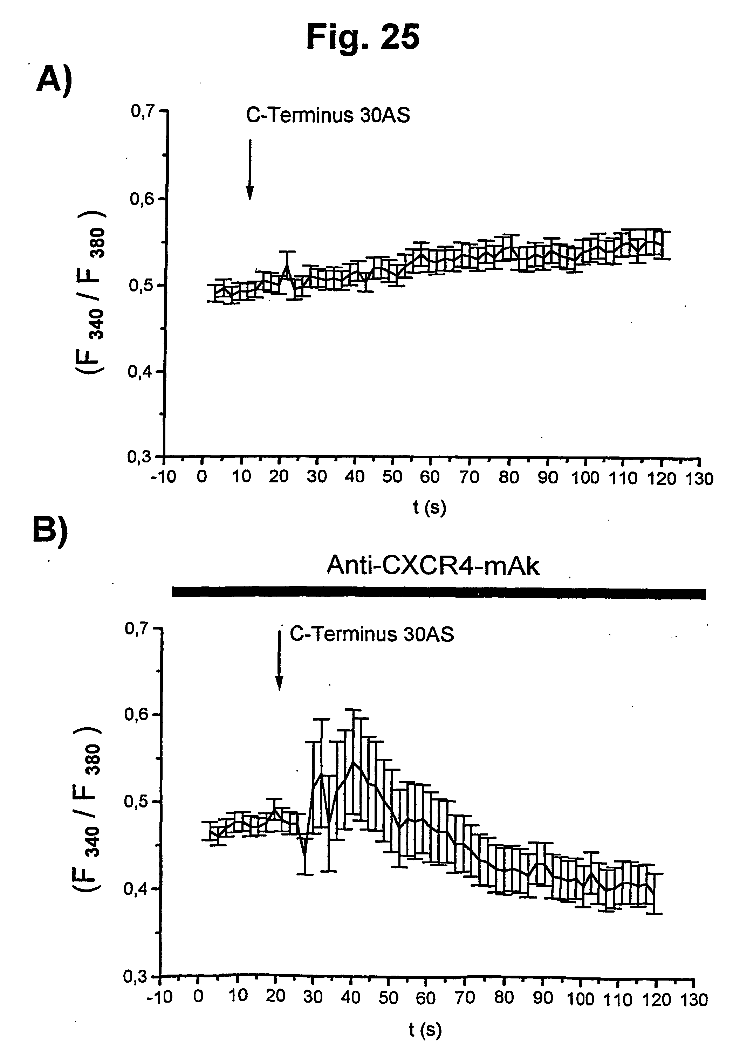

Figur 25 zeigt das Ergebnis eines Ca-Imaging-Experiments mit dem C-terminalen basischen Peptid von SDF-1γ in Astrozyten. Die Applikation von 1 µg/ml des synthetischen Peptids, das die C-terminalen 30 Aminosäuren von SDF-1γ repräsentiert, führte zu einer nur schwachen Beeinflussung der intrazellulären Ca-Konzentration der kultivierten Astrocyten (Figur 25 A). Wurden die Astrozyten zuvor 5 Minuten lang mit dem monoklonalen Antikörper 12G5 (Antikörper gegen CXCR4) inkubiert, so kommt es in den primären Astrocyten zu einer stark erhöhten intrazellulären Calciumausschüttung bei Applikation des gleichen C-terminalen Peptides (Figur 25 B).

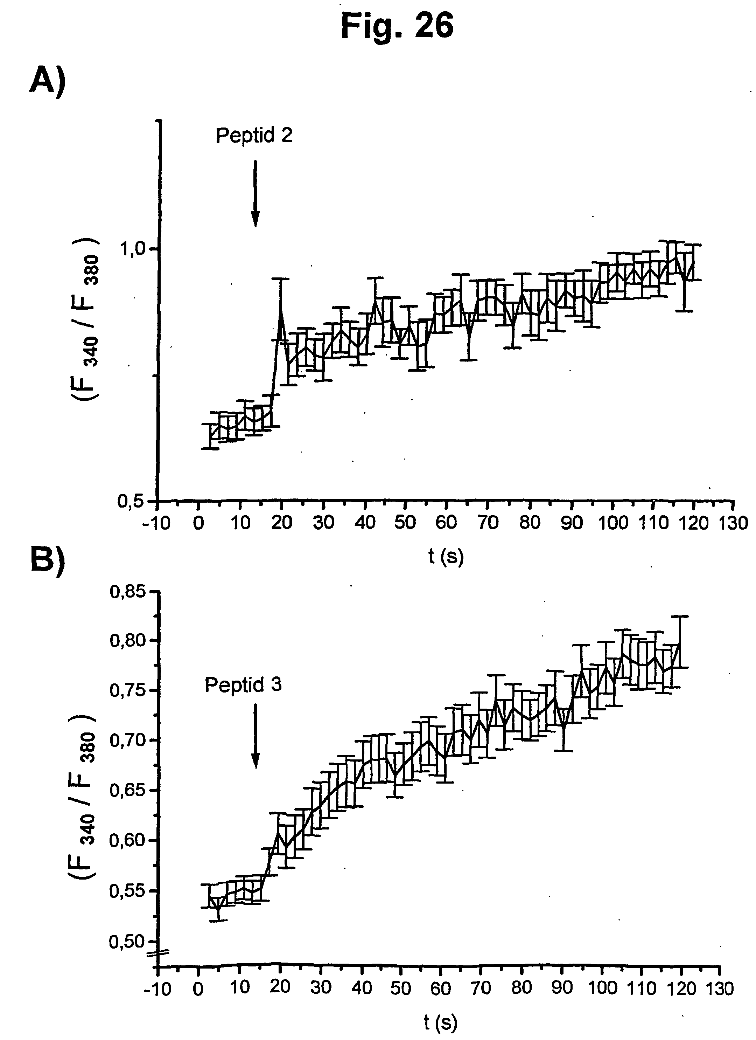

Wurde ein Ca-Imaging-Experiment mit den von dem C-terminalen Bereich von SDF-1γ abgeleiteten Peptiden RREEKVG (Peptid 1, SEQ ID NO:5), KKEKIG (Peptid 2, SEQ ID NO:6), KKKRQ (Peptid 3, SEQ ID NO:8), KKRKAAQ (Peptid 4, SEQ ID NO:9) und KKKN (Peptid 5, SEQ ID NO:10) sowie mit den wie in Eipper et al (1992) beschrieben amidierten Peptiden 1' (RREEKV(NH2)) und 2' (KKEKI(NH2)), so wurde festgestellt, dass die Zugabe der nicht-amidierten Peptide 1, 2 und 3 sowie der amidierten Peptide 1' und 2' zu einer Erhöhung der intrazellulären Ca-Konzentration in Astrozyten führten. In Figur 26 wird gezeigt, dass die Applikation von 1 mg/ml des Peptids 2 (KKEKIG; SEQ ID NO:6) (Figur 26 A) oder des Peptids 3 (KKKRQ; SEQ ID NO:8) (Figur 26 B) zu einem signifikanten Anstieg der intrazellulären Calciumkonzentration in kultivierten primären Astrocyten führt. Dagegen bewirkten die Peptide 4 und 5 keinen Anstieg der Ca-Konzentration in Astrozyten.

Diese Ergebnisse zeigen, dass die putativen Neuropeptide in unterschiedlicher Weise die intrazelluläre Calcium-Konzentration modulieren können. Sie legen ebenfalls nahe, dass für die von SDF-1γ hervorgerufene drastische Heraufregulation der intrazellulären Calcium-Konzentration nach Vorinkubation der Zellen mit dem Anti-CXCR4-Antikörper ausschließlich der C-terminale Bereich von SDF-1γ verantwortlich ist, nicht aber der mit den Chemokinen SDF-1α bzw. -1β übereinstimmende Molekülabschnitt. Dieser Befund belegt die besondere und spezifische Funktion des C-Terminus von SDF-1γ und bestätigt die Ergebnisse, die mit dem vollständigen SDF-1γ-Molekül erhalten wurden.

### Literatur

Altschul, S.F., Gish, W., Miller, W., Myers, E.W. und Lipman, D.J. (1990) Basic local alignment search tool. J. *Mol. Biol.* 215, 403-410.
Angerer, L.M., Stoler, M.H. und Angerer, R.C. (1987) In situ hybridization with RNA probes: An annotated recipe. In K.L. Valentno, R.H. Eberwine, J.D. Barchas (Hrsg.) In Situ Hybridization. Application to neurobiology, Oxford University Press, New York, 42-70.
Chomczynski, P. und Sacchi, N. (1987) Single step method of RNA isolation by acid guanidinium thiocyanate-phenol-chlorofrom extraction. *Anal. Biochem.* 162, 156-159.
Devereux, J., et al. (1984) *Nucleic Acids Research* 12 (12): 387.
Doranz, B.J., Orsini, M.J., Turner, J.D., Hoffman, T.L., Berson, J.F., Hoxie, J.A., Peiper, S.C., Brass, L.F. und Doms, R.W. (1999) Identification of CXCR4 domains that support coreceptor and chemokine receptor functions *J. Virol.* 73, 2752-2761.
Eipper, B., Stoffers, D. und Xu, R. (1992) The biosynthesis of neuropeptides: peptide alpha amidation. *Annu. Rev. Neurosci.* 15, 57-85.
Gellissen, G. (2000) Heterologous protein production in methylotrophic yeasts. *Appl. Microbiol. Biotechnol.* 54:741-750.
Henikoff und Henikoff (1992) *Proc. Natl. Acad. Sci.* USA 89:10915-10919.
Köller, H., Trimbom, M., von Giesen, H.-J., Schroeter, M. und Arendt, G. (2001) TNFα reduces glutamate induced intracellular Ca²⁺ increase in cultured cortical astrocytes. Brain Research 893:237-243.
Loetscher, P., Gong, J.H., Dewald, B., Baggiolini, M. und Clark-Lewis, I. (1998) N-terminal peptides of stromal cell-derived factor-1 with CXC chemokine receptor 4 agonist and antagonist activities. *J. Biochem.* 273 (35):22279-83.
Müller, H.W., Ignatius, M.J., Hangen, D.H. und Shooter, E.M. (1986) Expression of specific sheath cell proteins during peripheral nerve regeneration in mammals, *J. Cell Biol.* 102, 393-402.
Needleman und Wunsch (1970) *J. Mol. Biol*. 48:443-453.
Pearson, W.R. (1990) Rapid and sensitive sequence comparison with FASTP and FASTA. *Methods Enzymol.* 183, 63-98.
Rollins, B.J. (1997) Chemokines. *Blood* 90, 909-928.
Sambrook, J., Fritsch, E.F. und Maniatis, T. (1989) Molecular cloning: *A laboratory Manual* 2. Ausgabe, Cold Spring Harbor Laboratory Press, Cold Spring Harbor, NY.
Sanger, N., Nicklen, S. und Coulson, A.R., (1977) DNA sequencing with chain-terminating inhibitors. *Proc. Natl. Acad. Sci. USA,* 74, 144-148.
Shirozu, M., Nakano, T., Inazawa, J., Tashiro, K., Tada, H., Shinohara, T. und Honjo, T. (1995) Structure and chromosomal localization of the human stromal cell-derived factor 1 (SDF1) gene. *Genomics* 28, 495-500.
Siegel, G., Agranoff, B. Albers, R.W., Molinoff, P. (1989) Basic Neurochimstry, Raven Press, New York.
Stürzl, M., Roth, W.K., Viehweger, P. und Hoffschneider, H.Ü. (1991) Taq DNApolymerase synthesized single stranded DNA hybridization probes and their application in Northem blotting and in-situ hybridization. In Rolfs, A., Schumacher, A.C., Marx, P. (Hrsg.) PCR-Topics. Usage of Polymerase Chain Reaction in Genetic and Infectious Diseases. Berlin, Springer-Verlag, 41-45.
Tashiro, K. Tada, H., Heilker, R., Sherozou, M., Nakano, T. und Honjo, T. (1993) Signal sequence trap: a cloning strategy for secreted proteins and type I membrane proteins. *Science* 261, 600-602.
Weydemann, U., Keup, P., Piontek, M., Strasser, A.W.M., Schweden, J., Gellissen, G., Janowicz, Z.A. (1995) High level secretion of hirudin by *Hansenula polymorpha* - authentic processing of three different preprohirudins. *Appl. Microbiol. Biotechnol*. 44: 844-849.

## Patentansprüche

1. Nukleinsäure-Molekül mit einer Sequenz, umfassend:
(1) eine für ein Chemokin, einen Neuropeptid-Präkursor oder mindestens ein Neuropeptid kodierende Nukleinsäuresequenz, die aus folgenden Sequenzen ausgewählt ist:
(a) einer Nukleinsäuresequenz nach SEQ ID NO:1;
(b) einer Nukleinsäuresequenz, die ein Polypeptid mit einer Aminosäuresequenz nach SEQ ID NO:2 kodiert;
(c) einer Nukleinsäuresequenz, die mit der in (a) angegebenen Sequenz zu mindestens 80 % identisch ist;
(d) einer Sequenz, die mit dem Gegenstrang der in (a) angegebenen Sequenz unter stringenten Bedingungen hybridisiert;
oder
(2) eine komplementäre Sequenz zu einer der in (a) bis (d) angegebenen Nukleinsäuresequenzen.

2. Nukleinsäure-Molekül gemäß Anspruch 1, **dadurch gekennzeichnet, dass** die unter (1) (c) angegebene Nukleinsäuresequenz zu mindestens 90% identisch ist.

3. Nukleinsäure-Molekül gemäß Anspruch 1, **dadurch gekennzeichnet; dass** die unter (1) (c) angegebene Nukleinsäuresequenz zu mindestens 95% identisch ist.

4. Nukleinsäure-Molekül gemäß Anspruch 1, **dadurch gekennzeichnet; dass** es eine Nukleinsäuresequenz nach SEQ ID NO:3 umfasst.

5. Nukleinsäure-Molekül gemäß Anspruch 1, **dadurch gekennzeichnet, dass** es eine Nukleinsäuresequenz umfasst, die ein Polypeptid mit einer Aminosäuresequenz nach SEQ ID DO:4 kodiert.

6. Nukleinsäure-Molekül gemäß einem der Ansprüche 1 bis 5, weiterhin umfassend einen zur Expression geeigneten Promotor, wobei die kodierende Nukleinsäuresequenz unter der Kontrolle des Promotors steht.

7. Nukleinsäure-Molekül gemäß eines der Ansprüche 1 bis 6, weiterhin umfassend Sequenzen eines Vektors, die die Replikation des Nukleinsäure-Moleküls in einer Wirtszelle und/oder die Integration des Nukleinsäure-Moleküls in das Genom einer Wirtszelle ermöglichen.

8. Wirtszelle, enthaltend ein Nukleinsäure-Molekül gemäß einem der Ansprüche 1 bis 7, wobei die Wirtszelle eine zur Expression des Nukleinsäure-Moleküls geeignete prokaryontische oder eukaryontische Zelle ist.

9. Wirtszelle gemäß Anspruch 8, **dadurch gekennzeichnet, dass** die prokaryontische Wirtszelle *E. coli* ist.

10. Wirtszelle gemäß Anspruch 8, **dadurch gekennzeichnet, dass** die eukaryontische Wirtszelle eine Pilz-, eine Insekten- oder eine Säugerzelle ist.

11. Wirtszelle gemäß Anrpcuh 10, **dadurch gekennzeichnet, dass** die Wirtszelle die Hefe *Saccharomyces cerevisiae*, die methylotrophe Hefe *Hansenula polymorpha*, die dimorphe Hefe *Arxula adeninivorans* oder der filamentöse Pilz *Sordaria macrospora* ist.

12. Wirtszelle gemäß Anspruch 10, **dadurch gekennzeichnet, dass** die Wirtszelle eine CHO-, COS- oder HeLa-Zelle ist.

13. Polypeptid-Molekül, umfassend eine Aminosäuresequenz, die aus folgenden Sequenzen ausgewählt ist:
(i) einer Aminosäuresequenz, die eine Aminosäuresequenz nach SEQ ID NO:5 und/oder eine Kombination aus zwei oder mehreren der Aminosäuresequenzen SEQ ID NO:5, SEQ ID NO:6; SEQ ID NO:8, SEQ ID NO:9 und SEQ ID NO:10 umfasst;
(ii) einer Aminosäuresequenz nach SEQ ID NO:4;
(iii) einer Aminosäuresequenz, die der Sequenz von Aminosäure 20 bis Aminosäure 119 in SEQ ID NO:4 entspricht;
(iv) einer Aminosäuresequenz nach SEQ ID NO:22;
(v) einer Aminosäuresequenz, die mit der in (i), (ii) oder (iv) angegebenen Sequenz zu mindestens 85 % identisch ist;
(vi) einer Aminosäuresequenz, die eine Sequenz nach SEQ ID NO:6 oder eine Sequenz umfasst, die mit dieser zu mindestens 85 % identisch ist, ausgenommen folgende Aminosäuresequenz:
(vii) einer Aminosäuresequenz, die eine Sequenz nach SEQ ID NO:7, eine Kombination aus SEQ ID NO:7 und einer oder mehreren der Aminosäuresequenzen SEQ ID NO:5, SEQ ID NO:6, SEQ ID NO:8, SEQ ID NO:9 und SEQ ID NO:10 oder einer Sequenz umfasst, die mit einer Kombination aus dieser Sequenz und einer oder mehreren d3er vorgenannten Sequenzen zu mindestens 95 % identisch ist.
(viii) einer Aminosäuresequenz, die eine Sequenz nach SEQ ID NO:8 oder eine Sequenz umfasst, die mit dieser zu mindestens 85% identisch ist, ausgenommen die Aminosäuresequenz: und die Aminosäuresequenz: und die Aminosäuresequenz:
TRKKKRQRRKGSG;
(ix) einer Aminosäuresequenz, die eine Sequenz nach SEQ ID NO:9 oder eine Sequenz, die mit dieser zu mindestens 90% identisch ist, umfasst;
(x) einer Aminosäuresequenz, die eine Sequenz nach SEQ ID NO:10 umfasst, ausgenommen folgende Aminosäuresequenz: worin das Polypeptidmolekül ein Chemokin oder ein Neuropeptid ist.

14. Polypeptid-Molekül gemäß Anspruch 13, **dadurch gekennzeichnet, dass** die unter (v) angegebene Sequenz zu mindestens 90% identisch ist.

15. Polypeptid-Molekül gemäß Anspruch 13, **dadurch gekennzeichnet, dass** die unter (v) angegebene Sequenz zu mindestens 95% identisch ist.

16. Polypeptid-Molekül gemäß Anspruch 13, **dadurch gekennzeichnet, dass** es eine Aminosäuresequenz nach SEQ ID NO:12 oder SEQ ID NO:13 umfasst.

17. Fragment eines Polypeptid-Moleküls gemäß einem der Ansprüche 13 bis 16, das mindestens ein Neuropeptid umfasst.

18. Fragment gemäß Anspruch 17, umfassend mindestens eine der Aminosäuresequenzen nach SEQ ID NO:5, SEQ ID NO:6, SEQ ID NO: 7, SEQ ID NO: 8, SEQ ID NO:9 und/oder SEQ ID NO:10.

19. Verfahren zum Herstellen eines Polypeptid-Moleküls und/oder eines Fragments davon gemäß einem der Ansprüche 13 bis 18, umfassend das Kultivieren einer Wirtszelle gemäß einem der Ansprüche 8 bis 12 unter zur Expression und eventuellen Prozessierung geeigneten Bedingungen und gegebenenfalls das Aufreinigen des exprimierten Polypeptid-Moleküls oder Fragments.

20. In vitro-Verfahren zum Nachweisen eines Polypeptid-Moleküls und/oder eines Fragments davon gemäß einem der Ansprüche 13 bis 18 in einer biologischen Probe, umfassend das Inkontakbringen der Probe mit einem für das Polypeptid-Molekül und/oder das Fragment spezifischen Reagenz und das Nachweisen der Bindung.

21. Test-Kit zum Nachweisen eines Polypeptids und/oder eines Fragments davon gemäß einem der Ansprüche 13 bis 18, umfassend mindestens ein Reagenz, das für das Polypeptid und/oder das Fragment spezifisch ist.

22. In vitro-Verfahren zum Nachweisen einer ein Polypeptid-Molekül gemäß einem der Ansprüche 13 bis 16 kodierenden Nukleinsäure in einer biologischen Probe, umfassend:
- das Inkontaktbringen der Probe mit einem Nukleinsäure-Molekül gemäß einem der Ansprüche 1 bis 5 und/oder einem Fragment davon, wobei das Nukleinsäure-Molekül und/oder das Fragment eine nachweisbare Markierung tragen, und
- das Nachweisen der Markierung.

23. Pharmazeutische Zusammensetzung, enthaltend mindestens ein Polypeptid-Molekül und/oder ein Fragment davon gemäß einem der Ansprüche 13 bis 18 und/oder ein pharmazeutisch verträgliches Salz eines solchen Polypeptid-Moleküls oder Fragments.

24. Pharmazeutische Zusammensetzung gemäß Anspruch 23, **dadurch gekennzeichnet, dass** sie weiterhin mindestens einen Antikörper enthält.

25. Pharmazeutische Zusammensetzung gemäß Anspruch 23 oder 24 zur therapeutischen Anwendung bei demyelinisierenden oder neurodegenerativen Erkrankungen oder bei Entwicklungsstörungen des Nervensystems.

26. Pharmazeutische Zusammensetzung gemäß Anspruch 23 oder 24 zur Vorbeugung oder Behandlung einer HIV-Infektion und insbesondere einer HIV-Enzephalopathie in Menschen.

27. Pharmazeutische Zusammensetzung gemäß Anspruch 23 oder 24 zur therapeutischen Anwendung bei Erkrankungen des hämatopoietischen Systems, des Immunsystems und des Herz/Kreislaufsystems.

28. Pharmazeutische Zusammensetzung, enthaltend mindestens einen für ein Polypeptidmolekül und/oder Fragment davon gemäß einem der Ansprüche 13 - 18 spezifischen Antikörper.

29. Pharmazeutische Zusammensetzung, enthaltend mindestens ein Nukleinsäure-Molekül gemäß einem der Ansprüche 1 bis 7.

30. Pharmazeutische Zusammensetzung gemäß einem der Ansprüche 27 bis 29 zur diagnostischen oder therapeutischen Anwendung bei demyelinisierenden oder neurodegenerativen Erkrankungen oder bei Entwicklungsstörungen des Nervensystems.

31. Pharmazeutische Zusammensetzung gemäß einem der Ansprüche 27 bis 29 zur Diagnose oder Behandlung einer HIV-Infektion und insbeosndere einer HIV-Enzephalopathie in Menschen.

## Claims

1. Nucleic acid molecule with a sequence comprising
(1) nucleic acid sequence coding for a chemokine, a neuropeptide precursor or at least one neuropeptide, which is selected from the following sequences:
(a) a nucleic acid sequence agreeing with SEQ ID NO: 1;
(b) a nucleic acid sequence which codes for a polypeptide with an amino-acid sequence agreeing with SEQ ID NO:2;
(c) a nucleic acid sequence which is at least 80% identical with the sequence indicated in (a);
(d) a sequence which hybridizes with the opposing strand of the sequence indicated in (a), under stringent conditions;
or
(2) a sequence complementary to one of the nucleic acid sequences given under (a) to (d).

2. Nucleic acid molecule in accordance with Claim 1, **characterized** thereby that the nucleic acid sequence given under (1) (c) is at least 90% identical.

3. Nucleic acid molecule in accordance with Claim 1, **characterized** thereby that the nucleic acid sequence given under (1) (c) is at least 95% identical.

4. Nucleic acid molecule in accordance with Claim 1, **characterized** thereby that it includes a nucleic acid sequence agreeing with SEQ ID NO:3.

5. Nucleic acid molecule in accordance with Claims 1, **characterized** thereby that it includes a nucleic acid sequence which codes for a polypeptide with an amino acid sequence which agrees with SEQ ID NO:4.

6. Nucleic acid molecule in accordance with one of claims 1 to 5, comprising in addition a promotor suitable for expression, in which the coding nucleic acid sequence remains under the control of the promotor.

7. Nucleic acid molecule in accordance with one of claims 1 to 6, comprising in addition sequences of a vector which enables the replication of the nucleic acid molecule in a host cell and/or the integration of the nucleic acid molecule into the genome of a host cell.

8. Host cell containing a nucleic acid molecule in accordance with one of claims 1 to 7, by which the host cell is a prokaryotic or eukaryotic cell suitable for the expression of the nucleic acid molecule.

9. Host cell in accordance with Claim 8, **characterized** thereby that the prokaryotic host cell is *E. coli*.

10. Host cell in accordance with Claim 8, **characterized** thereby that the eukaryotic host cell is a fungal, an insect or a mammalian cell.

11. Host cell in accordance with Claim 10, **characterized** thereby that the host cell is the yeast *Saccharomyces cerevisiae*, the methylotrophic yeast *Hansenula polymorpha*, the dimorphic yeast *Arxula adeninivorans* or the filamentous fungus *Sordaria macrospora*.

12. Host cell in accordance with Claim 10, **characterized** thereby that the host cell is a CHO, COS or HeLa cell.

13. Polypeptide molecule comprising an amino-acid sequence selected from the following sequences:
(i) an amino-acid sequence which includes an amino-acid sequence agreeing with SEQ ID NO:5, and/or a combination of two or more of the amino-acid sequences SEQ ID NO:5, SEQ ID NO:6, SEQ ID NO:8, SEQ ID NO:9 and SEQ ID NO:10;
(ii) an amino-acid sequence agreeing with SEQ ID NO:4;
(iii) an amino-acid sequence corresponding to the sequence of amino-acid 20 to amino-acid 119 in SEQ ID NO:4;
(iv) an amino-acid sequence agreeing with SEQ ID NO:22;
(v) an amino-acid sequence which is at least 85% identical with the sequence given under (i), (ii), or (iv);
(vi) an amino-acid sequence comprising a sequence agreeing with SEQ ID NO:6 or a sequence at least 85% identical therewith, excluding the following amino-acid sequence:
(vii) an amino acid sequence comprising a sequence agreeing with SEQ ID NO:7, a combination of SEQ ID NO:7 and one or several of the amino acid sequences SEQ ID NO:5, SEQ ID NO:6, SEQ ID NO:8, SEQ ID NO:9 and SEQ ID NO:10 or a sequence which is at least 95% identical with a combination of this sequence and one or several of the aforementioned sequences.
(viii) an amino acid sequence comprising a sequence agreeing with SEQ ID NO:8 or a sequence which is at least 85% identical with the same, excluding the following amino acid sequence: and the amino-acid sequence: and the amino-acid sequence:
TRKKKRQRRKGSG;
(ix) an amino-acid sequence comprising a sequence agreeing with SEQ ID NO:9 or a sequence at least 90% identical with this.
(x) an amino-acid sequence comprising a sequence agreeing with SEQ ID NO:10, excluding the following amino-acid sequence: wherein the polypeptide molecule is a chemokine or a neuropeptide.

14. Polypeptide molecule in accordance with Claim 13, **characterized** thereby, that the sequence given under (v) is at least 90% identical.

15. Polypeptide molecule in accordance with Claim 13, **characterized** thereby, that the sequence given under (v) is at least 95% identical.

16. Polypeptide molecule in accordance with Claim 13, **characterized** thereby, that it comprises an amino-acid sequence agreeing with SEQ ID NO:12 or SEQ ID NO:13.

17. Fragment of a polypeptide molecule in accordance with one of the Claims 13 to 16 which comprises at least one neuropeptide.

18. Fragment in accordance with Claim 17, comprising at least one of the amino-acid sequences agreeing with SEQ ID NO:5, SEQ ID NO:6, SEQ ID NO:7, SEQ ID NO:8, SEQ ID NO:9 and/or SEQ ID NO:10.

19. Procedure for the manufacture of a polypeptide molecule and/or of a fragment thereof in accordance with one of the Claims 13 to 18, comprising the cultivation of a host cell in accordance with one of the Claims 8 to 12 under suitable conditions for the expression and possible processing and if necessary the purification of the polypeptide molecules or fragments expressed.

20. In vitro procedure for the demonstration in a biological specimen of a polypeptide molecule and/or a fragment thereof in accordance with one of Claims 13 to 18, comprising bringing into contact of the specimen with a reagent specific for the polypeptide molecule and/or the fragment, and the demonstration of binding.

21. Test-kit for demonstration of a polypeptide and/or a fragment thereof in accordance with one of Claims 13 to 18, comprising at least one reagent which is specific for the polypeptide and/or the fragment.

22. In vitro procedure for the demonstration of a nucleic acid coding for a polypeptide molecule in accordance with one of Claims 13 to 16 in a biological specimen, comprising:
- the bringing into contact of the specimen with a nucleic acid molecule in accordance with one of Claims 1 to 5 and/or a fragment thereof, of which the nucleic acid molecule and/or the fragment bears a demonstrable marking, and
- the demonstration of the marking.

23. Pharmaceutical preparation containing at least one polypeptide molecule and/or a fragment thereof in accordance with one of Claims 13 to 18, and/or a pharmaceutically compatible salt of such a polypeptide molecule or fragment.

24. Pharmaceutical preparation in accordance with Claim 23, **characterized** thereby that it contains in addition at least one antibody.

25. Pharmaceutical preparation in accordance with Claim 23 or 24, for therapeutic use in demyelinating or neurodegenerative diseases or in developmental disorders of the nervous system.

26. Pharmaceutical preparation in accordance with Claim 23 or 24, for the prevention or treatment of an HIV infection and in particular an HIV encephalopathy in humans.

27. Pharmaceutical preparation in accordance with Claim 23 or 24, for therapeutic use in diseases of the haemopoietic system, the immune system and the cardiovascular system.

28. Pharmaceutical preparation containing at least one antibody specific for a polypeptide molecule and/or a fragment thereof in accordance with one of the Claims 13 to 18.

29. Pharmaceutical preparation containing at least one nucleic acid molecule in accordance with one of the Claims 1 to 7.

30. Pharmaceutical preparation in accordance with one of Claims 27 to 29 for diagnostic or therapeutic use in demyelinating or neurodegenerative diseases or in developmental disorders of the nervous system.

31. Pharmaceutical preparation in accordance with one of Claims 27 to 29 for the diagnosis or treatment of an HIV infection and in particular an HIV encephalopathy in humans.

## Revendications

1. Molécule d'acide nucléique avec une séquence, comprenant :
(1) une séquence d'acide nucléique codant une chimiokine, un précurseur de neuropeptide ou au moins un neuropeptide, qui est choisie parmi les séquences suivantes :
(a) une séquence d'acide nucléique selon SEQ ID NO:1 ;
(b) une séquence d'acide nucléique qui code un polypeptide ayant une séquence d'acides aminés selon SEQ ID NO:2 ;
(c) une séquence d'acide nucléique qui est identique à au moins 80% à la séquence indiquée en (a) ;
(d) une séquence qui s'hybride sur le brin antisens de la séquence indiquée en (a) dans des conditions stringentes ;
ou
(2) une séquence complémentaire de l'une des séquences d'acide nucléique indiquées en (a) à (d).

2. Molécule d'acide nucléique selon la revendication 1, **caractérisée en ce que** la séquence d'acide nucléique indiquée en (1) (c) est identique à au moins 90%.

3. Molécule d'acide nucléique selon la revendication 1, **caractérisée en ce que** la séquence d'acide nucléique indiquée en (1) (c) est identique à au moins 95%.

4. Molécule d'acide nucléique selon la revendication 1, **caractérisée en ce qu'**elle comprend une séquence d'acide nucléique selon SEQ ID NO:3.

5. Molécule d'acide nucléique selon la revendication 1, **caractérisée en ce qu'**elle comprend une séquence d'acide nucléique qui code un polypeptide ayant une séquence d'acides aminés selon SEQ ID NO:4.

6. Molécule d'acide nucléique selon l'une quelconque des revendications 1 à 5, comprenant en outre un promoteur approprié à l'expression, dans laquelle la séquence d'acide nucléique codante est sous le contrôle dudit promoteur.

7. Molécule d'acide nucléique selon l'une quelconque des revendications 1 à 6, comprenant en outre des séquences d'un vecteur, qui permettent la réplication de la molécule d'acide nucléique dans une cellule hôte et/ou l'intégration de la molécule d'acide nucléique dans le génome d'une cellule hôte.

8. Cellule hôte contenant une molécule d'acide nucléique selon l'une quelconque des revendications 1 à 7, dans laquelle la cellule hôte est une cellule procaryote ou eucaryote appropriée à l'expression de la molécule d'acide nucléique.

9. Cellule hôte selon la revendication 8, **caractérisée en ce que** la cellule hôte procaryote est *E. coli*.

10. Cellule hôte selon la revendication 8, **caractérisée en ce que** la cellule hôte eucaryote est une cellule fongique, une cellule d'insecte ou de mammifère.

11. Cellule hôte selon la revendication 10, **caractérisée en ce que** la cellule hôte est la levure *Saccharomyces cerevisiae*, la levure méthylotrophe *Hansenula polymorpha*, la levure dimorphe *Arxula adeninivorans* ou le champignon filamenteux *Sordaria macrospora*.

12. Cellule hôte selon la revendication 10, **caractérisée en ce que** la cellule hôte est une cellule CHO, COS ou HeLa.

13. Molécule polypeptidique comprenant une séquence d'acides aminés qui est choisie parmi les séquences suivantes :
(i) une séquence d'acides aminés qui comprend une séquence d'acides aminés selon SEQ ID NO:5 et/ou une combinaison de deux ou plus des séquences d'acides aminés SEQ ID NO:5, SEQ ID NO:6, SEQ ID NO:8, SEQ ID NO:9 et SEQ ID NO:10 ;
(ii) une séquence d'acides aminés selon SEQ ID NO:4 ;
(iii) une séquence d'acides aminés qui correspond à la séquence de l'acide aminé 20 à l'acide aminé 119 dans SEQ ID NO:4 ;
(iv) une séquence d'acides aminés selon SEQ ID NO:22 ; (v) une séquence d'acides aminés qui est identique à au moins 85% à la séquence indiquée en (i), (ii) ou (iv) ;
(vi) une séquence d'acides aminés qui comprend une séquence selon SEQ ID NO:6 ou une séquence qui est identique à celle-ci à au moins 85%, à l'exception de la séquence d'acides aminés suivante :
(vii) une séquence d'acides aminés qui comprend une séquence selon SEQ ID NO:7, une combinaison de SEQ ID NO:7 et d'une ou plusieurs des séquences d'acides aminés SEQ ID NO:5, SEQ ID NO:6, SEQ ID NO:8, SEQ ID NO:9 et SEQ ID NO:10 ou une séquence qui est identique à au moins 95% à une combinaison de cette séquence et d'une ou plusieurs des séquences susmentionnées ;
(viii)une séquence d'acides aminés qui comprend une séquence selon SEQ ID NO:8 ou une séquence qui est identique à celle-ci à au moins 85%, à l'exception de la séquence d'acides aminés : et de la séquence d'acides aminés : et de la séquence d'acides aminés :
TRKKKRQRRKGSG;
(ix) une séquence d'acides aminés qui comprend une séquence selon SEQ ID NO:9 ou une séquence qui est identique à celle-ci à au moins 90% ;
(x) une séquence d'acides aminés qui comprend une séquence selon SEQ ID NO:10 à l'exception de la séquence d'acides aminés suivante :
dans laquelle la molécule polypeptidique est une chimiokine ou un neuropeptide.

14. Molécule polypeptidique selon la revendication 13, **caractérisée en ce que** la séquence indiquée en (v) est identique à au moins 90%.

15. Molécule polypeptidique selon la revendication 13, **caractérisée en ce que** la séquence indiquée en (v) est identique à au moins 95%.

16. Molécule polypeptidique selon la revendication 13, **caractérisée en ce qu'**elle comprend une séquence d'acides aminés selon SEQ ID NO:12 ou SEQ ID NO:13.

17. Fragment d'une molécule polypeptidique selon l'une quelconque des revendications 13 à 16, qui comprend au moins un neuropeptide.

18. Fragment selon la revendication 17, comprenant au moins l'une des séquences d'acides aminés selon SEQ ID NO:5, SEQ ID NO:6, SEQ ID NO:7, SEQ ID NO:8, SEQ ID NO:9 et/ou SEQ ID NO:10.

19. Procédé de préparation d'une molécule polypeptidique et/ou d'un fragment de celle-ci selon l'une quelconque des revendications 13 à 18, comprenant la mise en culture d'une cellule hôte selon l'une quelconque des revendications 8 à 12 dans des conditions appropriées à l'expression et à une éventuelle maturation, et éventuellement la purification de la molécule polypeptidique ou du fragment exprimé(e).

20. Procédé de détection in vitro d'une molécule polypeptidique et/ou d'un fragment de celle-ci selon l'une quelconque des revendications 13 à 18 dans un échantillon biologique, comprenant la mise en contact de l'échantillon avec un réactif spécifique de la molécule polypeptidique et/ou du fragment et la détection de la liaison.

21. Trousse de test pour détecter un polypeptide et/ou un fragment de celui-ci selon l'une quelconque des revendications 13 à 18, comprenant au moins un réactif qui est spécifique du polypeptide et/ou du fragment.

22. Procédé de détection in vitro d'un acide nucléique codant une molécule polypeptidique selon l'une quelconque des revendications 13 à 16 dans un échantillon biologique, comprenant :
- la mise en contact de l'échantillon avec une molécule d'acide nucléique selon l'une quelconque des revendications 1 à 5 et/ou un fragment de celle-ci, dans lequel la molécule d'acide nucléique et/ou le fragment portent un marqueur détectable, et
- la détection du marqueur.

23. Composition pharmaceutique contenant au moins une molécule polypeptidique et/ou un fragment de celle-ci selon l'une quelconque des revendications 13 à 18 et/ou un sel acceptable sur le plan pharmaceutique d'une telle molécule polypeptidique ou d'un tel fragment.

24. Composition pharmaceutique selon la revendication 23, **caractérisée en ce qu'**elle contient en outre au moins un anticorps.

25. Composition pharmaceutique selon la revendication 23 ou 24 pour une mise en oeuvre thérapeutique dans le cadre de maladies démyélinisantes ou neurodégénératives ou de troubles du développement du système nerveux.

26. Composition pharmaceutique selon la revendication 23 ou 24 pour la prophylaxie ou le traitement d'une infection à VIH et en particulier d'une encéphalopathie à VIH chez l'Homme.

27. Composition pharmaceutique selon la revendication 23 ou 24 pour une mise en oeuvre thérapeutique dans le cadre de maladies du système hématopoïétique, du système immunitaire et du système cardio-vasculaire.

28. Composition pharmaceutique contenant au moins un anticorps spécifique d'une molécule polypeptidique et/ou d'un fragment de celle-ci selon l'une quelconque des revendications 13 à 18.

29. Composition pharmaceutique contenant au moins une molécule d'acide nucléique selon l'une quelconque des revendications 1 à 7.

30. Composition pharmaceutique selon l'une quelconque des revendications 27 à 29 pour une mise en oeuvre diagnostique ou thérapeutique dans le cadre de maladies démyélinisantes ou neurodégénératives ou de troubles du développement du système nerveux.

31. Composition pharmaceutique selon l'une quelconque des revendications 27 à 29 pour le diagnostic ou le traitement d'une infection à VIH et en particulier d'un encéphalopathie à VIH chez l'Homme.
